# EUROPEAN PATENT SPECIFICATION

(11) **EP 2 464 967 B1**
(45) Date of publication and mention of the grant of the patent: **12.06.2013**
(21) Application number: 10744888.8
(22) Date of filing: 13.08.2010
(51) Int. Cl.: G01N 33/50, G01N 33/543

(54) **METHODS FOR THE IDENTIFICATION AND CHARACTERIZATION OF HDAC INTERACTING COMPOUNDS**
Verfahren zur Identifikation und Charakterisierung von HDAC-interagierenden Verbindungen
Procédés pour l'identification et la caractérisation des composés d'interaction HDAC

(30) Priority: 14.08.2009 EP 09010522; 23.06.2010 US 357820 P
(43) Date of publication of application: 20.06.2012
(73) Proprietor: CELLZOME AG, 69117 Heidelberg (DE)
(72) Inventor: DREWES, Gerard, 69121 Heidelberg (DE); BANTSCHEFF, Marcus, 69234 Dielheim (DE); KRUSE, Ulrich, 69221 Dossenheim (DE); HOPF, Carsten, 68165 Mannheim (DE); GRANDI, Paola, 69126 Heidelberg (DE)
(74) Representative: Povey, Alexander W.G.
(86) International application number: PCT/EP2010/005000
(87) International publication number: WO 2011/018241

(56) References cited:
- WO-A1-01/67107
- LI, J. ET AL: "Expression and functional characterization of recombinant human HDAC1 and HDAC3" LIFE SCIENCES, vol. 74, 2004, pages 2693-2705, XP002559920
- RIESTER ET AL: "Histone deacetylase inhibitor assay based on fluorescence resonance energy transfer" ANALYTICAL BIOCHEMISTRY, ACADEMIC PRESS INC, NEW YORK, vol. 362, no. 1, 1 March 2007 (2007-03-01) , pages 136-141, XP022056712 ISSN: 0003-2697
- BLACKWELL L ET AL: "The use of diversity profiling to characterize chemical modulators of the histone deacetylases" LIFE SCIENCES, PERGAMON PRESS, OXFORD, GB, vol. 82, no. 21-22, 23 May 2008 (2008-05-23), pages 1050-1058, XP022648475 ISSN: 0024-3205 [retrieved on 2008-03-21] cited in the application

### SEQUENCE LISTING

<110> cellzome AG
<120> Methods for the identification and characterization of HDAC interacting compounds
<130> CEL66494PC
<160> 24
<170> PatentIn version 3.5
<210> 1
   <211> 482
   <212> PRT
   <213> Homo sapiens
<400> 1
   Met Ala Gln Thr Gln Gly Thr Arg Arg Lys Val Cys Tyr Tyr Tyr Asp
   1               5                   10                  15
   Gly Asp Val Gly Asn Tyr Tyr Tyr Gly Gln Gly His Pro Met Lys Pro
               20                  25                  30
   His Arg Ile Arg Met Thr His Asn Leu Leu Leu Asn Tyr Gly Leu Tyr
           35                  40                  45
   Arg Lys Met Glu Ile Tyr Arg Pro His Lys Ala Asn Ala Glu Glu Met
       50                  55                  60
   Thr Lys Tyr His Ser Asp Asp Tyr Ile Lys Phe Leu Arg Ser Ile Arg
   65                  70                  75                  80
   Pro Asp Asn Met Ser Glu Tyr Ser Lys Gln Met Gln Arg Phe Asn Val
                   85                  90                  95
   Gly Glu Asp Cys Pro Val Phe Asp Gly Leu Phe Glu Phe Cys Gln Leu
               100                 105                 110
   Ser Thr Gly Gly Ser Val Ala Ser Ala Val Lys Leu Asn Lys Gln Gln
           115                 120                 125
   Thr Asp Ile Ala Val Asn Trp Ala Gly Gly Leu His His Ala Lys Lys
       130                 135                 140
   Ser Glu Ala Ser Gly Phe Cys Tyr Val Asn Asp Ile Val Leu Ala Ile
   145                 150                 155                 160
   Leu Glu Leu Leu Lys Tyr His Gln Arg Val Leu Tyr Ile Asp Ile Asp
                   165                 170                 175
   Ile His His Gly Asp Gly Val Glu Glu Ala Phe Tyr Thr Thr Asp Arg
               180                 185                 190
   Val Met Thr Val Ser Phe His Lys Tyr Gly Glu Tyr Phe Pro Gly Thr
   195 200 205
   Gly Asp Leu Arg Asp Ile Gly Ala Gly Lys Gly Lys Tyr Tyr Ala Val
   210 215 220
   Asn Tyr Pro Leu Arg Asp Gly Ile Asp Asp Glu Ser Tyr Glu Ala Ile
   225 230 235 240
   Phe Lys Pro Val Met Ser Lys Val Met Glu Met Phe Gln Pro Ser Ala
   245 250 255
   Val Val Leu Gln Cys Gly Ser Asp Ser Leu Ser Gly Asp Arg Leu Gly
   260 265 270
   Cys Phe Asn Leu Thr Ile Lys Gly His Ala Lys Cys Val Glu Phe Val
   275 280 285
   Lys Ser Phe Asn Leu Pro Met Leu Met Leu Gly Gly Gly Gly Tyr Thr
   290 295 300
   Ile Arg Asn Val Ala Arg Cys Trp Thr Tyr Glu Thr Ala Val Ala Leu
   305 310 315 320
   Asp Thr Glu Ile Pro Asn Glu Leu Pro Tyr Asn Asp Tyr Phe Glu Tyr
   325 330 335
   Phe Gly Pro Asp Phe Lys Leu His Ile Ser Pro Ser Asn Met Thr Asn
   340 345 350
   Gln Asn Thr Asn Glu Tyr Leu Glu Lys Ile Lys Gln Arg Leu Phe Glu
   355 360 365
   Asn Leu Arg Met Leu Pro His Ala Pro Gly Val Gln Met Gln Ala Ile
   370 375 380
   Pro Glu Asp Ala Ile Pro Glu Glu Ser Gly Asp Glu Asp Glu Asp Asp
   385 390 395 400
   Pro Asp Lys Arg Ile Ser Ile Cys Ser Ser Asp Lys Arg Ile Ala Cys
   405 410 415
   Glu Glu Glu Phe Ser Asp Ser Glu Glu Glu Gly Glu Gly Gly Arg Lys
   420 425 430
   Asn Ser Ser Asn Phe Lys Lys Ala Lys Arg Val Lys Thr Glu Asp Glu
   435 440 445
   Lys Glu Lys Asp Pro Glu Glu Lys Lys Glu Val Thr Glu Glu Glu Lys
   450 455 460
   Thr Lys Glu Glu Lys Pro Glu Ala Lys Gly Val Lys Glu Glu Val Lys
   465 470 475 480
   Leu Ala
<210> 2
   <211> 582
   <212> PRT
   <213> Homo sapiens
<400> 2
   Met Arg Ser Pro Pro Cys Gly Leu Leu Arg Trp Phe Gly Gly Pro Leu
   1               5                   10                  15
   Leu Ala Ser Trp Cys Arg Cys His Leu Arg Phe Arg Ala Phe Gly Thr
               20                  25                  30
   Ser Ala Gly Trp Tyr Arg Ala Phe Pro Ala Pro Pro Pro Leu Leu Pro
           35                  40                  45
   Pro Ala Cys Pro Ser Pro Arg Asp Tyr Arg Pro His Val Ser Leu Ser
       50                  55                  60
   Pro Phe Leu Ser Arg Pro Ser Arg Gly Gly Ser Ser Ser Ser Ser Ser
   65                  70                  75                  80
   Ser Arg Arg Arg Ser Pro Val Ala Ala Val Ala Gly Glu Pro Met Ala
                   85                  90                  95
   Tyr Ser Gln Gly Gly Gly Lys Lys Lys Val Cys Tyr Tyr Tyr Asp Gly
               100                 105                 110
   Asp Ile Gly Asn Tyr Tyr Tyr Gly Gln Gly His Pro Met Lys Pro His
           115                 120                 125
   Arg Ile Arg Met Thr His Asn Leu Leu Leu Asn Tyr Gly Leu Tyr Arg
       130                 135                 140
   Lys Met Glu Ile Tyr Arg Pro His Lys Ala Thr Ala Glu Glu Met Thr
   145                 150                 155                 160
   Lys Tyr His Ser Asp Glu Tyr Ile Lys Phe Leu Arg Ser Ile Arg Pro
                   165                 170                 175
   Asp Asn Met Ser Glu Tyr Ser Lys Gln Met Gln Arg Phe Asn Val Gly
               180                 185                 190
   Glu Asp Cys Pro Val Phe Asp Gly Leu Phe Glu Phe Cys Gln Leu Ser
           195                 200                 205
   Thr Gly Gly Ser Val Ala Gly Ala Val Lys Leu Asn Arg Gln Gln Thr
   210 215 220
   Asp Met Ala Val Asn Trp Ala Gly Gly Leu His His Ala Lys Lys Ser
   225 230 235 240
   Glu Ala Ser Gly Phe Cys Tyr Val Asn Asp Ile Val Leu Ala Ile Leu
   245 250 255
   Glu Leu Leu Lys Tyr His Gln Arg Val Leu Tyr Ile Asp Ile Asp Ile
   260 265 270
   His His Gly Asp Gly Val Glu Glu Ala Phe Tyr Thr Thr Asp Arg Val
   275 280 285
   Met Thr Val Ser Phe His Lys Tyr Gly Glu Tyr Phe Pro Gly Thr Gly
   290 295 300
   Asp Leu Arg Asp Ile Gly Ala Gly Lys Gly Lys Tyr Tyr Ala Val Asn
   305 310 315 320
   Phe Pro Met Arg Asp Gly Ile Asp Asp Glu Ser Tyr Gly Gln Ile Phe
   325 330 335
   Lys Pro Ile Ile Ser Lys Val Met Glu Met Tyr Gln Pro Ser Ala Val
   340 345 350
   Val Leu Gln Cys Gly Ala Asp Ser Leu Ser Gly Asp Arg Leu Gly Cys
   355 360 365
   Phe Asn Leu Thr Val Lys Gly His Ala Lys Cys Val Glu Val Val Lys
   370 375 380
   Thr Phe Asn Leu Pro Leu Leu Met Leu Gly Gly Gly Gly Tyr Thr Ile
   385 390 395 400
   Arg Asn Val Ala Arg Cys Trp Thr Tyr Glu Thr Ala Val Ala Leu Asp
   405 410 415
   Cys Glu Ile Pro Asn Glu Leu Pro Tyr Asn Asp Tyr Phe Glu Tyr Phe
   420 425 430
   Gly Pro Asp Phe Lys Leu His Ile Ser Pro Ser Asn Met Thr Asn Gln
   435 440 445
   Asn Thr Pro Glu Tyr Met Glu Lys Ile Lys Gln Arg Leu Phe Glu Asn
   450 455 460
   Leu Arg Met Leu Pro His Ala Pro Gly Val Gln Met Gln Ala Ile Pro
   465 470 475 480
   Glu Asp Ala Val His Glu Asp Ser Gly Asp Glu Asp Gly Glu Asp Pro
   485 490 495
   Asp Lys Arg Ile Ser Ile Arg Ala Ser Asp Lys Arg Ile Ala Cys Asp
   500 505 510
   Glu Glu Phe Ser Asp Ser Glu Asp Glu Gly Glu Gly Gly Arg Arg Asn
   515 520 525
   Val Ala Asp His Lys Lys Gly Ala Lys Lys Ala Arg Ile Glu Glu Asp
   530 535 540
   Lys Lys Glu Thr Glu Asp Lys Lys Thr Asp Val Lys Glu Glu Asp Lys
   545 550 555 560
   Ser Lys Asp Asn Ser Gly Glu Lys Thr Asp Thr Lys Gly Thr Lys Ser
   565 570 575
   Glu Gln Leu Ser Asn Pro
   580
<210> 3
   <211> 429
   <212> PRT
   <213> Homo sapiens
<400> 3
   Met Ile Val Phe Lys Pro Tyr Gln Ala Ser Gln His Asp Met Cys Arg
   1 5 10 15
   Phe His Tyr Gly Ala Gly His Pro Met Lys Pro His Arg Leu Ala Leu
   20 25 30
   Thr His Ser Leu Val Leu His Tyr Gly Leu Tyr Lys Lys Met Ile Val
   35 40 45
   Phe Lys Pro Tyr Gln Ala Ser Gln His Asp Met Cys Arg Phe His Ser
   50 55 60
   Glu Asp Tyr Ile Asp Phe Leu Gln Arg Val Ser Pro Thr Asn Met Gln
   65 70 75 80
   Gly Phe Thr Lys Ser Leu Asn Ala Phe Asn Val Gly Asp Asp Cys Pro
   85 90 95
   Val Phe Pro Gly Leu Phe Glu Phe Cys Ser Arg Tyr Thr Gly Ala Ser
   100 105 110
   Leu Gln Gly Ala Thr Gln Leu Asn Asn Lys Ile Cys Asp Ile Ala Ile
   115 120 125
   Asn Trp Ala Gly Gly Leu His His Ala Lys Lys Phe Glu Ala Ser Gly
   130 135 140
   Phe Cys Tyr Val Asn Asp Ile Val Ile Gly Ile Leu Glu Leu Leu Lys
   145                 150                 155                 160
   Tyr His Pro Arg Val Leu Tyr Ile Asp Ile Asp Ile His His Gly Asp
                   165                 170                 175
   Gly Val Gln Glu Ala Phe Tyr Leu Thr Asp Arg Val Met Thr Val Ser
               180                 185                 190
   Phe His Lys Tyr Gly Asn Tyr Phe Phe Pro Gly Thr Gly Asp Met Tyr
           195                 200                 205
   Glu Val Gly Ala Glu Ser Gly Arg Tyr Tyr Cys Leu Asn Val Pro Leu
       210                 215                 220
   Arg Asp Gly Ile Asp Asp Gln Ser Tyr Lys His Leu Phe Gln Pro Val
   225                 230                 235                 240
   Ile Asn Gln Val Val Asp Phe Tyr Gln Pro Thr Cys Ile Val Leu Gln
                   245                 250                 255
   Cys Gly Ala Asp Ser Leu Gly Cys Asp Arg Leu Gly Cys Phe Asn Leu
               260                 265                 270
   Ser Ile Arg Gly His Gly Glu Cys Val Glu Tyr Val Lys Ser Phe Asn
           275                 280                 285
   Ile Pro Leu Leu Val Leu Gly Gly Gly Gly Tyr Thr Val Arg Asn Val
       290                 295                 300
   Ala Arg Cys Trp Thr Tyr Glu Thr Ser Leu Leu Val Glu Glu Ala Ile
   305                 310                 315                 320
   Ser Glu Glu Leu Pro Tyr Ser Glu Tyr Phe Glu Tyr Phe Ala Pro Asp
                   325                 330                 335
   Phe Thr Leu His Pro Asp Val Ser Thr Arg Ile Glu Asn Gln Asn Ser
               340                 345                 350
   Arg Gln Tyr Leu Asp Gln Ile Arg Gln Thr Ile Phe Glu Asn Leu Lys
           355                 360                 365
   Met Leu Asn His Ala Pro Ser Val Gln Ile His Asp Val Pro Ala Asp
       370                 375                 380
   Leu Leu Thr Tyr Asp Arg Thr Asp Glu Ala Asp Ala Glu Glu Arg Gly
   385                 390                 395                 400
   Pro Glu Glu Asn Tyr Ser Arg Pro Glu Ala Pro Asn Glu Phe Tyr Asp
                   405                 410                 415
   Gly Asp His Asp Asn Asp Lys Glu Ser Asp Val Glu Ile
               420                 425
<210> 4
   <211> 1229
   <212> PRT
   <213> Homo sapiens
<400> 4
   Met Ser Gly Ala Asn Arg Gly Arg Gly Gln Ala Ser Ser Thr Met Thr
   1               5                   10                  15
   Ser Thr Gly Gln Asp Ser Thr Thr Thr Arg Gln Arg Arg Ser Arg Gln
               20                  25                  30
   Asn Pro Gln Ser Pro Pro Gln Asp Ser Ser Val Thr Ser Lys Arg Asn
           35                  40                  45
   Ile Lys Lys Gly Ala Val Pro Arg Ser Ile Pro Asn Leu Ala Glu Val
       50                  55                  60
   Lys Lys Lys Gly Lys Met Lys Lys Leu Gly Gln Ala Met Glu Glu Asp
   65                  70                  75                  80
   Leu Ile Val Gly Leu Gln Gly Met Asp Leu Asn Leu Glu Ala Glu Ala
                   85                  90                  95
   Leu Ala Gly Thr Gly Leu Val Leu Asp Glu Gln Leu Asn Glu Phe His
               100                 105                 110
   Cys Leu Trp Asp Asp Ser Phe Pro Glu Gly Pro Glu Arg Leu His Ala
           115                 120                 125
   Ile Lys Glu Gln Leu Ile Gln Glu Gly Leu Leu Asp Arg Cys Val Ser
       130                 135                 140
   Phe Gln Ala Arg Phe Ala Glu Lys Glu Glu Leu Met Leu Val His Ser
   145                 150                 155                 160
   Leu Glu Tyr Ile Asp Leu Met Glu Thr Thr Gln Tyr Met Asn Glu Gly
                   165                 170                 175
   Glu Leu Arg Val Leu Ala Asp Thr Tyr Asp Ser Val Tyr Leu His Pro
               180                 185                 190
   Asn Ser Tyr Ser Cys Ala Cys Leu Ala Ser Gly Ser Val Leu Arg Leu
           195                 200                 205
   Val Asp Ala Val Leu Gly Ala Glu Ile Arg Asn Gly Met Ala Ile Ile
       210                 215                 220
   Arg Pro Pro Gly His His Ala Gln His Ser Leu Met Asp Gly Tyr Cys
   225 230 235 240
   Met Phe Asn His Val Ala Val Ala Ala Arg Tyr Ala Gln Gln Lys His
   245 250 255
   Arg Ile Arg Arg Val Leu Ile Val Asp Trp Asp Val His His Gly Gln
   260 265 270
   Gly Thr Gln Phe Thr Phe Asp Gln Asp Pro Ser Val Leu Tyr Phe Ser
   275 280 285
   Ile His Arg Tyr Glu Gln Gly Arg Phe Trp Pro His Leu Lys Ala Ser
   290 295 300
   Asn Trp Ser Thr Thr Gly Phe Gly Gln Gly Gln Gly Tyr Thr Ile Asn
   305 310 315 320
   Val Pro Trp Asn Gln Val Gly Met Arg Asp Ala Asp Tyr Ile Ala Ala
   325 330 335
   Phe Leu His Val Leu Leu Pro Val Ala Leu Glu Phe Gln Pro Gln Leu
   340 345 350
   Val Leu Val Ala Ala Gly Phe Asp Ala Leu Gln Gly Asp Pro Lys Gly
   355 360 365
   Glu Met Ala Ala Thr Pro Ala Gly Phe Ala Gln Leu Thr His Leu Leu
   370 375 380
   Met Gly Leu Ala Gly Gly Lys Leu Ile Leu Ser Leu Glu Gly Gly Tyr
   385 390 395 400
   Asn Leu Arg Ala Leu Ala Glu Gly Val Ser Ala Ser Leu His Thr Leu
   405 410 415
   Leu Gly Asp Pro Cys Pro Met Leu Glu Ser Pro Gly Ala Pro Cys Arg
   420 425 430
   Ser Ala Gln Ala Ser Val Ser Cys Ala Leu Glu Ala Leu Glu Pro Phe
   435 440 445
   Trp Glu Val Leu Val Arg Ser Thr Glu Thr Val Glu Arg Asp Asn Met
   450 455 460
   Glu Glu Asp Asn Val Glu Glu Ser Glu Glu Glu Gly Pro Trp Glu Pro
   465 470 475 480
   Pro Val Leu Pro Ile Leu Thr Trp Pro Val Leu Gln Ser Arg Thr Gly
   485 490 495
   Leu Val Tyr Asp Gln Asn Met Met Asn His Cys Asn Leu Trp Asp Ser
               500                 505                 510
   His His Pro Glu Val Pro Gln Arg Ile Leu Arg Ile Met Cys Arg Leu
           515                 520                 525
   Glu Glu Leu Gly Leu Ala Gly Arg Cys Leu Thr Leu Thr Pro Arg Pro
       530                 535                 540
   Ala Thr Glu Ala Glu Leu Leu Thr Cys His Ser Ala Glu Tyr Val Gly
   545                 550                 555                 560
   His Leu Arg Ala Thr Glu Lys Met Lys Thr Arg Glu Leu His Arg Glu
                   565                 570                 575
   Ser Ser Asn Phe Asp Ser Ile Tyr Ile Cys Pro Ser Thr Phe Ala Cys
               580                 585                 590
   Ala Gln Leu Ala Thr Gly Ala Ala Cys Arg Leu Val Glu Ala Val Leu
           595                 600                 605
   Ser Gly Glu Val Leu Asn Gly Ala Ala Val Val Arg Pro Pro Gly His
       610                 615                 620
   His Ala Glu Gln Asp Ala Ala Cys Gly Phe Cys Phe Phe Asn Ser Val
   625                 630                 635                 640
   Ala Val Ala Ala Arg His Ala Gln Thr Ile Ser Gly His Ala Leu Arg
                   645                 650                 655
   Ile Leu Ile Val Asp Trp Asp Val His His Gly Asn Gly Thr Gln His
               660                 665                 670
   Met Phe Glu Asp Asp Pro Ser Val Leu Tyr Val Ser Leu His Arg Tyr
           675                 680                 685
   Asp His Gly Thr Phe Phe Pro Met Gly Asp Glu Gly Ala Ser Ser Gln
       690                 695                 700
   Ile Gly Arg Ala Ala Gly Thr Gly Phe Thr Val Asn Val Ala Trp Asn
   705                 710                 715                 720
   Gly Pro Arg Met Gly Asp Ala Asp Tyr Leu Ala Ala Trp His Arg Leu
                   725                 730                 735
   Val Leu Pro Ile Ala Tyr Glu Phe Asn Pro Glu Leu Val Leu Val Ser
               740                 745                 750
   Ala Gly Phe Asp Ala Ala Arg Gly Asp Pro Leu Gly Gly Cys Gln Val
           755                 760                 765
   Ser Pro Glu Gly Tyr Ala His Leu Thr His Leu Leu Met Gly Leu Ala
       770                 775                 780
   Ser Gly Arg Ile Ile Leu Ile Leu Glu Gly Gly Tyr Asn Leu Thr Ser
   785                 790                 795                 800
   Ile Ser Glu Ser Met Ala Ala Cys Thr Arg Ser Leu Leu Gly Asp Pro
                   805                 810                 815
   Pro Pro Leu Leu Thr Leu Pro Arg Pro Pro Leu Ser Gly Ala Leu Ala
               820                 825                 830
   Ser Ile Thr Glu Thr Ile Gln Val His Arg Arg Tyr Trp Arg Ser Leu
           835                 840                 845
   Arg Val Met Lys Val Glu Asp Arg Glu Gly Pro Ser Ser Ser Lys Leu
       850                 855                 860
   Val Thr Lys Lys Ala Pro Gln Pro Ala Lys Pro Arg Leu Ala Glu Arg
   865                 870                 875                 880
   Met Thr Thr Arg Glu Lys Lys Val Leu Glu Ala Gly Met Gly Lys Val
                   885                 890                 895
   Thr Ser Ala Ser Phe Gly Glu Glu Ser Thr Pro Gly Gln Thr Asn Ser
               900                 905                 910
   Glu Thr Ala Val Val Ala Leu Thr Gln Asp Gln Pro Ser Glu Ala Ala
           915                 920                 925
   Thr Gly Gly Ala Thr Leu Ala Gln Thr Ile Ser Glu Ala Ala Ile Gly
       930                 935                 940
   Gly Ala Met Leu Gly Gln Thr Thr Ser Glu Glu Ala Val Gly Gly Ala
   945                 950                 955                 960
   Thr Pro Asp Gln Thr Thr Ser Glu Glu Thr Val Gly Gly Ala Ile Leu
                   965                 970                 975
   Asp Gln Thr Thr Ser Glu Asp Ala Val Gly Gly Ala Thr Leu Gly Gln
               980                 985                 990
   Thr Thr Ser Glu Glu Ala Val Gly  Gly Ala Thr Leu Ala  Gln Thr Thr
           995                 1000                 1005
   Ser Glu  Ala Ala Met Glu Gly  Ala Thr Leu Asp Gln  Thr Thr Ser
       1010                 1015                 1020
   Glu Glu  Ala Pro Gly Gly Thr  Glu Leu Ile Gln Thr  Pro Leu Ala
       1025                 1030                 1035
   Ser Ser Thr Asp His Gln Thr Pro Pro Thr Ser Pro Val Gln Gly
   1040 1045 1050
   Thr Thr Pro Gln Ile Ser Pro Ser Thr Leu Ile Gly Ser Leu Arg
   1055 1060 1065
   Thr Leu Glu Leu Gly Ser Glu Ser Gln Gly Ala Ser Glu Ser Gln
   1070 1075 1080
   Ala Pro Gly Glu Glu Asn Leu Leu Gly Glu Ala Ala Gly Gly Gln
   1085 1090 1095
   Asp Met Ala Asp Ser Met Leu Met Gln Gly Ser Arg Gly Leu Thr
   1100 1105 1110
   Asp Gln Ala Ile Phe Tyr Ala Val Thr Pro Leu Pro Trp Cys Pro
   1115 1120 1125
   His Leu Val Ala Val Cys Pro Ile Pro Ala Ala Gly Leu Asp Val
   1130 1135 1140
   Thr Gln Pro Cys Gly Asp Cys Gly Thr Ile Gln Glu Asn Trp Val
   1145 1150 1155
   Cys Leu Ser Cys Tyr Gln Val Tyr Cys Gly Arg Tyr Ile Asn Gly
   1160 1165 1170
   His Met Leu Gln His His Gly Asn Ser Gly His Pro Leu Val Leu
   1175 1180 1185
   Ser Tyr Ile Asp Leu Ser Ala Trp Cys Tyr Tyr Cys Gln Ala Tyr
   1190 1195 1200
   Val His His Gln Ala Leu Leu Asp Val Lys Asn Ile Ala His Gln
   1205 1210 1215
   Asn Lys Phe Gly Glu Asp Met Pro His Pro His
   1220 1225
<210> 5
   <211> 377
   <212> PRT
   <213> Homo sapiens
<400> 5
<210> 6
   <211> 669
   <212> PRT
   <213> Homo sapiens
<400> 6
   Met Gly Thr Ala Leu Val Tyr His Glu Asp Met Thr Ala Thr Arg Leu
   1 5 10 15
   Leu Trp Asp Asp Pro Glu Cys Glu Ile Glu Arg Pro Glu Arg Leu Thr
   20 25 30
   Ala Ala Leu Asp Arg Leu Arg Gln Arg Gly Leu Glu Gln Arg Cys Leu
   35 40 45
   Arg Leu Ser Ala Arg Glu Ala Ser Glu Glu Glu Leu Gly Leu Val His
   50 55 60
   Ser Pro Glu Tyr Val Ser Leu Val Arg Glu Thr Gln Val Leu Gly Lys
   65 70 75 80
   Glu Glu Leu Gln Ala Leu Ser Gly Gln Phe Asp Ala Ile Tyr Phe His
   85 90 95
   Pro Ser Thr Phe His Cys Ala Arg Leu Ala Ala Gly Ala Gly Leu Gln
   100 105 110
   Leu Val Asp Ala Val Leu Thr Gly Ala Val Gln Asn Gly Leu Ala Leu
   115 120 125
   Val Arg Pro Pro Gly His His Gly Gln Arg Ala Ala Ala Asn Gly Phe
   130 135 140
   Cys Val Phe Asn Asn Val Ala Ile Ala Ala Ala His Ala Lys Gln Lys
   145 150 155 160
   His Gly Leu His Arg Ile Leu Val Val Asp Trp Asp Val His His Gly
   165 170 175
   Gln Gly Ile Gln Tyr Leu Phe Glu Asp Asp Pro Ser Val Leu Tyr Phe
   180 185 190
   Ser Trp His Arg Tyr Glu His Gly Arg Phe Trp Pro Phe Leu Arg Glu
   195 200 205
   Ser Asp Ala Asp Ala Val Gly Arg Gly Gln Gly Leu Gly Phe Thr Val
   210 215 220
   Asn Leu Pro Trp Asn Gln Val Gly Met Gly Asn Ala Asp Tyr Val Ala
   225 230 235 240
   Ala Phe Leu His Leu Leu Leu Pro Leu Ala Phe Glu Phe Asp Pro Glu
   245 250 255
   Leu Val Leu Val Ser Ala Gly Phe Asp Ser Ala Ile Gly Asp Pro Glu
   260 265 270
   Gly Gln Met Gln Ala Thr Pro Glu Cys Phe Ala His Leu Thr Gln Leu
   275 280 285
   Leu Gln Val Leu Ala Gly Gly Arg Val Cys Ala Val Leu Glu Gly Gly
   290 295 300
   Tyr His Leu Glu Ser Leu Ala Glu Ser Val Cys Met Thr Val Gln Thr
   305 310 315 320
   Leu Leu Gly Asp Pro Ala Pro Pro Leu Ser Gly Pro Met Ala Pro Cys
   325 330 335
   Gln Ser Ala Leu Glu Ser Ile Gln Ser Ala Arg Ala Ala Gln Ala Pro
   340 345 350
   His Trp Lys Ser Leu Gln Gln Gln Asp Val Thr Ala Val Pro Met Ser
   355 360 365
   Pro Ser Ser His Ser Pro Glu Gly Arg Pro Pro Pro Leu Leu Pro Gly
   370 375 380
   Gly Pro Val Cys Lys Ala Ala Ala Ser Ala Pro Ser Ser Leu Leu Asp
   385 390 395 400
   Gln Pro Cys Leu Cys Pro Ala Pro Ser Val Arg Thr Ala Val Ala Leu
   405 410 415
   Thr Thr Pro Asp Ile Thr Leu Val Leu Pro Pro Asp Val Ile Gln Gln
   420 425 430
   Glu Ala Ser Ala Leu Arg Glu Glu Thr Glu Ala Trp Ala Arg Pro His
   435 440 445
   Glu Ser Leu Ala Arg Glu Glu Ala Leu Thr Ala Leu Gly Lys Leu Leu
   450 455 460
   Tyr Leu Leu Asp Gly Met Leu Asp Gly Gln Val Asn Ser Gly Ile Ala
   465 470 475 480
   Ala Thr Pro Ala Ser Ala Ala Ala Ala Thr Leu Asp Val Ala Val Arg
   485 490 495
   Arg Gly Leu Ser His Gly Ala Gln Arg Leu Leu Cys Val Ala Leu Gly
   500 505 510
   Gln Leu Asp Arg Pro Pro Asp Leu Ala His Asp Gly Arg Ser Leu Trp
   515 520 525
   Leu Asn Ile Arg Gly Lys Glu Ala Ala Ala Leu Ser Met Phe His Val
   530 535 540
   Ser Thr Pro Leu Pro Val Met Thr Gly Gly Phe Leu Ser Cys Ile Leu
   545 550 555 560
   Gly Leu Val Leu Pro Leu Ala Tyr Gly Phe Gln Pro Asp Leu Val Leu
   565 570 575
   Val Ala Leu Gly Pro Gly His Gly Leu Gln Gly Pro His Ala Ala Leu
   580 585 590
   Leu Ala Ala Met Leu Arg Gly Leu Ala Gly Gly Arg Val Leu Ala Leu
   595 600 605
   Leu Glu Glu Asn Ser Thr Pro Gln Leu Ala Gly Ile Leu Ala Arg Val
   610 615 620
   Leu Asn Gly Glu Ala Pro Pro Ser Leu Gly Pro Ser Ser Val Ala Ser
   625 630 635 640
   Pro Glu Asp Val Gln Ala Leu Met Tyr Leu Arg Gly Gln Leu Glu Pro
   645 650 655
   Gln Trp Lys Met Leu Gln Cys His Pro His Leu Val Ala
   660 665
<210> 7
   <211> 876
   <212> PRT
   <213> Homo sapiens
<400> 7
<210> 8
   <211> 1217
   <212> PRT
   <213> Homo sapiens
<400> 8
   Met Lys Gly Met Ser His Glu Pro Lys Ser Pro Ser Leu Gly Met Leu
   1 5 10 15
   Ser Thr Ala Thr Arg Thr Thr Ala Thr Val Asn Pro Leu Thr Pro Ser
   20 25 30
   Pro Leu Asn Gly Ala Leu Val Pro Ser Gly Ser Pro Ala Thr Ser Ser
   35 40 45
   Ala Leu Ser Ala Gln Ala Ala Pro Ser Ser Ser Phe Ala Ala Ala Leu
   50 55 60
   Arg Lys Leu Ala Lys Gln Ala Glu Glu Pro Arg Gly Ser Ser Leu Ser
   65 70 75 80
   Ser Glu Ser Ser Pro Val Ser Ser Pro Ala Thr Asn His Ser Ser Pro
   85 90 95
   Ala Ser Thr Pro Lys Arg Val Pro Met Gly Pro Ile Ile Val Pro Pro
   100 105 110
   Gly Gly His Ser Val Pro Ser Thr Pro Pro Val Val Thr Ile Ala Pro
   115 120 125
   Thr Lys Thr Val Asn Gly Val Trp Arg Ser Glu Ser Arg Gln Asp Ala
   130 135 140
   Gly Ser Arg Ser Ser Ser Gly Gly Arg Glu Arg Leu Ile Val Glu Pro
   145 150 155 160
   Pro Leu Pro Gln Glu Lys Ala Gly Gly Pro Ala Ile Pro Ser His Leu
   165 170 175
   Leu Ser Thr Pro Tyr Pro Phe Gly Leu Ser Pro Ser Ser Val Val Gln
   180 185 190
   Asp Ser Arg Phe Pro Pro Leu Asn Leu Gln Arg Pro Val His His Val
   195 200 205
   Val Pro Pro Ser Thr Val Thr Glu Asp Tyr Leu Arg Ser Phe Arg Pro
   210 215 220
   Tyr His Thr Thr Asp Asp Leu Arg Met Ser Ser Leu Pro Pro Leu Gly
   225 230 235 240
   Leu Asp Pro Ala Thr Ala Ala Ala Tyr Tyr His Pro Ser Tyr Leu Ala
   245 250 255
   Pro His Pro Phe Pro His Pro Ala Phe Arg Met Asp Asp Ser Tyr Cys
   260 265 270
   Leu Ser Ala Leu Arg Ser Pro Phe Tyr Pro Ile Pro Thr Pro Gly Ser
   275 280 285
   Leu Pro Pro Leu His Pro Ser Ala Met His Leu His Leu Ser Gly Val
   290 295 300
   Arg Tyr Pro Pro Glu Leu Ser His Ser Ser Leu Ala Ala Leu His Ser
   305 310 315 320
   Glu Arg Met Ser Gly Leu Ser Ala Glu Arg Leu Gln Met Asp Glu Glu
   325 330 335
   Leu Arg Arg Glu Arg Glu Arg Glu Arg Glu Arg Glu Arg Glu Arg Glu
   340 345 350
   Ala Asp Arg Glu Arg Glu Lys Glu Arg Glu Arg Glu Arg Glu Lys Glu
   355 360 365
   Arg Glu Gln Glu Lys Glu Arg Glu Arg Glu Lys Glu Arg Glu Arg Glu
   370 375 380
   Leu Glu Arg Gln Arg Glu Gln Arg Ala Arg Glu Lys Glu Leu Leu Ala
   385 390 395 400
   Ala Lys Ala Leu Glu Pro Ser Phe Leu Pro Val Ala Glu Leu His Gly
   405 410 415
   Leu Arg Gly His Ala Thr Glu Glu Arg Gly Lys Pro Ser Glu Gln Leu
   420 425 430
   Thr Pro Thr Arg Ala Glu Lys Leu Lys Asp Ala Gly Leu Gln Ala Pro
   435 440 445
   Lys Pro Val Gln His Pro Leu His Pro Val Pro Thr Pro His His Thr
   450 455 460
   Val Pro Ser Leu Ile Ser Asn His Gly Ile Phe Ser Leu Pro Ser Ser
   465 470 475 480
   Ser Ala Ala Thr Ala Leu Leu Ile Gln Arg Thr Asn Glu Glu Glu Lys
   485 490 495
   Trp Leu Ala Arg Gln Arg Arg Leu Arg Gln Glu Lys Glu Asp Arg Gln
   500 505 510
   Ser Gln Val Ser Glu Phe Arg Gln Gln Val Leu Glu Gln His Leu Asp
   515 520 525
   Met Gly Arg Pro Pro Val Pro Ala Glu Ala Glu His Arg Pro Glu Ser
   530 535 540
   Thr Thr Arg Pro Gly Pro Asn Arg His Glu Pro Gly Gly Arg Asp Pro
   545 550 555 560
   Pro Gln His Phe Gly Gly Pro Pro Pro Leu Ile Ser Pro Lys Pro Gln
   565 570 575
   Leu His Ala Ala Pro Thr Ala Leu Trp Asn Pro Val Ser Leu Met Asp
   580 585 590
   Asn Thr Leu Glu Thr Arg Arg Ala Glu Ser His Ser Leu His Ser His
   595 600 605
   Pro Ala Ala Phe Glu Pro Ser Arg Gln Ala Ala Val Pro Leu Val Lys
   610 615 620
   Val Glu Arg Val Phe Cys Pro Glu Lys Ala Glu Glu Gly Pro Arg Lys
   625 630 635 640
   Arg Glu Pro Ala Pro Leu Asp Lys Tyr Gln Pro Pro Pro Pro Pro Pro
   645 650 655
   Arg Glu Gly Gly Ser Leu Glu His Gln Pro Phe Leu Pro Gly Pro Gly
   660 665 670
   Pro Phe Leu Ala Glu Leu Glu Lys Ser Thr Gln Thr Ile Leu Gly Gln
   675 680 685
   Gln Arg Ala Ser Leu Pro Gln Ala Ala Thr Phe Gly Glu Leu Ser Gly
   690 695 700
   Pro Leu Lys Pro Gly Ser Pro Tyr Arg Pro Pro Val Pro Arg Ala Pro
   705 710 715 720
   Asp Pro Ala Tyr Ile Tyr Asp Glu Phe Leu Gln Gln Arg Arg Arg Leu
   725 730 735
   Val Ser Lys Leu Asp Leu Glu Glu Arg Arg Arg Arg Glu Ala Gln Glu
   740 745 750
   Lys Gly Tyr Tyr Tyr Asp Leu Asp Asp Ser Tyr Asp Glu Ser Asp Glu
   755 760 765
   Glu Glu Val Arg Ala His Leu Arg Cys Val Ala Glu Gln Pro Pro Leu
   770 775 780
   Lys Leu Asp Thr Ser Ser Glu Lys Leu Glu Phe Leu Gln Leu Phe Gly
   785 790 795 800
   Leu Thr Thr Gln Gln Gln Lys Glu Glu Leu Val Ala Gln Lys Arg Arg
   805 810 815
   Lys Arg Arg Arg Met Leu Arg Glu Arg Ser Pro Ser Pro Pro Thr Ile
   820 825 830
   Gln Ser Lys Arg Gln Thr Pro Ser Pro Arg Leu Ala Leu Ser Thr Arg
   835 840 845
   Tyr Ser Pro Asp Glu Met Asn Asn Ser Pro Asn Phe Glu Glu Lys Lys
   850 855 860
   Lys Phe Leu Thr Ile Phe Asn Leu Thr His Ile Ser Ala Glu Lys Arg
   865 870 875 880
   Lys Asp Lys Glu Arg Leu Val Glu Met Leu Arg Ala Met Lys Gln Lys
   885 890 895
   Ala Leu Ser Ala Ala Val Ala Asp Ser Leu Thr Asn Ser Pro Arg Asp
   900 905 910
   Ser Pro Ala Val Ser Leu Ser Glu Pro Ala Thr Gln Gln Ala Ser Leu
   915 920 925
   Asp Val Glu Lys Pro Val Gly Val Ala Ala Ser Leu Ser Asp Ile Pro
   930 935 940
   Lys Ala Ala Glu Pro Gly Lys Leu Glu Gln Val Arg Pro Gln Glu Leu
   945 950 955 960
   Ser Arg Val Gln Glu Leu Ala Pro Ala Ser Gly Glu Lys Ala Arg Leu
   965 970 975
   Ser Glu Ala Pro Gly Gly Lys Lys Ser Leu Ser Met Leu His Tyr Ile
   980 985 990
   Arg Gly Ala Ala Pro Lys Asp Ile Pro Val Pro Leu Ser His Ser Thr
   995 1000 1005
   Asn Gly Lys Ser Lys Pro Trp Glu Pro Phe Val Ala Glu Glu Phe
   1010 1015 1020
   Ala His Gln Phe His Glu Ser Val Leu Gln Ser Thr Gln Lys Ala
   1025 1030 1035
   Leu Gln Lys His Lys Gly Ser Val Ala Val Leu Ser Ala Glu Gln
   1040 1045 1050
   Asn His Lys Val Asp Thr Ser Val His Tyr Asn Ile Pro Glu Leu
   1055 1060 1065
   Gln Ser Ser Ser Arg Ala Pro Pro Pro Gln His Asn Gly Gln Gln
   1070 1075 1080
   Glu Pro Pro Thr Ala Arg Lys Gly Pro Pro Thr Gln Glu Leu Asp
   1085 1090 1095
   Arg Asp Ser Glu Glu Glu Glu Glu Glu Asp Asp Glu Asp Gly Glu
   1100 1105 1110
   Asp Glu Glu Glu Val Pro Lys Arg Lys Trp Gln Gly Ile Glu Ala
   1115 1120 1125
   Val Phe Glu Ala Tyr Gln Glu His Ile Glu Glu Gln Asn Leu Glu
   1130 1135 1140
   Arg Gln Val Leu Gln Thr Gln Cys Arg Arg Leu Glu Ala Arg His
   1145 1150 1155
   Tyr Ser Leu Ser Leu Thr Ala Glu Gln Leu Ser His Ser Val Ala
   1160 1165 1170
   Glu Leu Arg Ser Gln Lys Gln Lys Met Val Ser Glu Arg Glu Arg
   1175 1180 1185
   Leu Gln Ala Glu Leu Asp His Leu Arg Lys Cys Leu Ala Leu Pro
   1190 1195 1200
   Ala Met His Trp Pro Arg Gly Tyr Leu Lys Gly Tyr Pro Arg
   1205 1210 1215
<210> 9
   <211> 347
   <212> PRT
   <213> Homo sapiens
<400> 9
   Met Glu Asn Leu Met Thr Ser Ser Thr Leu Pro Pro Leu Phe Ala Asp
   1 5 10 15
   Glu Asp Gly Ser Lys Glu Ser Asn Asp Leu Ala Thr Thr Gly Leu Asn
               20                  25                  30
   His Pro Glu Val Pro Tyr Ser Ser Gly Ala Thr Ser Ser Thr Asn Asn
           35                  40                  45
   Pro Glu Phe Val Glu Asp Leu Ser Gln Gly Gln Leu Leu Gln Ser Glu
       50                  55                  60
   Ser Ser Asn Ala Ala Glu Gly Asn Glu Gln Arg His Glu Asp Glu Gln
   65                  70                  75                  80
   Arg Ser Lys Arg Gly Gly Trp Ser Lys Gly Arg Lys Arg Lys Lys Pro
                   85                  90                  95
   Leu Arg Asp Ser Asn Ala Pro Lys Ser Pro Leu Thr Gly Tyr Val Arg
               100                 105                 110
   Phe Met Asn Glu Arg Arg Glu Gln Leu Arg Ala Lys Arg Pro Glu Val
           115                 120                 125
   Pro Phe Pro Glu Ile Thr Arg Met Leu Gly Asn Glu Trp Ser Lys Leu
       130                 135                 140
   Pro Pro Glu Glu Lys Gln Arg Tyr Leu Asp Glu Ala Asp Arg Asp Lys
   145                 150                 155                 160
   Glu Arg Tyr Met Lys Glu Leu Glu Gln Tyr Gln Lys Thr Glu Ala Tyr
                   165                 170                 175
   Lys Val Phe Ser Arg Lys Thr Gln Asp Arg Gln Lys Gly Lys Ser His
               180                 185                 190
   Arg Gln Asp Ala Ala Arg Gln Ala Thr His Asp His Glu Lys Glu Thr
           195                 200                 205
   Glu Val Lys Glu Arg Ser Val Phe Asp Ile Pro Ile Phe Thr Glu Glu
       210                 215                 220
   Phe Leu Asn His Ser Lys Ala Arg Glu Ala Glu Leu Arg Gln Leu Arg
   225                 230                 235                 240
   Lys Ser Asn Met Glu Phe Glu Glu Arg Asn Ala Ala Leu Gln Lys His
                   245                 250                 255
   Val Glu Ser Met Arg Thr Ala Val Glu Lys Leu Glu Val Asp Val Ile
               260                 265                 270
   Gln Glu Arg Ser Arg Asn Thr Val Leu Gln Gln His Leu Glu Thr Leu
           275                 280                 285
   Arg Gln Val Leu Thr Ser Ser Phe Ala Ser Met Pro Leu Pro Gly Ser
       290                 295                 300
   Gly Glu Thr Pro Thr Val Asp Thr Ile Asp Ser Tyr Met Asn Arg Leu
   305                 310                 315                 320
   His Ser Ile Ile Leu Ala Asn Pro Gln Asp Asn Glu Asn Phe Ile Ala
                   325                 330                 335
   Thr Val Arg Glu Val Val Asn Arg Leu Asp Arg
               340                 345
<210> 10
   <211> 317
   <212> PRT
   <213> Homo sapiens
<400> 10
   Met Ser His Gly Pro Lys Gln Pro Gly Ala Ala Ala Ala Pro Ala Gly
   1               5                   10                  15
   Gly Lys Ala Pro Gly Gln His Gly Gly Phe Val Val Thr Val Lys Gln
               20                  25                  30
   Glu Arg Gly Glu Gly Pro Arg Ala Gly Glu Lys Gly Ser His Glu Glu
           35                  40                  45
   Glu Pro Val Lys Lys Arg Gly Trp Pro Lys Gly Lys Lys Arg Lys Lys
       50                  55                  60
   Ile Leu Pro Asn Gly Pro Lys Ala Pro Val Thr Gly Tyr Val Arg Phe
   65                  70                  75                  80
   Leu Asn Glu Arg Arg Glu Gln Ile Arg Thr Arg His Pro Asp Leu Pro
                   85                  90                  95
   Phe Pro Glu Ile Thr Lys Met Leu Gly Ala Glu Trp Ser Lys Leu Gln
               100                 105                 110
   Pro Thr Glu Lys Gln Arg Tyr Leu Asp Glu Ala Glu Arg Glu Lys Gln
           115                 120                 125
   Gln Tyr Met Lys Glu Leu Arg Ala Tyr Gln Gln Ser Glu Ala Tyr Lys
       130                 135                 140
   Met Cys Thr Glu Lys Ile Gln Glu Lys Lys Ile Lys Lys Glu Asp Ser
   145                 150                 155                 160
   Ser Ser Gly Leu Met Asn Thr Leu Leu Asn Gly His Lys Gly Gly Asp
                   165                 170                 175
   Cys Asp Gly Phe Ser Thr Phe Asp Val Pro Ile Phe Thr Glu Glu Phe
               180                 185                 190
   Leu Asp Gln Asn Lys Ala Arg Glu Ala Glu Leu Arg Arg Leu Arg Lys
           195                 200                 205
   Met Asn Val Ala Phe Glu Glu Gln Asn Ala Val Leu Gln Arg His Thr
       210                 215                 220
   Gln Ser Met Ser Ser Ala Arg Glu Arg Leu Glu Gln Glu Leu Ala Leu
   225                 230                 235                 240
   Glu Glu Arg Arg Thr Leu Ala Leu Gln Gln Gln Leu Gln Ala Val Arg
                   245                 250                 255
   Gln Ala Leu Thr Ala Ser Phe Ala Ser Leu Pro Val Pro Gly Thr Gly
               260                 265                 270
   Glu Thr Pro Thr Leu Gly Thr Leu Asp Phe Tyr Met Ala Arg Leu His
           275                 280                 285
   Gly Ala Ile Glu Arg Asp Pro Ala Gln His Glu Lys Leu Ile Val Arg
       290                 295                 300
   Ile Lys Glu Ile Leu Ala Gln Val Ala Ser Glu His Leu
   305                 310                 315
<210> 11
   <211> 482
   <212> PRT
   <213> Homo sapiens
<400> 11
   Met Val Glu Lys Gly Pro Glu Val Ser Gly Lys Arg Arg Gly Arg Asn
   1               5                   10                  15
   Asn Ala Ala Ala Ser Ala Ser Ala Ala Ala Ala Ser Ala Ala Ala Ser
               20                  25                  30
   Ala Ala Cys Ala Ser Pro Ala Ala Thr Ala Ala Ser Gly Ala Ala Ala
           35                  40                  45
   Ser Ser Ala Ser Ala Ala Ala Ala Ser Ala Ala Ala Ala Pro Asn Asn
       50                  55                  60
   Gly Gln Asn Lys Ser Leu Ala Ala Ala Ala Pro Asn Gly Asn Ser Ser
   65                  70                  75                  80
   Ser Asn Ser Trp Glu Glu Gly Ser Ser Gly Ser Ser Ser Asp Glu Glu
                   85                  90                  95
   His Gly Gly Gly Gly Met Arg Val Gly Pro Gln Tyr Gln Ala Val Val
               100                 105                 110
   Pro Asp Phe Asp Pro Ala Lys Leu Ala Arg Arg Ser Gln Glu Arg Asp
           115                 120                 125
   Asn Leu Gly Met Leu Val Trp Ser Pro Asn Gln Asn Leu Ser Glu Ala
       130                 135                 140
   Lys Leu Asp Glu Tyr Ile Ala Ile Ala Lys Glu Lys His Gly Tyr Asn
   145                 150                 155                 160
   Met Glu Gln Ala Leu Gly Met Leu Phe Trp His Lys His Asn Ile Glu
                   165                 170                 175
   Lys Ser Leu Ala Asp Leu Pro Asn Phe Thr Pro Phe Pro Asp Glu Trp
               180                 185                 190
   Thr Val Glu Asp Lys Val Leu Phe Glu Gln Ala Phe Ser Phe His Gly
           195                 200                 205
   Lys Thr Phe His Arg Ile Gln Gln Met Leu Pro Asp Lys Ser Ile Ala
       210                 215                 220
   Ser Leu Val Lys Phe Tyr Tyr Ser Trp Lys Lys Thr Arg Thr Lys Thr
   225                 230                 235                 240
   Ser Val Met Asp Arg His Ala Arg Lys Gln Lys Arg Glu Arg Glu Glu
                   245                 250                 255
   Ser Glu Asp Glu Leu Glu Glu Ala Asn Gly Asn Asn Pro Ile Asp Ile
               260                 265                 270
   Glu Val Asp Gln Asn Lys Glu Ser Lys Lys Glu Val Pro Pro Thr Glu
           275                 280                 285
   Thr Val Pro Gln Val Lys Lys Glu Lys His Ser Thr Gln Ala Lys Asn
       290                 295                 300
   Arg Ala Lys Arg Lys Pro Pro Lys Gly Met Phe Leu Ser Gln Glu Asp
   305                 310                 315                 320
   Val Glu Ala Val Ser Ala Asn Ala Thr Ala Ala Thr Thr Val Leu Arg
                   325                 330                 335
   Gln Leu Asp Met Glu Leu Val Ser Val Lys Arg Gln Ile Gln Asn Ile
               340                 345                 350
   Lys Gln Thr Asn Ser Ala Leu Lys Glu Lys Leu Asp Gly Gly Ile Glu
           355                 360                 365
   Pro Tyr Arg Leu Pro Glu Val Ile Gln Lys Cys Asn Ala Arg Trp Thr
       370                 375                 380
   Thr Glu Glu Gln Leu Leu Ala Val Gln Ala Ile Arg Lys Tyr Gly Arg
   385 390 395 400
   Asp Phe Gln Ala Ile Ser Asp Val Ile Gly Asn Lys Ser Val Val Gln
   405 410 415
   Val Lys Asn Phe Phe Val Asn Tyr Arg Arg Arg Phe Asn Ile Asp Glu
   420 425 430
   Val Leu Gln Glu Trp Glu Ala Glu His Gly Lys Glu Glu Thr Asn Gly
   435 440 445
   Pro Ser Asn Gln Lys Pro Val Lys Ser Pro Asp Asn Ser Ile Lys Met
   450 455 460
   Pro Glu Glu Glu Asp Glu Ala Pro Val Leu Asp Val Arg Tyr Ala Ser
   465 470 475 480
   Ala Ser
<210> 12
   <211> 553
   <212> PRT
   <213> Homo sapiens
<400> 12
   Met Pro Gly Met Met Glu Lys Gly Pro Glu Leu Leu Gly Lys Asn Arg
   1 5 10 15
   Ser Ala Asn Gly Ser Ala Lys Ser Pro Ala Gly Gly Gly Gly Ser Gly
   20 25 30
   Ala Ser Ser Thr Asn Gly Gly Leu His Tyr Ser Glu Pro Glu Ser Gly
   35 40 45
   Cys Ser Ser Asp Asp Glu His Asp Val Gly Met Arg Val Gly Ala Glu
   50 55 60
   Tyr Gln Ala Arg Ile Pro Glu Phe Asp Pro Gly Ala Thr Lys Tyr Thr
   65 70 75 80
   Asp Lys Asp Asn Gly Gly Met Leu Val Trp Ser Pro Tyr His Ser Ile
   85 90 95
   Pro Asp Ala Lys Leu Asp Glu Tyr Ile Ala Ile Ala Lys Glu Lys His
   100 105 110
   Gly Tyr Asn Val Glu Gln Ala Leu Gly Met Leu Phe Trp His Lys His
   115 120 125
   Asn Ile Glu Lys Ser Leu Ala Asp Leu Pro Asn Phe Thr Pro Phe Pro
       130                 135                 140
   Asp Glu Trp Thr Val Glu Asp Lys Val Leu Phe Glu Gln Ala Phe Ser
   145                 150                 155                 160
   Phe His Gly Lys Ser Phe His Arg Ile Gln Gln Met Leu Pro Asp Lys
                   165                 170                 175
   Thr Ile Ala Ser Leu Val Lys Tyr Tyr Tyr Ser Trp Lys Lys Thr Arg
               180                 185                 190
   Ser Arg Thr Ser Leu Met Asp Arg Gln Ala Arg Lys Leu Ala Asn Arg
           195                 200                 205
   His Asn Gln Gly Asp Ser Asp Asp Asp Val Glu Glu Thr His Pro Met
       210                 215                 220
   Asp Gly Asn Asp Ser Asp Tyr Asp Pro Lys Lys Glu Ala Lys Lys Glu
   225                 230                 235                 240
   Gly Asn Thr Glu Gln Pro Val Gln Thr Ser Lys Ile Gly Leu Gly Arg
                   245                 250                 255
   Arg Glu Tyr Gln Ser Leu Gln His Arg His His Ser Gln Arg Ser Lys
               260                 265                 270
   Cys Arg Pro Pro Lys Gly Met Tyr Leu Thr Gln Glu Asp Val Val Ala
           275                 280                 285
   Val Ser Cys Ser Pro Asn Ala Ala Asn Thr Ile Leu Arg Gln Leu Asp
       290                 295                 300
   Met Glu Leu Ile Ser Leu Lys Arg Gln Val Gln Asn Ala Lys Gln Val
   305                 310                 315                 320
   Asn Ser Ala Leu Lys Gln Lys Met Glu Gly Gly Ile Glu Glu Phe Lys
                   325                 330                 335
   Pro Pro Glu Ser Asn Gln Lys Ile Asn Ala Arg Trp Thr Thr Glu Glu
               340                 345                 350
   Gln Leu Leu Ala Val Gln Gly Val Arg Lys Tyr Gly Lys Asp Phe Gln
           355                 360                 365
   Ala Ile Ala Asp Val Ile Gly Asn Lys Thr Val Gly Gln Val Lys Asn
       370                 375                 380
   Phe Phe Val Asn Tyr Arg Arg Arg Phe Asn Leu Glu Glu Val Leu Gln
   385                 390                 395                 400
   Glu Trp Glu Ala Glu Gln Gly Thr Gln Ala Ser Asn Gly Asp Ala Ser
   405 410 415
   Thr Leu Gly Glu Glu Thr Lys Ser Ala Ser Asn Val Pro Ser Gly Lys
   420 425 430
   Ser Thr Asp Glu Glu Glu Glu Ala Gln Thr Pro Gln Ala Pro Arg Thr
   435 440 445
   Leu Gly Pro Ser Pro Pro Ala Pro Ser Ser Thr Pro Thr Pro Thr Ala
   450 455 460
   Pro Ile Ala Thr Leu Asn Gln Pro Pro Pro Leu Leu Arg Pro Thr Leu
   465 470 475 480
   Pro Ala Ala Pro Ala Leu His Arg Gln Pro Pro Pro Leu Gln Gln Gln
   485 490 495
   Ala Arg Phe Ile Gln Pro Arg Pro Thr Leu Asn Gln Pro Pro Pro Pro
   500 505 510
   Leu Ile Arg Pro Ala Asn Ser Met Pro Pro Arg Leu Asn Pro Arg Pro
   515 520 525
   Val Leu Ser Thr Val Gly Gly Gln Gln Pro Pro Ser Leu Ile Gly Ile
   530 535 540
   Gln Thr Asp Ser Gln Ser Ser Leu His
   545 550
<210> 13
   <211> 1379
   <212> PRT
   <213> Homo sapiens
<400> 13
<210> 14
   <211> 653
   <212> PRT
   <213> Homo sapiens
<400> 14
   Met Ser Leu Trp Pro Trp Pro Thr Gly Arg Thr Ser Thr Pro Tyr Pro
   1               5                   10                  15
   Glu Ala Arg Met Thr Glu Glu Ala Cys Arg Thr Arg Ser Gln Lys Arg
               20                  25                  30
   Ala Leu Glu Arg Asp Pro Thr Glu Asp Asp Val Glu Ser Lys Lys Ile
           35                  40                  45
   Lys Met Glu Arg Gly Leu Leu Ala Ser Asp Leu Asn Thr Asp Gly Asp
       50                  55                  60
   Met Arg Val Thr Pro Glu Pro Gly Ala Gly Pro Thr Gln Gly Leu Leu
   65                  70                  75                  80
   Arg Ala Thr Glu Ala Thr Ala Met Ala Met Gly Arg Gly Glu Gly Leu
                   85                  90                  95
   Val Gly Asp Gly Pro Val Asp Met Arg Thr Ser His Ser Asp Met Lys
               100                 105                 110
   Ser Glu Arg Arg Pro Pro Ser Pro Asp Val Ile Val Leu Ser Asp Asn
           115                 120                 125
   Glu Gln Pro Ser Ser Pro Arg Val Asn Gly Leu Thr Thr Val Ala Leu
       130                 135                 140
   Lys Glu Thr Ser Thr Glu Ala Leu Met Lys Ser Ser Pro Glu Glu Arg
   145                 150                 155                 160
   Glu Arg Met Ile Lys Gln Leu Lys Glu Glu Leu Arg Leu Glu Glu Ala
                   165                 170                 175
   Lys Leu Val Leu Leu Lys Lys Leu Arg Gln Ser Gln Ile Gln Lys Glu
               180                 185                 190
   Ala Thr Ala Gln Lys Pro Thr Gly Ser Val Gly Ser Thr Val Thr Thr
           195                 200                 205
   Pro Pro Pro Leu Val Arg Gly Thr Gln Asn Ile Pro Ala Gly Lys Pro
       210                 215                 220
   Ser Leu Gln Thr Ser Ser Ala Arg Met Pro Gly Ser Val Ile Pro Pro
   225                 230                 235                 240
   Pro Leu Val Arg Gly Gly Gln Gln Ala Ser Ser Lys Leu Gly Pro Gln
                   245                 250                 255
   Ala Ser Ser Gln Val Val Met Pro Pro Leu Val Arg Gly Ala Gln Gln
               260                 265                 270
   Ile His Ser Ile Arg Gln His Ser Ser Thr Gly Pro Pro Pro Leu Leu
   275 280 285
   Leu Ala Pro Arg Ala Ser Val Pro Ser Val Gln Ile Gln Gly Gln Arg
   290 295 300
   Ile Ile Gln Gln Gly Leu Ile Arg Val Ala Asn Val Pro Asn Thr Ser
   305 310 315 320
   Leu Leu Val Asn Ile Pro Gln Pro Thr Pro Ala Ser Leu Lys Gly Thr
   325 330 335
   Thr Ala Thr Ser Ala Gln Ala Asn Ser Thr Pro Thr Ser Val Ala Ser
   340 345 350
   Val Val Thr Ser Ala Glu Ser Pro Ala Ser Arg Gln Ala Ala Ala Lys
   355 360 365
   Leu Ala Leu Arg Lys Gln Leu Glu Lys Thr Leu Leu Glu Ile Pro Pro
   370 375 380
   Pro Lys Pro Pro Ala Pro Glu Met Asn Phe Leu Pro Ser Ala Ala Asn
   385 390 395 400
   Asn Glu Phe Ile Tyr Leu Val Gly Leu Glu Glu Val Val Gln Asn Leu
   405 410 415
   Leu Glu Thr Gln Ala Gly Arg Met Ser Ala Ala Thr Val Leu Ser Arg
   420 425 430
   Glu Pro Tyr Met Cys Ala Gln Cys Lys Thr Asp Phe Thr Cys Arg Trp
   435 440 445
   Arg Glu Glu Lys Ser Gly Ala Ile Met Cys Glu Asn Cys Met Thr Thr
   450 455 460
   Asn Gln Lys Lys Ala Leu Lys Val Glu His Thr Ser Arg Leu Lys Ala
   465 470 475 480
   Ala Phe Val Lys Ala Leu Gln Gln Glu Gln Glu Ile Glu Gln Arg Leu
   485 490 495
   Leu Gln Gln Gly Thr Ala Pro Ala Gln Ala Lys Ala Glu Pro Thr Ala
   500 505 510
   Ala Pro His Pro Val Leu Lys Gln Val Ile Lys Pro Arg Arg Lys Leu
   515 520 525
   Ala Phe Arg Ser Gly Glu Ala Arg Asp Trp Ser Asn Gly Ala Val Leu
   530 535 540
   Gln Ala Ser Ser Gln Leu Ser Arg Gly Ser Ala Thr Thr Pro Arg Gly
   545 550 555 560
   Val Leu His Thr Phe Ser Pro Ser Pro Lys Leu Gln Asn Ser Ala Ser
   565 570 575
   Ala Thr Ala Leu Val Ser Arg Thr Gly Arg His Ser Glu Arg Thr Val
   580 585 590
   Ser Ala Gly Lys Gly Ser Ala Thr Ser Asn Trp Lys Lys Thr Pro Leu
   595 600 605
   Ser Thr Gly Gly Thr Leu Ala Phe Val Ser Pro Ser Leu Ala Val His
   610 615 620
   Lys Ser Ser Ser Ala Val Asp Arg Gln Arg Glu Tyr Leu Leu Asp Met
   625 630 635 640
   Ile Pro Pro Arg Ser Ile Pro Gln Ser Ala Thr Trp Lys
   645 650
<210> 15
   <211> 593
   <212> PRT
   <213> Homo sapiens
<400> 15
<210> 16
   <211> 291
   <212> PRT
   <213> Homo sapiens
<400> 16
   Met Glu Arg Lys Arg Trp Glu Cys Pro Ala Leu Pro Gln Gly Trp Glu
   1 5 10 15
   Arg Glu Glu Val Pro Arg Arg Ser Gly Leu Ser Ala Gly His Arg Asp
   20 25 30
   Val Phe Tyr Tyr Ser Pro Ser Gly Lys Lys Phe Arg Ser Lys Pro Gln
   35 40 45
   Leu Ala Arg Tyr Leu Gly Gly Ser Met Asp Leu Ser Thr Phe Asp Phe
       50                  55                  60
   Arg Thr Gly Lys Met Leu Met Ser Lys Met Asn Lys Ser Arg Gln Arg
   65                  70                  75                  80
   Val Arg Tyr Asp Ser Ser Asn Gln Val Lys Gly Lys Pro Asp Leu Asn
                   85                  90                  95
   Thr Ala Leu Pro Val Arg Gln Thr Ala Ser Ile Phe Lys Gln Pro Val
               100                 105                 110
   Thr Lys Ile Thr Asn His Pro Ser Asn Lys Val Lys Ser Asp Pro Gln
           115                 120                 125
   Lys Ala Val Asp Gln Pro Arg Gln Leu Phe Trp Glu Lys Lys Leu Ser
       130                 135                 140
   Gly Leu Asn Ala Phe Asp Ile Ala Glu Glu Leu Val Lys Thr Met Asp
   145                 150                 155                 160
   Leu Pro Lys Gly Leu Gln Gly Val Gly Pro Gly Cys Thr Asp Glu Thr
                   165                 170                 175
   Leu Leu Ser Ala Ile Ala Ser Ala Leu His Thr Ser Thr Met Pro Ile
               180                 185                 190
   Thr Gly Gln Leu Ser Ala Ala Val Glu Lys Asn Pro Gly Val Trp Leu
           195                 200                 205
   Asn Thr Thr Gln Pro Leu Cys Lys Ala Phe Met Val Thr Asp Glu Asp
       210                 215                 220
   Ile Arg Lys Gln Glu Glu Leu Val Gln Gln Val Arg Lys Arg Leu Glu
   225                 230                 235                 240
   Glu Ala Leu Met Ala Asp Met Leu Ala His Val Glu Glu Leu Ala Arg
                   245                 250                 255
   Asp Gly Glu Ala Pro Leu Asp Lys Ala Cys Ala Glu Asp Asp Asp Glu
               260                 265                 270
   Glu Asp Glu Glu Glu Glu Glu Glu Glu Pro Asp Pro Asp Pro Glu Met
           275                 280                 285
   Glu His Val
       290
<210> 17
   <211> 668
   <212> PRT
   <213> Homo sapiens
<400> 17
   Met Ala Ala Asn Met Tyr Arg Val Gly Asp Tyr Val Tyr Phe Glu Asn
   1               5                   10                  15
   Ser Ser Ser Asn Pro Tyr Leu Val Arg Arg Ile Glu Glu Leu Asn Lys
               20                  25                  30
   Thr Ala Asn Gly Asn Val Glu Ala Lys Val Val Cys Leu Phe Arg Arg
           35                  40                  45
   Arg Asp Ile Ser Ser Ser Leu Asn Ser Leu Ala Asp Ser Asn Ala Arg
       50                  55                  60
   Glu Phe Glu Glu Glu Ser Lys Gln Pro Gly Val Ser Glu Gln Gln Arg
   65                  70                  75                  80
   His Gln Leu Lys His Arg Glu Leu Phe Leu Ser Arg Gln Phe Glu Ser
                   85                  90                  95
   Leu Pro Ala Thr His Ile Arg Gly Lys Cys Ser Val Thr Leu Leu Asn
               100                 105                 110
   Glu Thr Asp Ile Leu Ser Gln Tyr Leu Glu Lys Glu Asp Cys Phe Phe
           115                 120                 125
   Tyr Ser Leu Val Phe Asp Pro Val Gln Lys Thr Leu Leu Ala Asp Gln
       130                 135                 140
   Gly Glu Ile Arg Val Gly Cys Lys Tyr Gln Ala Glu Ile Pro Asp Arg
   145                 150                 155                 160
   Leu Val Glu Gly Glu Ser Asp Asn Arg Asn Gln Gln Lys Met Glu Met
                   165                 170                 175
   Lys Val Trp Asp Pro Asp Asn Pro Leu Thr Asp Arg Gln Ile Asp Gln
               180                 185                 190
   Phe Leu Val Val Ala Arg Ala Val Gly Thr Phe Ala Arg Ala Leu Asp
           195                 200                 205
   Cys Ser Ser Ser Ile Arg Gln Pro Ser Leu His Met Ser Ala Ala Ala
       210                 215                 220
   Ala Ser Arg Asp Ile Thr Leu Phe His Ala Met Asp Thr Leu Gln Arg
   225                 230                 235                 240
   Asn Gly Tyr Asp Leu Ala Lys Ala Met Ser Thr Leu Val Pro Gln Gly
                   245                 250                 255
   Gly Pro Val Leu Cys Arg Asp Glu Met Glu Glu Trp Ser Ala Ser Glu
               260                 265                 270
   Ala Met Leu Phe Glu Glu Ala Leu Glu Lys Tyr Gly Lys Asp Phe Asn
           275                 280                 285
   Asp Ile Arg Gln Asp Phe Leu Pro Trp Lys Ser Leu Ala Ser Ile Val
       290                 295                 300
   Gln Phe Tyr Tyr Met Trp Lys Thr Thr Asp Arg Tyr Ile Gln Gln Lys
   305                 310                 315                 320
   Arg Leu Lys Ala Ala Glu Ala Asp Ser Lys Leu Lys Gln Val Tyr Ile
                   325                 330                 335
   Pro Thr Tyr Thr Lys Pro Asn Pro Asn Gln Ile Ile Ser Val Gly Ser
               340                 345                 350
   Lys Pro Gly Met Asn Gly Ala Gly Phe Gln Lys Gly Leu Thr Cys Glu
           355                 360                 365
   Ser Cys His Thr Thr Gln Ser Ala Gln Trp Tyr Ala Trp Gly Pro Pro
       370                 375                 380
   Asn Met Gln Cys Arg Leu Cys Ala Ser Cys Trp Ile Tyr Trp Lys Lys
   385                 390                 395                 400
   Tyr Gly Gly Leu Lys Thr Pro Thr Gln Leu Glu Gly Ala Thr Arg Gly
                   405                 410                 415
   Thr Thr Glu Pro His Ser Arg Gly His Leu Ser Arg Pro Glu Ala Gln
               420                 425                 430
   Ser Leu Ser Pro Tyr Thr Thr Ser Ala Asn Arg Ala Lys Leu Leu Ala
           435                 440                 445
   Lys Asn Arg Gln Thr Phe Leu Leu Gln Thr Thr Lys Leu Thr Arg Leu
       450                 455                 460
   Ala Arg Arg Met Cys Arg Asp Leu Leu Gln Pro Arg Arg Ala Ala Arg
   465                 470                 475                 480
   Arg Pro Tyr Ala Pro Ile Asn Ala Asn Ala Ile Lys Ala Glu Cys Ser
                   485                 490                 495
   Ile Arg Leu Pro Lys Ala Ala Lys Thr Pro Leu Lys Ile His Pro Leu
               500                 505                 510
   Val Arg Leu Pro Leu Ala Thr Ile Val Lys Asp Leu Val Ala Gln Ala
           515                 520                 525
   Pro Leu Lys Pro Lys Thr Pro Arg Gly Thr Lys Thr Pro Ile Asn Arg
       530                 535                 540
   Asn Gln Leu Ser Gln Asn Arg Gly Leu Gly Gly Ile Met Val Lys Arg
   545                 550                 555                 560
   Ala Tyr Glu Thr Met Ala Gly Ala Gly Val Pro Phe Ser Ala Asn Gly
                   565                 570                 575
   Arg Pro Leu Ala Ser Gly Ile Arg Ser Ser Ser Gln Pro Ala Ala Lys
               580                 585                 590
   Arg Gln Lys Leu Asn Pro Ala Asp Ala Pro Asn Pro Val Val Phe Val
           595                 600                 605
   Ala Thr Lys Asp Thr Arg Ala Leu Arg Lys Ala Leu Thr His Leu Glu
       610                 615                 620
   Met Arg Arg Ala Ala Arg Arg Pro Asn Leu Pro Leu Lys Val Lys Pro
   625                 630                 635                 640
   Thr Leu Ile Ala Val Arg Pro Pro Val Pro Leu Pro Ala Pro Ser His
                   645                 650                 655
   Pro Ala Ser Thr Asn Glu Pro Ile Val Leu Glu Asp
               660                 665
<210> 18
   <211> 594
   <212> PRT
   <213> Homo sapiens
<400> 18
   Met Ala Ala Asn Met Tyr Arg Val Gly Asp Tyr Val Tyr Phe Glu Asn
   1               5                   10                  15
   Ser Ser Ser Asn Pro Tyr Leu Ile Arg Arg Ile Glu Glu Leu Asn Lys
               20                  25                  30
   Thr Ala Ser Gly Asn Val Glu Ala Lys Val Val Cys Phe Tyr Arg Arg
           35                  40                  45
   Arg Asp Ile Ser Asn Thr Leu Ile Met Leu Ala Asp Lys His Ala Lys
       50                  55                  60
   Glu Ile Glu Glu Glu Ser Glu Thr Thr Val Glu Ala Asp Leu Thr Asp
   65                  70                  75                  80
   Lys Gln Lys His Gln Leu Lys His Arg Glu Leu Phe Leu Ser Arg Gln
                   85                  90                  95
   Tyr Glu Ser Leu Pro Ala Thr His Ile Arg Gly Lys Cys Ser Val Ala
   100 105 110
   Leu Leu Asn Glu Thr Glu Ser Val Leu Ser Tyr Leu Asp Lys Glu Asp
   115 120 125
   Thr Phe Phe Tyr Ser Leu Val Tyr Asp Pro Ser Leu Lys Thr Leu Leu
   130 135 140
   Ala Asp Lys Gly Glu Ile Arg Val Gly Pro Arg Tyr Gln Ala Asp Ile
   145 150 155 160
   Pro Glu Met Leu Leu Glu Gly Glu Ser Asp Glu Arg Glu Gln Ser Lys
   165 170 175
   Leu Glu Val Lys Val Trp Asp Pro Asn Ser Pro Leu Thr Asp Arg Gln
   180 185 190
   Ile Asp Gln Phe Leu Val Val Ala Arg Ala Val Gly Thr Phe Ala Arg
   195 200 205
   Ala Leu Asp Cys Ser Ser Ser Val Arg Gln Pro Ser Leu His Met Ser
   210 215 220
   Ala Ala Ala Ala Ser Arg Asp Ile Thr Leu Phe His Ala Met Asp Thr
   225 230 235 240
   Leu Tyr Arg His Ser Tyr Asp Leu Ser Ser Ala Ile Ser Val Leu Val
   245 250 255
   Pro Leu Gly Gly Pro Val Leu Cys Arg Asp Glu Met Glu Glu Trp Ser
   260 265 270
   Ala Ser Glu Ala Ser Leu Phe Glu Glu Ala Leu Glu Lys Tyr Gly Lys
   275 280 285
   Asp Phe Asn Asp Ile Arg Gln Asp Phe Leu Pro Trp Lys Ser Leu Thr
   290 295 300
   Ser Ile Ile Glu Tyr Tyr Tyr Met Trp Lys Thr Thr Asp Arg Tyr Val
   305 310 315 320
   Gln Gln Lys Arg Leu Lys Ala Ala Glu Ala Glu Ser Lys Leu Lys Gln
   325 330 335
   Val Tyr Ile Pro Thr Tyr Ser Lys Pro Asn Pro Asn Gln Ile Ser Thr
   340 345 350
   Ser Asn Gly Lys Pro Gly Ala Val Asn Gly Ala Val Gly Thr Thr Phe
   355 360 365
   Gln Pro Gln Asn Pro Leu Leu Gly Arg Ala Cys Glu Ser Cys Tyr Ala
   370 375 380
   Thr Gln Ser His Gln Trp Tyr Ser Trp Gly Pro Pro Asn Met Gln Cys
   385 390 395 400
   Arg Leu Cys Ala Ile Cys Trp Leu Tyr Trp Lys Lys Tyr Gly Gly Leu
   405 410 415
   Lys Met Pro Thr Gln Ser Glu Glu Glu Lys Leu Ser Pro Ser Pro Thr
   420 425 430
   Thr Glu Asp Pro Arg Val Arg Ser His Val Ser Arg Gln Ala Met Gln
   435 440 445
   Gly Met Pro Val Arg Asn Thr Gly Ser Pro Lys Ser Ala Val Lys Thr
   450 455 460
   Arg Gln Ala Phe Phe Leu His Thr Thr Tyr Phe Thr Lys Phe Ala Arg
   465 470 475 480
   Gln Val Cys Lys Asn Thr Leu Arg Leu Arg Gln Ala Ala Arg Arg Pro
   485 490 495
   Phe Val Ala Ile Asn Tyr Ala Ala Ile Arg Ala Glu Tyr Ala Asp Arg
   500 505 510
   His Ala Glu Leu Ser Gly Ser Pro Leu Lys Ser Lys Ser Thr Arg Lys
   515 520 525
   Pro Leu Ala Cys Ile Ile Gly Tyr Leu Glu Ile His Pro Ala Lys Lys
   530 535 540
   Pro Asn Val Ile Arg Ser Thr Pro Ser Leu Gln Thr Pro Thr Thr Lys
   545 550 555 560
   Arg Met Leu Thr Thr Pro Asn His Thr Ser Leu Ser Ile Leu Gly Lys
   565 570 575
   Arg Asn Tyr Ser His His Asn Gly Leu Asp Glu Leu Thr Cys Cys Val
   580 585 590
   Ser Asp
<210> 19
   <211> 1214
   <212> PRT
   <213> Homo sapiens
<400> 19
<210> 20
   <211> 577
   <212> PRT
   <213> Homo sapiens
<400> 20
   Met Thr Glu Leu Ala Gly Ala Ser Ser Ser Cys Cys His Arg Pro Ala
   1 5 10 15
   Gly Arg Gly Ala Met Gln Ser Val Leu His His Phe Gln Arg Leu Arg
   20 25 30
   Gly Arg Glu Gly Gly Ser His Phe Ile Asn Thr Ser Ser Pro Arg Gly
   35 40 45
   Glu Ala Lys Met Ser Ile Thr Ser Asp Glu Val Asn Phe Leu Val Tyr
   50 55 60
   Arg Tyr Leu Gln Glu Ser Gly Phe Ser His Ser Ala Phe Thr Phe Gly
   65 70 75 80
   Ile Glu Ser His Ile Ser Gln Ser Asn Ile Asn Gly Thr Leu Val Pro
   85 90 95
   Pro Ala Ala Leu Ile Ser Ile Leu Gln Lys Gly Leu Gln Tyr Val Glu
   100 105 110
   Ala Glu Ile Ser Ile Asn Glu Asp Gly Thr Val Phe Asp Gly Arg Pro
   115 120 125
   Ile Glu Ser Leu Ser Leu Ile Asp Ala Val Met Pro Asp Val Val Gln
   130 135 140
   Thr Arg Gln Gln Ala Phe Arg Glu Lys Leu Ala Gln Gln Gln Ala Ser
   145 150 155 160
   Ala Ala Ala Ala Ala Ala Ala Ala Thr Ala Ala Ala Thr Ala Ala Thr
   165 170 175
   Thr Thr Ser Ala Gly Val Ser His Gln Asn Pro Ser Lys Asn Arg Glu
   180 185 190
   Ala Thr Val Asn Gly Glu Glu Asn Arg Ala His Ser Val Asn Asn His
   195 200 205
   Ala Lys Pro Met Glu Ile Asp Gly Glu Val Glu Ile Pro Ser Ser Lys
   210 215 220
   Ala Thr Val Leu Arg Gly His Glu Ser Glu Val Phe Ile Cys Ala Trp
   225 230 235 240
   Asn Pro Val Ser Asp Leu Leu Ala Ser Gly Ser Gly Asp Ser Thr Ala
   245 250 255
   Arg Ile Trp Asn Leu Asn Glu Asn Ser Asn Gly Gly Ser Thr Gln Leu
   260 265 270
   Val Leu Arg His Cys Ile Arg Glu Gly Gly His Asp Val Pro Ser Asn
   275 280 285
   Lys Asp Val Thr Ser Leu Asp Trp Asn Thr Asn Gly Thr Leu Leu Ala
   290 295 300
   Thr Gly Ser Tyr Asp Gly Phe Ala Arg Ile Trp Thr Glu Asp Gly Asn
   305 310 315 320
   Leu Ala Ser Thr Leu Gly Gln His Lys Gly Pro Ile Phe Ala Leu Lys
   325 330 335
   Trp Asn Arg Lys Gly Asn Tyr Ile Leu Ser Ala Gly Val Asp Lys Thr
   340 345 350
   Thr Ile Ile Trp Asp Ala His Thr Gly Glu Ala Lys Gln Gln Phe Pro
   355 360 365
   Phe His Ser Ala Pro Ala Leu Asp Val Asp Trp Gln Asn Asn Thr Thr
   370 375 380
   Phe Ala Ser Cys Ser Thr Asp Met Cys Ile His Val Cys Arg Leu Gly
   385 390 395 400
   Cys Asp Arg Pro Val Lys Thr Phe Gln Gly His Thr Asn Glu Val Asn
   405 410 415
   Ala Ile Lys Trp Asp Pro Ser Gly Met Leu Leu Ala Ser Cys Ser Asp
   420 425 430
   Asp Met Thr Leu Lys Ile Trp Ser Met Lys Gln Glu Val Cys Ile His
   435 440 445
   Asp Leu Gln Ala His Asn Lys Glu Ile Tyr Thr Ile Lys Trp Ser Pro
   450 455 460
   Thr Gly Pro Ala Thr Ser Asn Pro Asn Ser Asn Ile Met Leu Ala Ser
   465 470 475 480
   Ala Ser Phe Asp Ser Thr Val Arg Leu Trp Asp Ile Glu Arg Gly Val
   485 490 495
   Cys Thr His Thr Leu Thr Lys His Gln Glu Pro Val Tyr Ser Val Ala
   500 505 510
   Phe Ser Pro Asp Gly Lys Tyr Leu Ala Ser Gly Ser Phe Asp Lys Cys
   515 520 525
   Val His Ile Trp Asn Thr Gln Ser Gly Asn Leu Val His Ser Tyr Arg
   530 535 540
   Gly Thr Gly Gly Ile Phe Glu Val Cys Trp Asn Ala Arg Gly Asp Lys
   545 550 555 560
   Val Gly Ala Ser Ala Ser Asp Gly Ser Val Cys Val Leu Asp Leu Arg
   565 570 575
   Lys
<210> 21
   <211> 514
   <212> PRT
   <213> Homo sapiens
<400> 21
<210> 22
   <211> 1273
   <212> PRT
   <213> Homo sapiens
<400> 22
<210> 23
   <211> 425
   <212> PRT
   <213> Homo sapiens
<400> 23
   Met Ala Asp Lys Glu Ala Ala Phe Asp Asp Ala Val Glu Glu Arg Val
   1 5 10 15
   Ile Asn Glu Glu Tyr Lys Ile Trp Lys Lys Asn Thr Pro Phe Leu Tyr
   20 25 30
   Asp Leu Val Met Thr His Ala Leu Glu Trp Pro Ser Leu Thr Ala Gln
   35 40 45
   Trp Leu Pro Asp Val Thr Arg Pro Glu Gly Lys Asp Phe Ser Ile His
   50 55 60
   Arg Leu Val Leu Gly Thr His Thr Ser Asp Glu Gln Asn His Leu Val
   65                  70                  75                  80
   Ile Ala Ser Val Gln Leu Pro Asn Asp Asp Ala Gln Phe Asp Ala Ser
                   85                  90                  95
   His Tyr Asp Ser Glu Lys Gly Glu Phe Gly Gly Phe Gly Ser Val Ser
               100                 105                 110
   Gly Lys Ile Glu Ile Glu Ile Lys Ile Asn His Glu Gly Glu Val Asn
           115                 120                 125
   Arg Ala Arg Tyr Met Pro Gln Asn Pro Cys Ile Ile Ala Thr Lys Thr
       130                 135                 140
   Pro Ser Ser Asp Val Leu Val Phe Asp Tyr Thr Lys His Pro Ser Lys
   145                 150                 155                 160
   Pro Asp Pro Ser Gly Glu Cys Asn Pro Asp Leu Arg Leu Arg Gly His
                   165                 170                 175
   Gln Lys Glu Gly Tyr Gly Leu Ser Trp Asn Pro Asn Leu Ser Gly His
               180                 185                 190
   Leu Leu Ser Ala Ser Asp Asp His Thr Ile Cys Leu Trp Asp Ile Ser
           195                 200                 205
   Ala Val Pro Lys Glu Gly Lys Val Val Asp Ala Lys Thr Ile Phe Thr
       210                 215                 220
   Gly His Thr Ala Val Val Glu Asp Val Ser Trp His Leu Leu His Glu
   225                 230                 235                 240
   Ser Leu Phe Gly Ser Val Ala Asp Asp Gln Lys Leu Met Ile Trp Asp
                   245                 250                 255
   Thr Arg Ser Asn Asn Thr Ser Lys Pro Ser His Ser Val Asp Ala His
               260                 265                 270
   Thr Ala Glu Val Asn Cys Leu Ser Phe Asn Pro Tyr Ser Glu Phe Ile
           275                 280                 285
   Leu Ala Thr Gly Ser Ala Asp Lys Thr Val Ala Leu Trp Asp Leu Arg
       290                 295                 300
   Asn Leu Lys Leu Lys Leu His Ser Phe Glu Ser His Lys Asp Glu Ile
   305                 310                 315                 320
   Phe Gln Val Gln Trp Ser Pro His Asn Glu Thr Ile Leu Ala Ser Ser
                   325                 330                 335
   Gly Thr Asp Arg Arg Leu Asn Val Trp Asp Leu Ser Lys Ile Gly Glu
               340                 345                 350
   Glu Gln Ser Pro Glu Asp Ala Glu Asp Gly Pro Pro Glu Leu Leu Phe
           355                 360                 365
   Ile His Gly Gly His Thr Ala Lys Ile Ser Asp Phe Ser Trp Asn Pro
       370                 375                 380
   Asn Glu Pro Trp Val Ile Cys Ser Val Ser Glu Asp Asn Ile Met Gln
   385                 390                 395                 400
   Val Trp Gln Met Ala Glu Asn Ile Tyr Asn Asp Glu Asp Pro Glu Gly
                   405                 410                 415
   Ser Val Asp Pro Glu Gly Gln Gly Ser
               420                 425
<210> 24
   <211> 469
   <212> PRT
   <213> Homo sapiens
<400> 24
   Met Ala Ala Glu Ala Gly Val Val Gly Ala Gly Ala Ser Pro Asp Gly
   1               5                   10                  15
   Asp Trp Arg Asp Gln Ala Cys Gly Leu Leu Leu His Val His Leu Ser
               20                  25                  30
   Ser Arg Leu Gly Arg Ala Ala Pro Val Arg Thr Gly Arg His Leu Arg
           35                  40                  45
   Thr Val Phe Glu Asp Thr Val Glu Glu Arg Val Ile Asn Glu Glu Tyr
       50                  55                  60
   Lys Ile Trp Lys Lys Asn Thr Pro Phe Leu Tyr Asp Leu Val Met Thr
   65                  70                  75                  80
   His Ala Leu Gln Trp Pro Ser Leu Thr Val Gln Trp Leu Pro Glu Val
                   85                  90                  95
   Thr Lys Pro Glu Gly Lys Asp Tyr Ala Leu His Trp Leu Val Leu Gly
               100                 105                 110
   Thr His Thr Ser Asp Glu Gln Asn His Leu Val Val Ala Arg Val His
           115                 120                 125
   Ile Pro Asn Asp Asp Ala Gln Phe Asp Ala Ser His Cys Asp Ser Asp
       130                 135                 140
   Lys Gly Glu Phe Gly Gly Phe Gly Ser Val Thr Gly Lys Ile Glu Cys
   145 150 155 160
   Glu Ile Lys Ile Asn His Glu Gly Glu Val Asn Arg Ala Arg Tyr Met
   165 170 175
   Pro Gln Asn Pro His Ile Ile Ala Thr Lys Thr Pro Ser Ser Asp Val
   180 185 190
   Leu Val Phe Asp Tyr Thr Lys His Pro Ala Lys Pro Asp Pro Ser Gly
   195 200 205
   Glu Cys Asn Pro Asp Leu Arg Leu Arg Gly His Gln Lys Glu Gly Tyr
   210 215 220
   Gly Leu Ser Trp Asn Ser Asn Leu Ser Gly His Leu Leu Ser Ala Ser
   225 230 235 240
   Asp Asp His Thr Val Cys Leu Trp Asp Ile Asn Ala Gly Pro Lys Glu
   245 250 255
   Gly Lys Ile Val Asp Ala Lys Ala Ile Phe Thr Gly His Ser Ala Val
   260 265 270
   Val Glu Asp Val Ala Trp His Leu Leu His Glu Ser Leu Phe Gly Ser
   275 280 285
   Val Ala Asp Asp Gln Lys Leu Met Ile Trp Asp Thr Arg Ser Asn Thr
   290 295 300
   Thr Ser Lys Pro Ser His Leu Val Asp Ala His Thr Ala Glu Val Asn
   305 310 315 320
   Cys Leu Ser Phe Asn Pro Tyr Ser Glu Phe Ile Leu Ala Thr Gly Ser
   325 330 335
   Ala Asp Lys Thr Val Ala Leu Trp Asp Leu Arg Asn Leu Lys Leu Lys
   340 345 350
   Leu His Thr Phe Glu Ser His Lys Asp Glu Ile Phe Gln Val His Trp
   355 360 365
   Ser Pro His Asn Glu Thr Ile Leu Ala Ser Ser Gly Thr Asp Arg Arg
   370 375 380
   Leu Asn Val Trp Asp Leu Ser Lys Ile Gly Glu Glu Gln Ser Ala Glu
   385 390 395 400
   Asp Ala Glu Asp Gly Pro Pro Glu Leu Leu Phe Ile His Gly Gly His
   405 410 415
   Thr Ala Lys Ile Ser Asp Phe Ser Trp Asn Pro Asn Glu Pro Trp Val
               420                 425                 430
   Ile Cys Ser Val Ser Glu Asp Asn Ile Met Gln Ile Trp Gln Met Ala
           435                 440                 445
   Glu Asn Ile Tyr Asn Asp Glu Glu Ser Asp Val Thr Thr Ser Glu Leu
       450                 455                 460
   Glu Gly Gln Gly Ser
   465

## Description

The present invention relates to methods for the identification and characterization (e.g. selectivity profiling) of HDAC interacting compounds.

The approval of the drug vorinostat (Zolinza, Merck) by the FDA for the treatment of cutaneous T-cell lymphoma in October 2006 significantly increased the interest in developing inhibitors for the class of enzymes known as histone deacetylases (HDACs). The discovery of the HDAC inhibitor vorinostat (suberoylanilide hydroxamic acid; SAHA) resulted from efforts to improve first-generation hybrid polar compounds - originally derived from DMSO - that acted as differentiation inducers of transformed cells. Subsequently, it was discovered that SAHA potently inhibits several HDACs, for example HDAC1, HDAC2, HDAC3 and HDAC6. Thus, SAHA represents a non-selective HDAC inhibitor and is sometimes referred to as a "pan-HDAC" inhibitor. The mechanisms underlying SAHA's anticancer activity are complex and not fully characterized. For example, SAHA induces the accumulation of acetylated histones with an effect on gene expression. In addition, non-histone proteins are substrates for HDACs and the effects on these proteins could contribute to its anticancer activities (Marks and Breslow, 2007. Nature Biotechnology 25(1):84-90; Grant et al., 2007. Nature Reviews Drug Discovery 6, 21-22).

HDACs catalyse the removal of acetyl groups from lysine residues in histone amino termini, leading to chromatin condensation and changes in gene expression. In humans 18 HDACs have been identified and subdivided into four classes based on their homology to yeast HDACs, their subcellular localization and their enzymatic activities. The class I HDACs (1, 2, 3 and 8) are homologous to the yeast RPD3 protein, can generally be detected in the nucleus and show ubiquitous expression in human cell lines and tissues. Class II HDACs (4, 5, 6, 7, 9 and 10) are similar to the yeast Hda proteins and can shuttle between the nucleus and the cytoplasm. HDAC6 can deacetylate the cytoskeletal protein α-tubulin. The class III HDACs (SIRT1, 2, 3, 4, 5, 6 and 7) are homologues of the yeast protein Sir2 and require NAD⁺ for their activity. HDAC11 is the sole member of class IV HDACs (Bolden et al., 2006. Nat. Rev. Drug Discov. 5(9):769-784).

Histone acetylation is a dynamic process controlled by HDACs and histone acetyltransferases (HATs) and the balance between these two processes together with other protein and DNA modifications such as DNA methylation regulates chromatin accessibility and gene expression. These reversible heritable changes in gene function occur without a change in the sequence of nuclear DNA and are therefore referred to as epigenetic gene regulation. HDACs play a key role in developmental processes, normal physiology and disease states. The ubiquitous expression and high homology between class I HDACs suggests functional redundancy among the HDACs in vivo. However, deletion of each member of the class I HDAC family leads to lethality, indicating a unique role of each HDAC in development (Haberland et al., 2009. Nat. Rev. Genet. 10(1):32-42).

Typically HDACs are present within multisubunit protein complexes together with other components that determine HDAC target gene specificity due to interactions with sequence-specific DNA-binding proteins (Cunliffe 2008. Curr. Opin. Genet. Dev. 18(5):404-410; Yang and Seto, 2008. Nat. Rev. Mol. Cell Biol. 9(3):206-218). Class I HDACs are found primarily in four distinct multiprotein complexes known as the Sin3, NuRD, CoREST and NCoR/SMRT complexes.

Mammalians have two Sin3 homologues, Sin3A and Sin3B. The Sin3A complex contains as components HDAC1, HDAC2, RbAp46 (retinoblastoma (RB) associated protein-46) and RbAp48. In addition, the complex contains RBP1 (Rb-binding protein-1), SAP180 (which is similar to RBP1), SDS3, BRMS1 (breast cancer metastasis suppressor-1, similar to SDS3), SAP30,SAP18 and ING1 (or ING2). Because some of the subunits have different isoforms, there are several distinct Sin3Acomplexes. The Sin3B complex shares some similarity to the Sin3Acomplex but might also contain distinct subunits (Yang and Seto, 2008. Nat. Rev. Mol. Cell Biol. 9(3):206-218).

The nucleosome remodeling and deacetylase (NuRD) complex contains a catalytic core of HDAC1, HDAC2, RbAp46 and RbAp48. In addition, this complex contains MTA (metastasis-associated) proteins, the nucleosome-remodelling ATPase Mi-2 (dermatomyositis-specific autoantigen), MBD2 (methyl CpG-binding domain-2) and p66. Through MBD2, this complex is recruited for DNA methylation-dependent gene silencing.

Three mammalian MTA isoforms (MTA1, -2 and -3) have been identified that contain SANT domains, which are crucial for the integrity and deacetylase activity of NuRD. Mi-2 and p66 have α- and β-isoforms, enabling the formation of distinct complexes.

The CoREST complex contains the corepressor protein CoREST interacting with the transcriptional repressor REST, HDAC1 and HDAC2. This complex is also referred to as neuronal corepressor complex because the REST zinc finger protein to target sites in the promoters of neuronal genes. One subunit encodes the Lys-specific demethylase-1 (LSD1) protein which functions as a demethylase for K4-methylated histone H3.

The NCoR/SNRT complex contains the SANT-domain-containing co-repressors SMRT, NCoR and SMRTER and is targeted to DNA by transcription factor such as CSL and nuclear receptors.

Several HDAC inhibitors are in preclinical development and clinical trials for the treatment of a wide variety of diseases including cancer, inflammatory, cardiac, and neurodegenerative diseases (Bolden et al., 2006. Nat. Rev. Drug Discov. 5(9):769-784; Haberland et al., 2009. Nat. Rev. Genet. 10(1):32-42). It is expected that the development of selective HDAC inhibitors targeting only one member of the HDAC family should lead to improved efficacy and drug safety compared to non-selective "pan-HDAC inhibitors" (Kalin et al., 2009. Curr. Opin. Chem. Biol. 13:1-9; Balasubramanian et al., 2009. Cancer Lett. 280(2):211-21).

The majority *of in vitro* studies of HDAC activity are performed using *in vitro* biochemical assays. These assays are also used for the identification of HDAC inhibitors (Hauser et al., 2009. Curr. Top. Med. Chem. 9(3):227-234). For example, HDAC activity can be measured using purified recombinant enzyme in solution-based assays with acetylated peptide substrates (Blackwell et al., 2008. Life Sciences 82(21-22):1050-1058).

Typically, these assays require the availability of purified or recombinant HDACs. However, not all HDACs can be produced will sufficient enzymatic activity to allow for inhibitor screening (Blackwell et al., 2008. Life Sciences 82(21-22):1050-1058). In addition, some preparations of HDACs expressed in insect cells are contaminated with endogenous insect HDACs making the interpretation of assay results ambiguous.

Another, although not in all instances necessary prerequisite for the identification of selective HDAC inhibitors is a method that allows to determine the target selectivity of these molecules. For example, it can be intended to provide molecules that bind to and inhibit a particular drug target but do not interact with a closely related target, inhibition of which could lead to unwanted side effects. Conventionally, large panels of individual enzyme assays are used to assess the inhibitory effect of a compound for HDACs (Khan et al., 2008. Biochem. J. 409(2):581-9; Blackwell et al., 2008. Life Sci. 82(21-22):1050-1058).

More recently, chemical proteomics methods have been described using acitivity-based probes that irreversibly bind to HDACs (Salisbury and Cravatt, 2007. PNAS 104(4):1171-1176; Salisbury and Cravatt, 2008. J. Am. Chem. Soc. 130(7):2184-2194).

In view of the above, there is a need for providing effective tools and methods for the identification and selectivity profiling of HDAC interacting compounds.

In a first aspect, the present invention relates to a method for the identification of a histone deacetylase (HDAC) interacting compound, comprising the steps of
a) providing an HDAC protein complex containing protein preparation derived from a cell endogenously expressing an HDAC,
b) contacting the protein preparation with a non-proteinaceous HDAC ligand immobilized on a solid support and with a given compound thereby allowing the reversible binding of said ligand to said HDAC protein complex, and
c) determining to what extent in step b) a binding between the ligand and the protein complex has occurred.

In a second aspect, the present invention relates to a method for the identification of a histone deacetylase (HDAC) interacting compound, comprising the steps of
a) providing two aliquots of an HDAC protein complex containing protein preparation derived from a cell endogenously expressing an HDAC,
b) contacting one aliquot with a non-proteinaceous HDAC ligand immobilized on a solid support thereby allowing the reversible binding of said ligand to said HDAC protein complex,
c) contacting the other aliquot with a non-proteinaceous HDAC ligand immobilized on a solid support and with a given compound thereby allowing the reversible binding of said ligand to said HDAC protein complex, and
d) determining to what extent in steps b) and c) a binding between the ligand and the protein complex has occurred.

In a third aspect, the present invention relates to a method for the identification of a histone deacetylase (HDAC) interacting compound, comprising the steps of:
a) providing two aliquots of a cell preparation each comprising at least one cell endogenously expressing an HDAC,
b) incubating one aliquot with a given compound,
c) harvesting the cells of each aliquot,
d) lysing the cells of each aliquot in order to obtain protein preparations containing an HDAC protein complex,
e) contacting the protein preparations with a non-proteinaceous HDAC ligand immobilized on a solid support thereby allowing the reversible binding of said ligand to said HDAC protein complex, and
f) determining to what extent in step e) a binding between the ligand and the protein complex has occurred.

In a fourth aspect, the present invention relates to a method for the identification of a histone deacetylase (HDAC) interacting compound, comprising the steps of
a) providing at least two aliquots of an HDAC containing protein preparation derived from a cell endogenously expressing an HDAC,
b) contacting one aliquot with a non-proteinaceous HDAC ligand immobilized on a solid support thereby allowing the reversible binding of said ligand to HDAC,
c) contacting the remaining aliquots with an HDAC ligand immobilized on a solid support and with various concentrations of a compound thereby allowing the reversible binding of said ligand to HDAC,
d) separating the HDAC from the the ligand, and
e) determining by mass spectrometry or immunodetection the amount of HDAC eluted from the ligand, thereby determining to what extent in steps b) and c) a binding between the ligand and the protein has occurred.

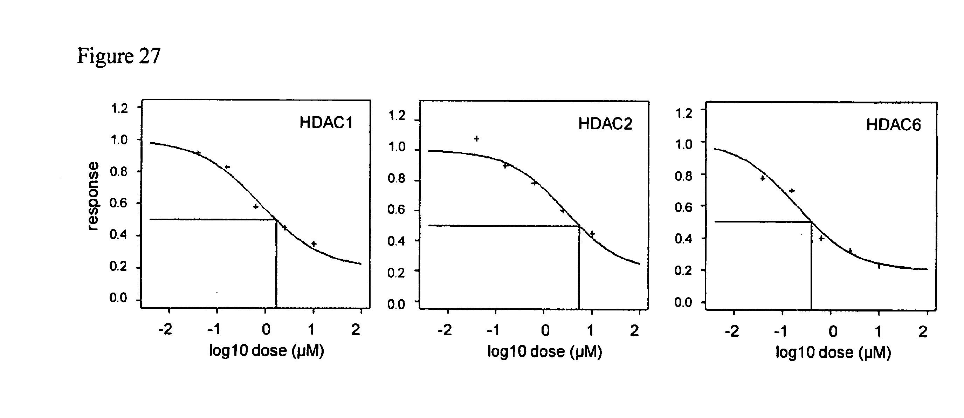

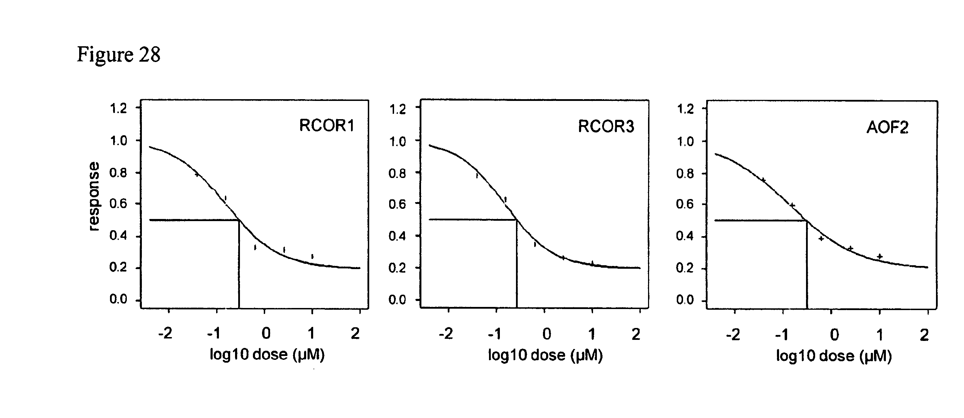

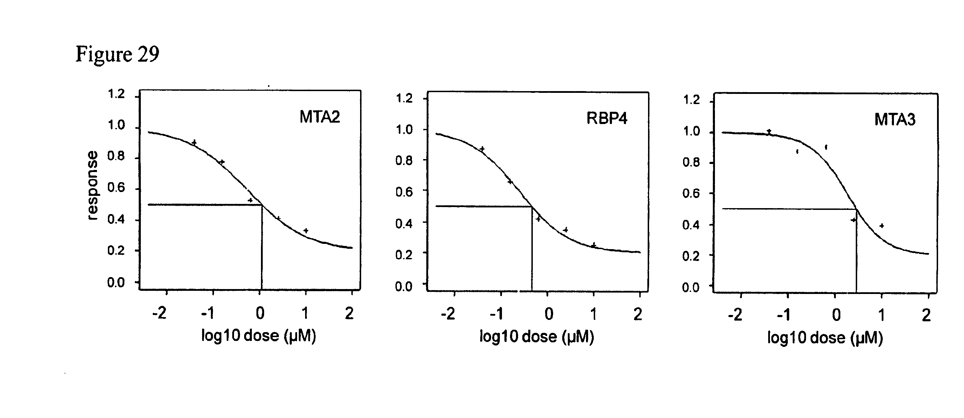

In the context of the present invention, it has been found that the methods of the present invention are suitable for the identification of compounds interacting with HDACs. The methods according to the first three aspects of the invention are especially suitable because they rely on the use of HDAC complexes which should ensure that the HDAC is present as much as possible in its natural environment. The method according to the fourth aspect of the invention has been found especially suitable in that the binding characteristics of known HDAC inhibitors could be very well characterized using this method of the invention. Especially, as shown in Examples 6 and 8 with the help of said method of the invention, it is possible to determine the IC₅₀ values of known HDAC interacting compounds yielding different selectivity profiles compared to selectivity profiles based on methods known in the art. Furthermore, Examples 2 to 5 (see also Tables 6 to 9 and Figures 27 to 29) demonstrate that, with the help of said method of the invention, it is possible to detect a high variety of components of HDAC-containing protein complexes.

According to the present invention, the expression "HDAC" or "histone deacetylase" means enzymes that remove acetyl groups from histones or other substrate proteins. These enzymes are known in the art.

Examples of HDACs are:
Class I HDACs (HDAC1, HDAC2, HDAC3, HDAC8);
class IIa HDAC (HDAC4, HDAC5, HDAC7, HDAC9);
class IIb HDAC (HDAC6, HDAC10);
class IV HDAC (HDAC11).

Each of them can be used in the context of the present invention. Preferred examples are HDAC1, HDAC2, HDAC3, HDAC6, HDAC8 and HDAC10.

According to the present invention, the expression "HDAC" relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 µg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

Moreover, according to the present invention, the expression "HDAC" includes mutant forms said HDACs.

In the aspects of the invention, first a protein preparation containing said HDAC (see the fourth aspect of the invention) or HDAC complex (see the first three aspects of the invention) is provided.

The methods of the present invention can be performed with any protein preparation as a starting material, as long as the respective HDAC or HDAC complex is present in the preparation and is derived from a cell endogenously expressing an HDAC. Examples include a liquid mixture of several proteins, a cell lysate, a partial cell lysate which contains not all proteins present in the original cell or a combination of several cell lysates. The term "protein preparation" also includes dissolved purified protein.

In the context of the present invention, the term "endogenously" means that the respective cell expresses said HDAC without being transfected with an HDAC-encoding nucleic acid. This ensures that the HDAC is present, as much as possible, in its natural environment, especially is contained in a naturally occurring HDAC protein complex as discussed above.

In another aspect of the invention, aliquots of a cell preparation are provided as the starting material. In the context of the present invention, the term "cell preparation" refers to any preparation containing at least one cell with the desired properties. Suitable cell preparations are described below.

The presence of the HDACs in a protein preparation of interest can be detected on Western blots probed with antibodies that are specifically directed against said HDAC. Alternatively, also mass spectrometry (MS) could be used to detect the HDACs (see below).

The presence of a given HDAC complex can be e.g. determined by determining whether, in a given protein preparation, HDAC is complexed to another known component of said complex. Corresponding methods are well known in the art and include co-immunoprecipitation or purification of HDAC under conditions allowing that the HDAC complex is not dissociated.

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

In a preferred embodiment of the methods of the invention, the provision of a protein preparation includes the steps of harvesting at least one cell containing the HDAC or the HDAC protein complex and lysing the cell.

Suitable cells for this purpose as well as for the cell preparations used as the starting material in one aspect of the present invention are those cells or tissues where the HDACs are endogenously expressed. In any given cell or tissue only a subset of the HDACs may be expressed. Therefore it may be necessary to generate multiple protein preparations from a variety of cell types and tissues to cover the HDAC family, especially for selectivity profiling of HDAC inhibitors. As established cell lines may not reflect the physiological expression pattern of HDACs, primary animal or human cells may be used, for example cells isolated from blood samples.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection).

As an alternative to primary human cells cultured cell lines (e.g. MOLT-4 cells, Jurkat, Ramos, HeLa or K-562 cells) can be used.

In a preferred embodiment, the cell is part of a cell culture system and methods for the harvest of a cell out of a cell culture system are known in the art (literature supra).

The choice of the cell will mainly depend on the expression of the HDACs, since it has to be ensured that the protein is principally present in the cell of choice. In order to determine whether a given cell is a suitable starting system for the methods of the invention, methods like Westernblot, PCR-based nucleic acids detection methods, Northemblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. B-cells). By contrast, for the elucidation of protein targets mediating unwanted side effects the cell or tissue may be analysed in which the side effect is observed (e.g. cardiomyocytes, vascular smooth muscle or epithelium cells).

Furthermore, it is envisaged within the present invention that the cell containing the HDACs or the HDAC complex may be obtained from an organism, e.g. by biopsy. Corresponding methods are known in the art. For example, a biopsy is a diagnostic procedure used to obtain a small amount of tissue, which can then be examined microscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing the HDAC or the HDAC protein complex is contacted with an HDAC ligand thereby allowing the reversible binding of the HDAC complex or the HDAC to the ligand.

In the context of the present invention, the term "HDAC ligand" denotes every molecule being able to bind to HDAC. Preferably, the HDAC ligand is a small molecule as defined below.

In the present invention, the term "allowing the reversible binding" means that reversibly a complex is formed between the HDAC complex or the HDAC and the ligand. Conditions allowing the reversible binding of molecules to proteins or protein complexes are known in the art. The skilled person will know which conditions can be applied in order to enable the formation of said complex.

In the context of the present invention, compounds are identified which interfere with the binding between the HDAC ligand and an HDAC or an HDAC protein complex present in a cell or protein preparation.

According to the aspects of the invention as defined above, the HDAC ligand is a non-proteinaceous ligand and is immobilized on a solid support.

Throughout the invention, the term "solid support" relates to every undissolved support being able to immobilize a small molecule ligand on its surface. The solid support may be selected from the group consisting of agarose, modified agarose, sepharose beads (e.g. NHS-activated sepharose), latex, cellulose, and ferro- or ferrimagnetic particles.

Methods and strategies for choosing appropriate solid supports and for coupling compounds to said solid supports are known in the art (see e.g. Wong, Shan S. Chemistry of protein conjugation and cross-linking (1991), CRC Press, Inc. ISBN 0-8493-5886-8 Chapter 12: Conjugation of proteins to solid matrices, pages 295-318).

The HDAC ligand may be coupled to the solid support either covalently or non-covalently. Non-covalent binding includes binding via biotin affinity ligands binding to steptavidin matrices.

Preferably, the HDAC ligand is covalently coupled to the solid support.

Methods for immobilizing compounds on solid supports are known in the art. In general, before the coupling, the matrixes can contain active groups such as NHS, Carbodimide etc. to enable the coupling reaction with the immobilization compound. The ligand can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the ligand and non-covalent binding of biotin to streptavidin which is bound directly to the solid support). The linkage to the solid support material may involve cleavable and non-cleavable linkers. The cleavage may be achieved by enzymatic cleavage or treatment with suitable chemical methods.

Consequently, in case that the HDAC ligand is immobilized on a solid support, the term "allowing the reversible binding" includes all conditions under which such binding is possible. This includes the possibility of having the solid support on an immobilized phase and pouring the lysate onto it. In another preferred embodiment, it is also included that the solid support is in a particulate form and mixed with the cell lysate. Such conditions are known to the person skilled in the art.

In the context of non-covalent binding, the binding between the HDAC ligand and the HDAC or the HDAC complex is, e.g., via salt bridges, hydrogen bonds, hydrophobic interactions or a combination thereof.

In a preferred embodiment, the steps of the formation of said complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the HDAC to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2 mM, wherein more preferably the buffer is selected from the group consisting of Tris-HCl or HEPES.

According to the second aspect of the invention, in steps b) and c) aliquots are contacted with an HDAC ligand. In a preferred embodiment of the invention, the ligand used in steps b) and c) is the same.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the methods of the invention, the read-out system is whether a binding between the HDAC complex and the HDAC ligand (first three aspects of the invention) or whether a binding HDAC and the HDAC ligand (fourth aspect of the invention) has occurred.

Methods for determining whether a binding between a ligand and a protein or protein complex has occurred are known in the art. These methods include in situ methods where the binding is assessed without separating the protein or protein complex from the ligand.

For example, anti-HDAC antibodies can be used in combination with the ALPHAScreen technology can be used where the excitation of a donor bead at 680 nm produces singlet oxygen which can diffuse to an acceptor bead undergoing a chemiluminescent reaction (Glickman et al., 2002. J. Biomol. Screen. 7(1):3-10).

In a preferred embodiment of the first three aspects according to the invention as well as according to the fourth aspect of the invention, the binding between the ligand and the HDAC complex or protein is determined by separating bound HDAC or HDAC complex from the ligand and subsequent determination of the HDAC or the HDAC complex. This subsequent determination of the HDAC or HDAC complex may either be the detection of the HDAC or the HDAC complex in the eluate or the determination of their amount.

In general, the fact that, in the methods of the invention, a binding between the HDAC ligand and the HDAC complex or the HDAC as occurred preferably indicates that the compound does not completely inhibit the binding. On the other hand, if no binding takes place in the presence of the compound, the compound is presumably a strong interactor with the HDAC, which is indicative for its therapeutic potential. In case that the amount is determined, the less HDAC or HDAC complex can be detected in the eluate, the stronger the respective compound interacts with the HDAC, which is indicative for its therapeutic potential.

Consequently, in a preferred embodiment of the methods of the invention, a reduced binding observed for the aliquot incubated with the compound in comparison to the aliquot not incubated with the compound indicates that HDAC is a target of the compound

According to invention, separating means every action which destroys the interactions between the ligand and the HDAC or the HDAC protein complex. This includes in a preferred embodiment the elution of the HDAC or the HDAC protein complex from the ligand. The elution can be achieved by using non-specific reagents (ionic strength, pH value, detergents).

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized compound. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl₂, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

Methods for the detection of proteins (and, therefore, also for HDAC or a HDAC protein complex) or for the determination of their amounts are known in the art and include physico-chemical methods such as protein sequencing (e.g. Edmann degradation), analysis by mass spectrometry methods or immunodetection methods employing antibodies directed against the HDAC.

According to a preferred embodiment of the methods according to the first, second and third aspect of the invention, the extent of the binding is determined by separating the HDAC protein complex from the ligand and subsequent detection of a component of the separated HDAC protein complex or subsequent determination of the amount of a component of the separated HDAC protein complex.

This preferred embodiment is especially advantageous, because it ensures that only HDAC protein complex is detected and not also free HDAC which has been bound to the ligand. In this context, the component may be any component known to be part of the respective HDAC complex which binding to the ligand is to be assessed. As shown in Example 5 and Figures 27, 28 and 29, the detection of a component of the HDAC protein complex is especially suitable for identifying an HDAC interacting compound according to the methods of the invention. Consequently, in a preferred embodiment, the term "component of an HDAC protein complex" denotes a component of said complex which is not an HDAC protein.

Throughout the invention, if an antibody is used in order to detect a respective protein (e.g. HDAC or a component of the HDAC complex), a specific antibody may be used (Wu and Olson, 2002. J. Clin. Invest. 109(10):1327-1333). As indicated above, such antibodies are known in the art. Furthermore, the skilled person is aware of methods for producing the same.

Preferably, a mass spectrometry or immunodetection methods are used in the context of the methods of the invention.

The identification of proteins with mass spectrometric analysis (mass spectrometry) is known in the art (Shevchenko et al., 1996, Analytical Chemistry 68: 850-858; Mann et al., 2001, Analysis of proteins and proteomes by mass spectrometry, Annual Review of Biochemistry 70, 437-473) and is further illustrated in the example section.

Preferably, the mass spectrometry analysis is performed in a quantitative manner, for example by using iTRAQ technology (isobaric tags for relative and absolute quantification) or cICAT (cleavable isotope-coded affinity tags) (Wu et al., 2006. J. Proteome Res. 5, 651-658).

According to a further preferred embodiment of the present invention, the characterization by mass spectrometry (MS) is performed by the identification of proteotypic peptides of the HDAC. The idea is that the HDAC or the component of the HDAC complex is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given HDAC or component of the HDAC complex, it is possible to compare the proteotypic peptides obtained for a given sample with the proteotypic peptides already known for HDACs or the component and thereby identifying the HDAC or the component being present in the sample.

Suitable immunodetection methods include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a HDAC interacting protein of interest, but also to analyse posttranslational modification patterns of HDAC or of other components of the HDAC protein complex such as phosphorylation or ubiquitin modification.

As detailed above, the identification methods of the invention involve the use of compounds which are tested for their ability to be a HDAC interacting compound.

Principally, according to the present invention, such a compound can be every molecule which is able to interact with the HDAC, for example by inhibiting its binding to the immobilization product of the invention. Preferably, the compound has an effect on the HDAC, e.g. a stimulatory or inhibitory effect.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr. Med. Chem. 2002; 9(23):2129-2145, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. The current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the methods of the invention, the HDAC or HDAC protein complex containing protein preparation is first incubated with the compound and then with the ligand. However, the simultaneous incubation is equally preferred (competitive binding assay).

In case that the incubation with the compound is first, the HDAC or HDAC protein complex containing protein preparation is preferably first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 nM to 100 µM, preferably from 10 nM to 10 µM. The second step, contacting with the ligand, is preferably performed for 10 to 60 minutes at 4°C.

In case of simultaneous incubation, the HDAC or HDAC protein complex containing protein preparation is preferably simultaneously incubated with the compound and the immobilization product of the invention for 30 to 120 minutes, more preferred 60 to 120 minutes at a temperature of 4°C to 37°C, more preferred 4°C to 25°C, most preferred 4°C. Preferably compounds are used at concentrations ranging from 1 nM to 100 µM, preferably from 10 nM to 10 µM.

Furthermore, the methods of the invention may be performed with several protein preparations in order to test different compounds. This embodiment is especially interesting in the context of medium or high throughput screenings.

Preferably, the identification methods of the invention are performed as a medium or high throughput screening.

The interaction compound identified according to the present invention may be further characterized by determining whether it has an effect on the HDAC, for example on its HDAC activity (Khan et al., 2008. Biochem. J. 409(2):581-9; Blackwell et al., 2008. Life Sci. 82(21-22):1050-1058).

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis. An example for lead optimization of HDAC inhibitors was reported (Remiszewski et al., 2003. J. Med. Chem. 46(21):4609-4624).

The invention further relates to a method for the preparation of a pharmaceutical composition comprising the steps of
a) identifying a HDAC interacting compound as described above, and
b) formulating the interacting compound to a pharmaceutical composition.

Methods for the formulation of identified compounds are known in the art. Furthermore, it is known in the art how to administer such pharmaceutical compositions.

The obtained pharmaceutical composition can be used for the prevention or treatment of diseases where the respective HDAC plays a role, e.g. for the prevention or treatment of cancer (Bolden et al., 2006. Nat. Rev. Drug Discov. 5(9):769-784). For example, HDAC inhibitors may be useful for the treatment of inflammatory diseases, cancer or neurodegenerative diseases.

The invention is further described by the following figures and examples, which are intended to illustrate, but not to limit the present invention. In case where in the following examples the term "affinity matrix" is used, this term refers to the immobilized ligand as defined in the present application.

### Short description of the figures

**Figure 1****:** Synthesis of the immobilization compound. Steps: i) DCC, HOBt, DMF, rt, ii) NaOHaq 4N, MeOH, iii) H2NOCH2Ph.HCl, PyBrop, DIEA, DMF, iv) 10% Pd/C, EtOH, v) 4N HCl in Dioxane. Methods used in the synthesis of the immobilization compound are described in example 1.
**Figure 2****:** Structure of the immobilization compound based on SAHA.
**Figure 3****:** Amino acid sequence of human HDAC1 (IPI00013774.1). Peptides identified by mass spectrometry are underlined (K562; experiment X003787).
**Figure 4****:** Amino acid sequence of human HDAC2 (IPI00289601.10). Peptides identified by mass spectrometry are underlined (K562; experiment X003787).
**Figure 5****:** Amino acid sequence of human HDAC3 (IPI00217965.1). Peptides identified by mass spectrometry are underlined (K562; experiment X003787).
**Figure 6****:** Amino acid sequence of human HDAC6 (IPI00005711.4). Peptides identified by mass spectrometry are underlined (K562; experiment X003787).
**Figure 7****:** Amino acid sequence of human HDAC8 (IPI00747259.2). Peptides identified by mass spectrometry are underlined (K562; experiment X003787).
**Figure 8****:** Amino acid sequence of human HDAC10 (IPI00012439.5). Peptides identified by mass spectrometry are underlined (K562; experiment X003787).
**Figure 9****:** Amino acid sequence of human AOF2 / LSD1 (IPI00217540.7). Peptides identified by mass spectrometry are underlined (K562; experiment X003787).
**Figure 10****:** Amino acid sequence of human GSE1 (IPI00215963.5). Peptides identified by mass spectrometry are underlined (K562; experiment X003787).
**Figure 11****:** Amino acid sequence of human HMG20A (IPI00018924.3). Peptides identified by mass spectrometry are underlined (K562; experiment X003787).
**Figure 12****:** Amino acid sequence of human HMG20B (IPI00464951.5). Peptides identified by mass spectrometry are underlined (K562; experiment X003787).
**Figure 13****:** Amino acid sequence of human RCOR1 (IPI00008531.1). Peptides identified by mass spectrometry are underlined (K562; experiment X003787).
**Figure 14****:** Amino acid sequence of human RCOR3 (IPI00914887.1). Peptides identified by mass spectrometry are underlined (K562; experiment X003787).
**Figure 15****:** Amino acid sequence of human ZNYM2 (IPI00294603.6). Peptides identified by mass spectrometry are underlined (K562; experiment X003787).
**Figure 16****:** Amino acid sequence of human GATAD2A (IPI00478128.2). Peptides identified by mass spectrometry are underlined (K562; experiment X003787).
**Figure 17****:** Amino acid sequence of human GATAD2B (IPI00103554.1). Peptides identified by mass spectrometry are underlined (K562; experiment X003787).
**Figure 18****:** Amino acid sequence of human MBD3 (IPI00439194.1). Peptides identified by mass spectrometry are underlined (K562; experiment X003787).
**Figure 19****:** Amino acid sequence of human MTA2 (IPI00171798.1). Peptides identified by mass spectrometry are underlined (K562; experiment X003787).
**Figure 20****:** Amino acid sequence of human MTA3 (IPI00165357.4). Peptides identified by mass spectrometry are underlined (K562; experiment X003787).
**Figure 21****:** Amino acid sequence of human SMARCC2 (IPI00216047.3). Peptides identified by mass spectrometry are underlined (K562; experiment X003787).
**Figure 22****:** Amino acid sequence of human TBL1X (IPI00640917.1). Peptides identified by mass spectrometry are underlined (K562; experiment X003787).
**Figure 23****:** Amino acid sequence of human TBL1XR1 (IPI00002922.5). Peptides identified by mass spectrometry are underlined (K562; experiment X003787).
**Figure 24****:** Amino acid sequence of human SIN3A (IPI00170596.1). Peptides identified by mass spectrometry are underlined (K562; experiment X003787).
**Figure 25****:** Amino acid sequence of human RBBP4 (IPI00328319.8). Peptides identified by mass spectrometry are underlined (K562; experiment X003787).
**Figure 26****:** Amino acid sequence of human RBBP7 (IPI00646512.1). Peptides identified by mass spectrometry are underlined (K562; experiment X003787).
**Figure 27****:** Dose response curves for HDACs (Example 5). HDAC1: IC₅₀ = 0.78 µM;
HDAC2: IC₅₀ = 2.8 µM; HDAC6: IC₅₀ = 0.20 µM.
**Figure 28****:** Dose response curves for components of the Co-REST protein complex (Example 5): RCOR1: IC₅₀ = 0.15 µM; RCOR3: IC₅₀ = 0.15 µM; AOF2: IC₅₀ = 0.14 µM.
**Figure 29****:** Dose response curves for components of the NuRD protein complex (Example 5). MTA2: IC₅₀ = 0.53 µM; RBBP4: IC₅₀ = 0.46 µM; MTA3: IC₅₀ = 1.8 µM.
**Figure 30****:** Dose response curves for components of the NCoR protein complex (Example 7). HDAC3: IC₅₀ = 73 µM; TBL1X: IC₅₀= 78 µM; TBL1XR1 (IC₅₀= 60 µM).

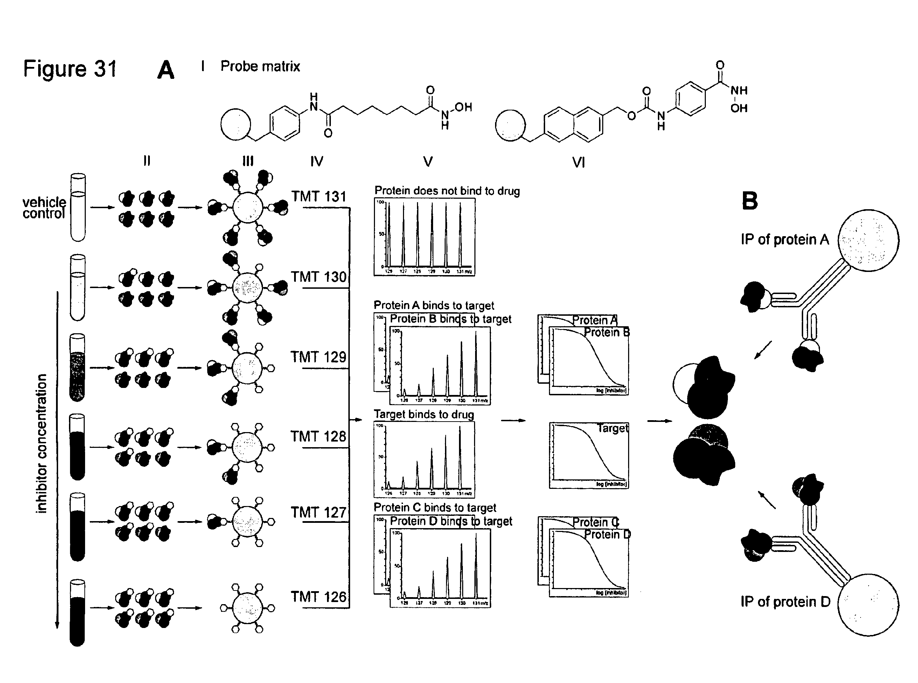

**Figure 31****:** Mapping of HDAC drug target complexes in chemical space and in proteome space. (a) Chemoproteomics competition binding assay to profile HDAC inhibitor target complexes in cell extract. (I) Generation of a probe matrix by the derivatization of sepharose with analogues of nonselective HDACi (SAHA, ITF2357). (II) Cell extract aliquots are incubated with vehicle or with drug over a range of concentrations. (III) The "free" drug competes with the immobilized probes for the drug binding sites of the target protein complexes. (IV) Captured proteins are trypsinized and each peptide mixture is tagged with a distinct isobaric TMT tag. (V) Tagged samples are pooled and analyzed by LC-MS/MS such that each peptide gives rise to six reporter signals in the MS/MS spectrum. (VI) When capturing of a protein is competed away by the drug, a decrease of signal intensity compared to the vehicle control is detected for each peptide originating from this protein. Protein complexes formed by the target and associated proteins are defined by matching inhibition (IC₅₀) curves. (b) Definition of target protein complexes in biological space by quantitative immunoaffinity purification. Data are generated from the same cell extracts and are used to deconvolute protein complexes formed around the drug target.

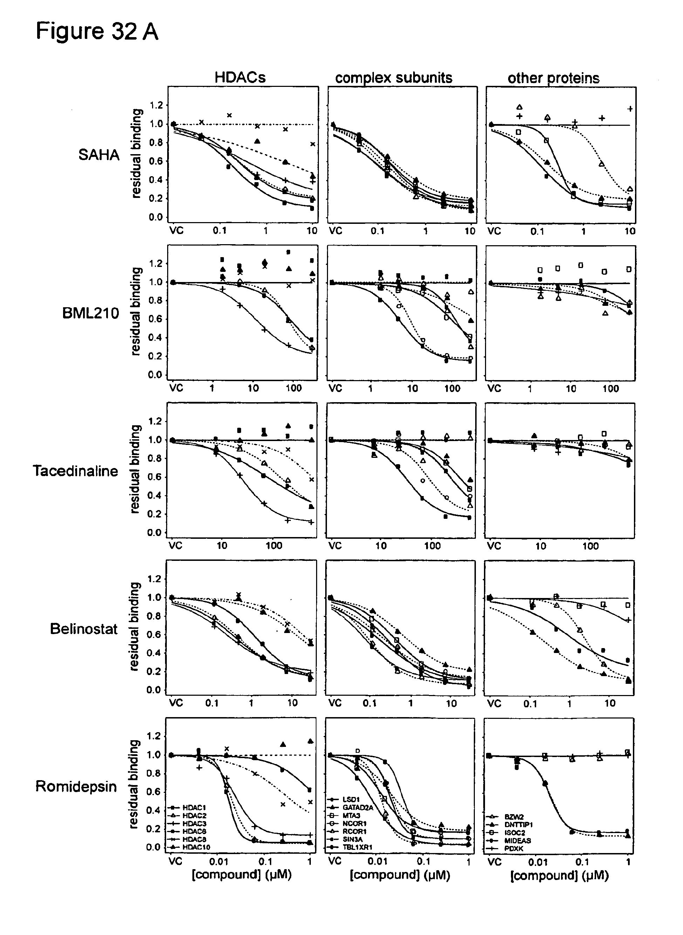

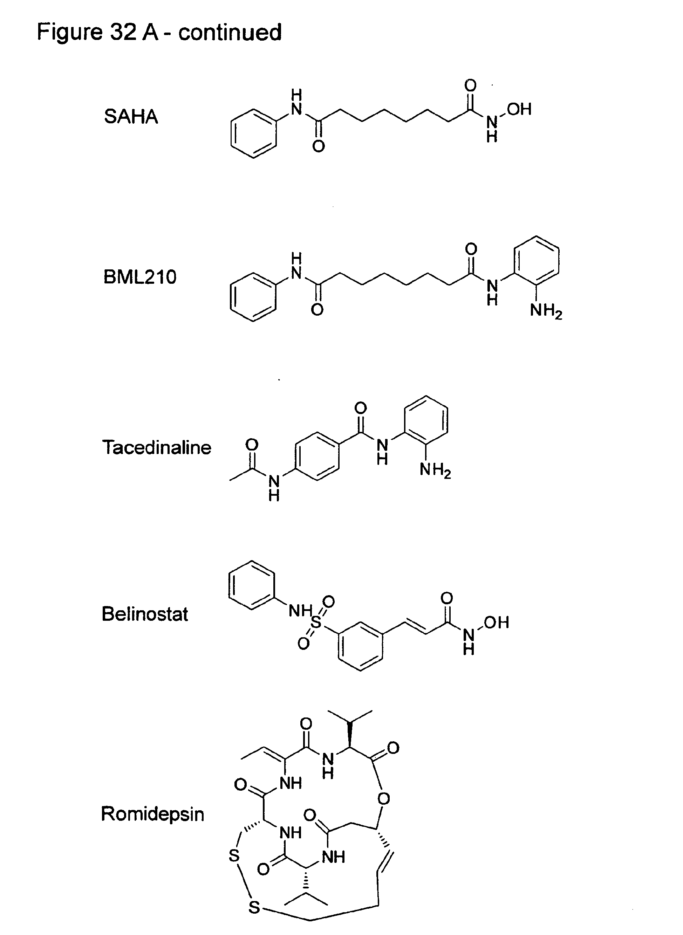

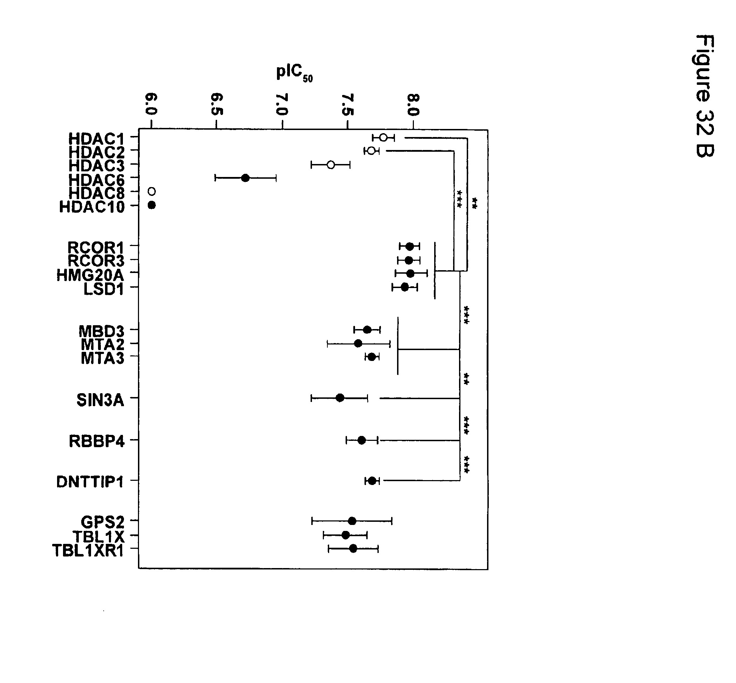

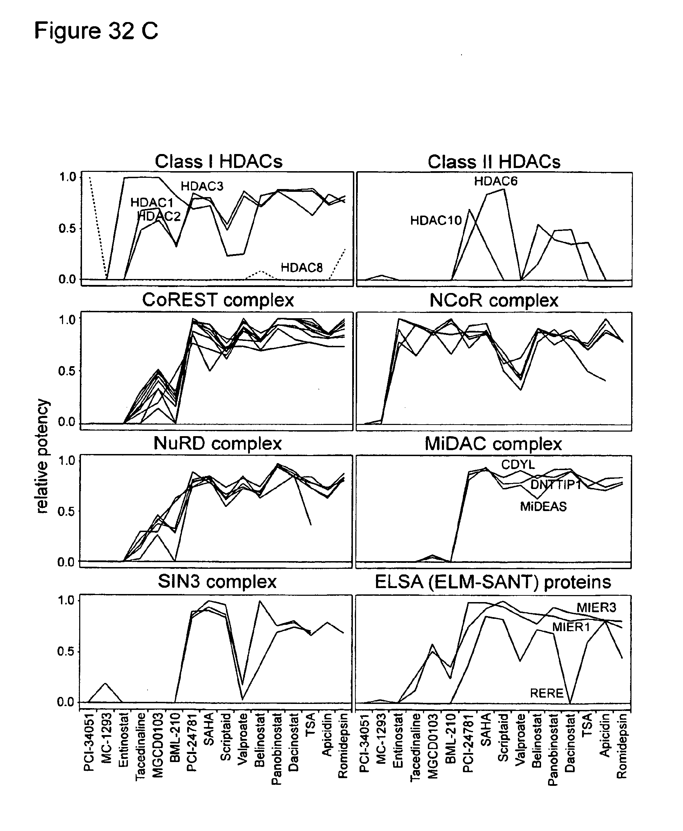

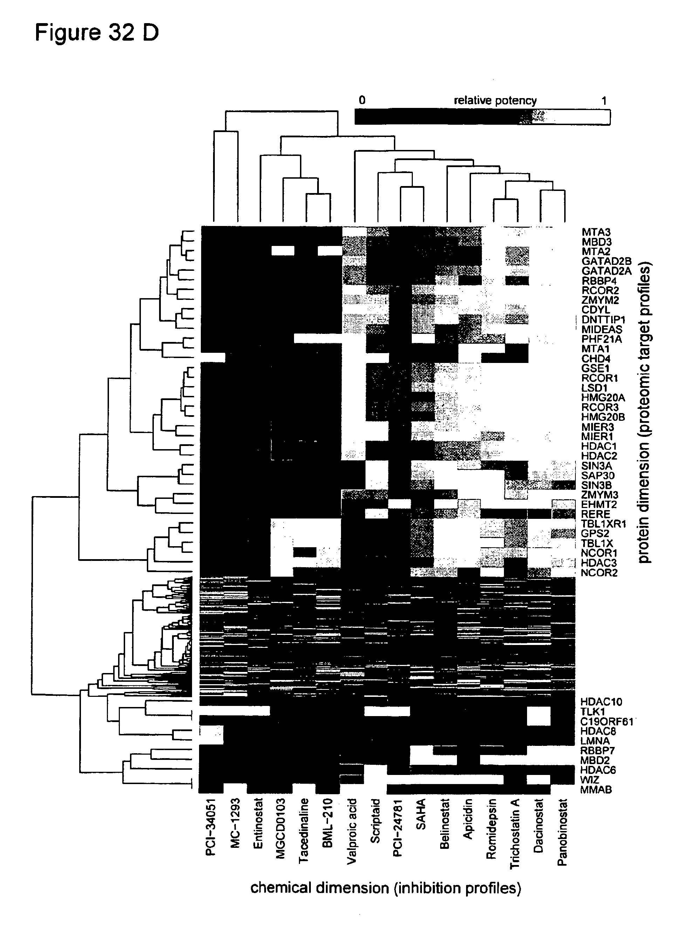

**Figure 32****:** HDACi drug targets and target complexes are defined by chemoproteomic profiling of clinical and tool compounds. (a) Representative concentration-inhibition profiles of SAHA, BML-210 (the benzamide analog of SAHA), tacedinaline (CI-994), belinostat, and romidepsin were determined in K562 cell extract as outlined in Figure 31. For clarity, selected profiles are grouped in 3 plots for each inhibitor: HDACs (left), components of CoREST, NuRD, Sin3, and NCoR complexes (middle), and other proteins either representing novel direct targets or complex components (right). (b) Assay reproducibility. Mean pIC₅₀ data for Trichostatin A from seven independent replicates are shown and statistically significant differences between potencies observed for individual HDAC1/2 complexes are indicated (**: p < 0.01; ***: p<0.001). (c) Binding profiles for HDACs and protein complexes across a panel of 16 inhibitors. For each compound, its relative potency for each affected protein was calculated in relation to its most potently inhibited target as (pIC₅₀-min(pIC₅₀))/(max(pIC₅₀)-min(pIC₅₀)). The profile for each protein versus all compounds is traced by a grey lines with proteins of interest highlighted in different panels: class I HDACs, class II HDACS, CoREST components, NCoR components, NuRD components, and Sin3 components. Putative novel HDAC complexes formed around MIDEAS (mitotic deacetylase complex, MiDAC) and other ELSA (ELM-SANT) domain proteins are represented. (d) Bidirectional hierarchic clustering of the concentration-inhibition (relative potency) data for 16 inhibitors versus 344 proteins (each targeted by at least one inhibitor). For readability the parts of the clustering around HDAC targets is represented at a higher vertical resolution. Previously published associations of proteins to complexes are represented.

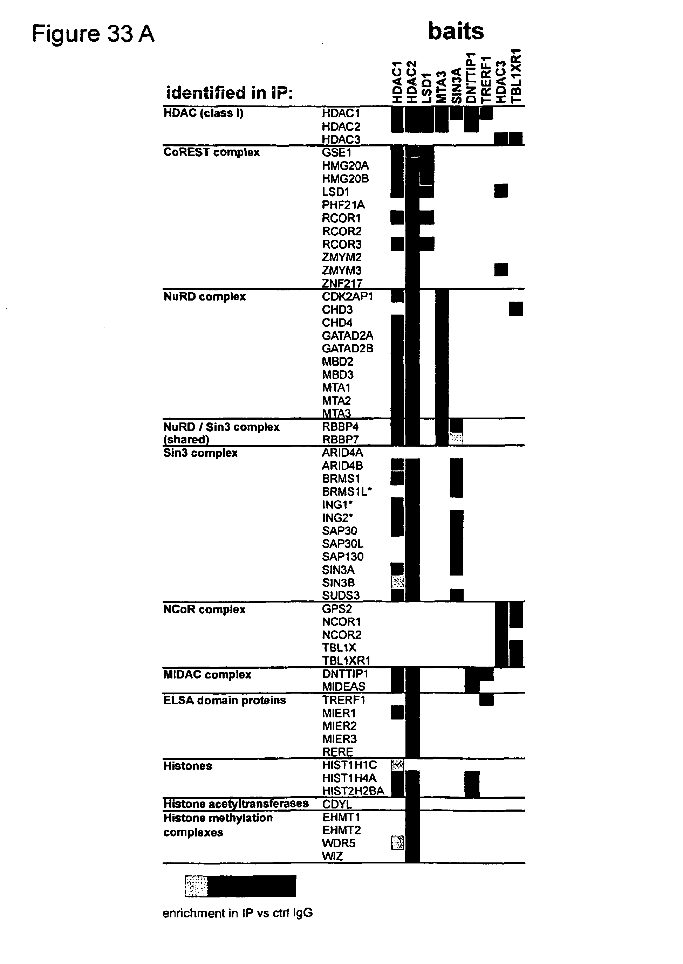

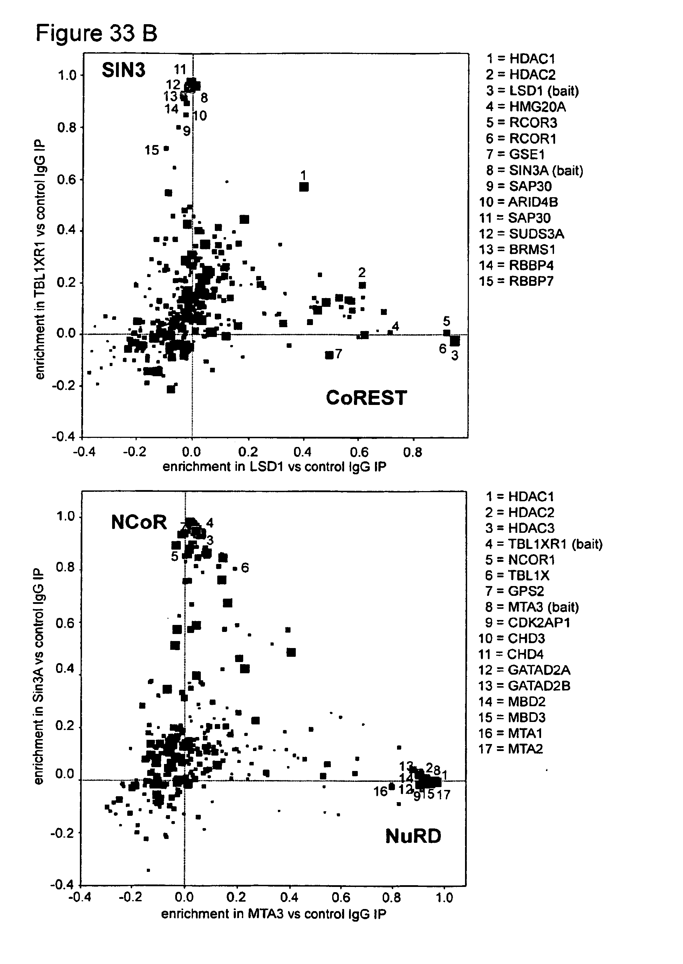

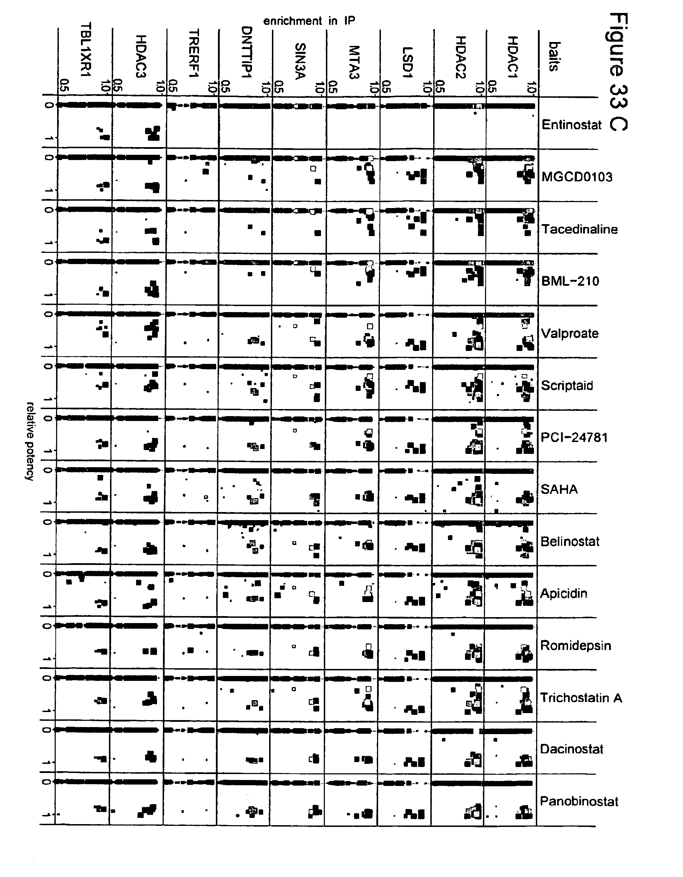

**Figure 33****:** Deconvolution of protein complexes by IP-MS/MS analysis confirms novel HDAC complexes. (a) Selected probe matrix interactome proteins identified in IP-MS/MS analysis of HDAC complexes. IPs were performed in K562 cells using antibodies directed against HDAC1, 2, and 3, known complex subunits (the CoREST subunit LSD1; the NuRD subunit MTA3; the Sin3 subunit SIN3A; and the NCoR-subunit TBL1XR1), and examples of novel HDACi binding proteins (DNTTIP1 and TRERF1). * denotes previously reported complex components not identified in the matrix interactome. (b) Examples of the quantitative mapping of immunoaffinity-purified protein complexes by MS/MS. Purifications conducted with two different antibodies each and their corresponding isotype controls were combined after PAGE purification, trypsinisation and isobaric tagging. Protein quantification data are represented as plots of relative enrichment in immunoprecipitates of (upper panel) Sin3 vs. LSD1, and (lower panel) MTA3 vs. TBL1XR1. Each square represents a protein with its size scaled according to the number of sequence-to-spectrum matches. (c) HDAC protein complexes in chemical and biological space. The enrichment of proteins in the IP samples is plotted against the inhibitor potency data.

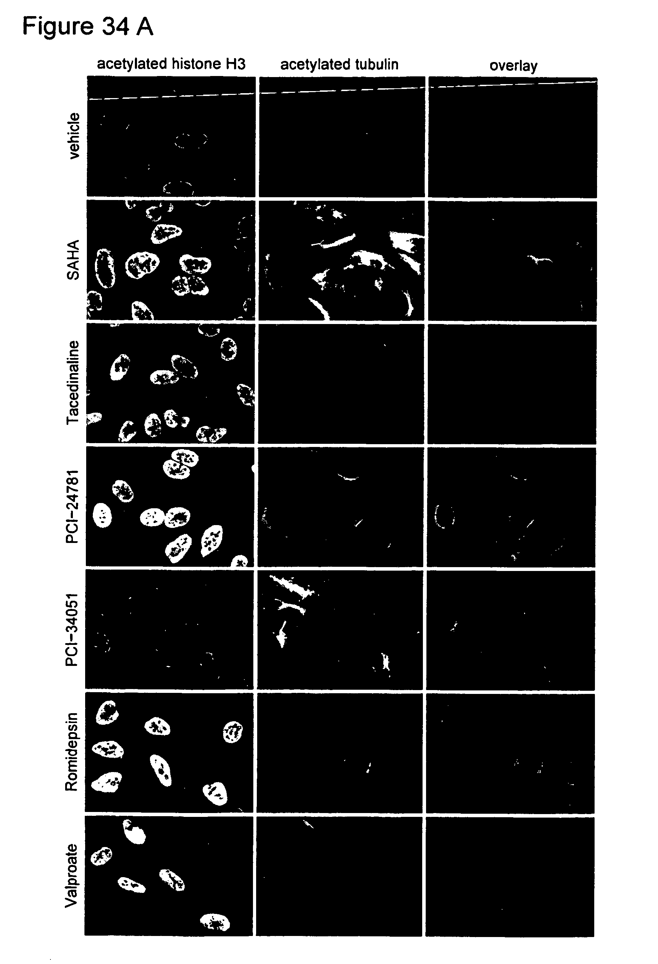

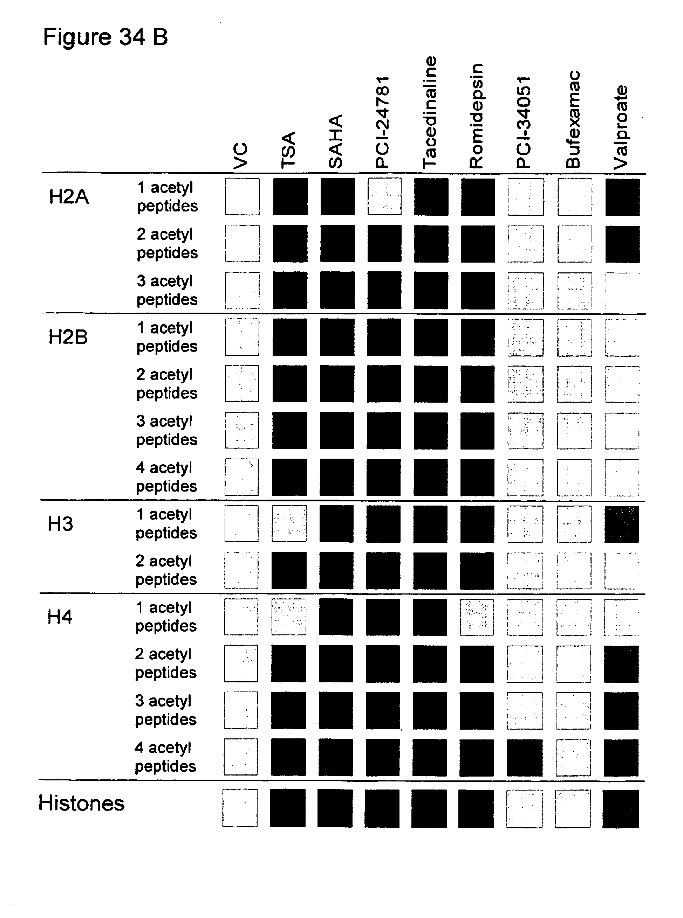

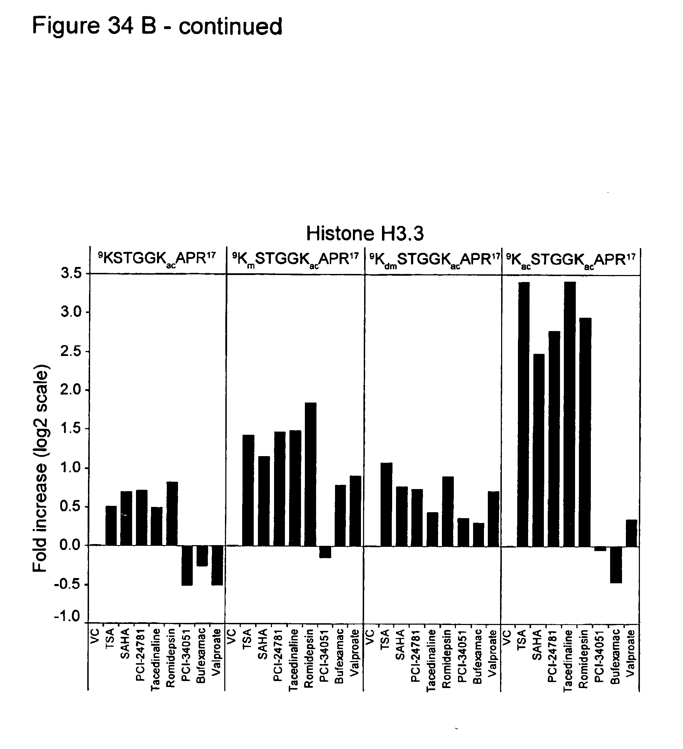

**Figure 34****:** Differential effects of HDAC inhibitors on histone and tubulin acetylation. (a) Immunofluorescence analysis of histone H3 (K9ac/K14ac) and tubulin acetylation in HeLa cells treated for 4 hours with vehicle, SAHA (10 µM), tacedinaline (50 µM), PCI-24781 (20 µM), PCI -34051 (100µM), romidepsin (1µM) or valproate (2000µM). (b) Mapping of histone acetylation by LC-MS/MS of K562 cells treated with HDACi. Cells were treated with TSA (10 µM), SAHA (5 µM), PCI-24781 (2 µM), tacedinaline (50 µM), romidepsin (1 µM), PCI-34051 (20 µM), bufexamac (100 µM), MC 1293 (100 µM), or valproate (2000µM) for 6 hours. Histones were extracted from cells, trypsinized, and acetylated peptides were quantified after isobaric mass (TMT) tagging. Heat map representation of the abundance of histone peptides with single or multiple acetylated lysines as a result of inhibitor treatment and bar chart representation of abundance of differently modified variants of the Histone H3.3 peptide 9-17.

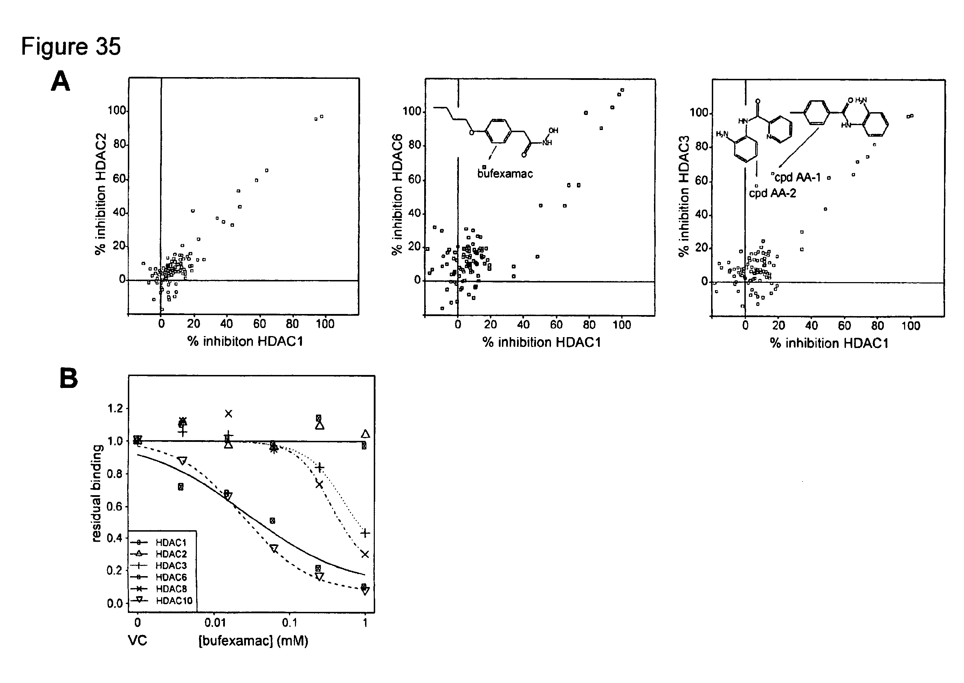

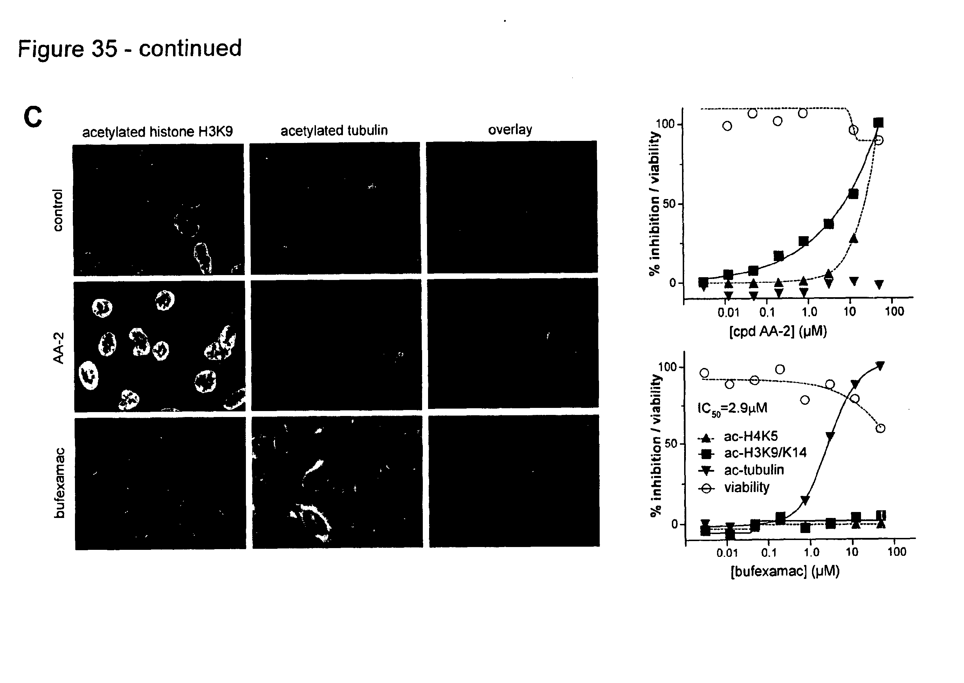

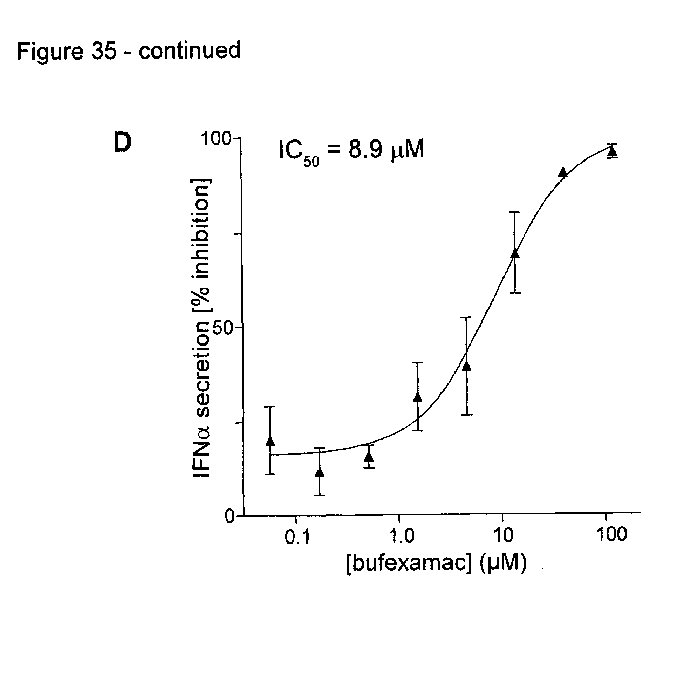

**Figure 35****:** The non-steroidal anti-inflammatory drug bufexamac is a novel Class IIb HDAC inhibitor. (a) Screen of a focused compound library against HDACs 1, 2, 3 and 6 using a chemoproteomics binding assay with the SAHA matrix in whole cell extract from Jurkat and Ramos cells. The plots outline inhibition relative to HDACI for HDAC6, HDAC3, and HDAC2, as quantified by antibodies on dot-blot arrays. The compound concentration was 10 µM and chemical structures of selective hit compounds are shown. (b) HDAC selectivity profile of bufexamac in K562 cells, measured as outlined in Figure 31. (c) Treatment of HeLa cells with bufexamac elicits hyperacetylation of tubulin whereas treatment with the o-aminoanilide AA-2 leads to hyperacetylation of histones. Cultured cells were treated with vehicle or drug for 4h, and cells were analyzed by immunofluorescence microscopy and by western blotting using antibodies for acetylated tubulin (EC₅₀=2.9 µM) and acetylated histones H3 (K9) and H4 (K5), respectively. (d) Treatment of peripheral blood mononuclear cells with bufexamac inhibits the secretion of IFNα (EC₅₀=8.9 +/- 4.9 µM, 3 independent experiments).

### Examples

### Example 1: Preparation of the affinity matrix

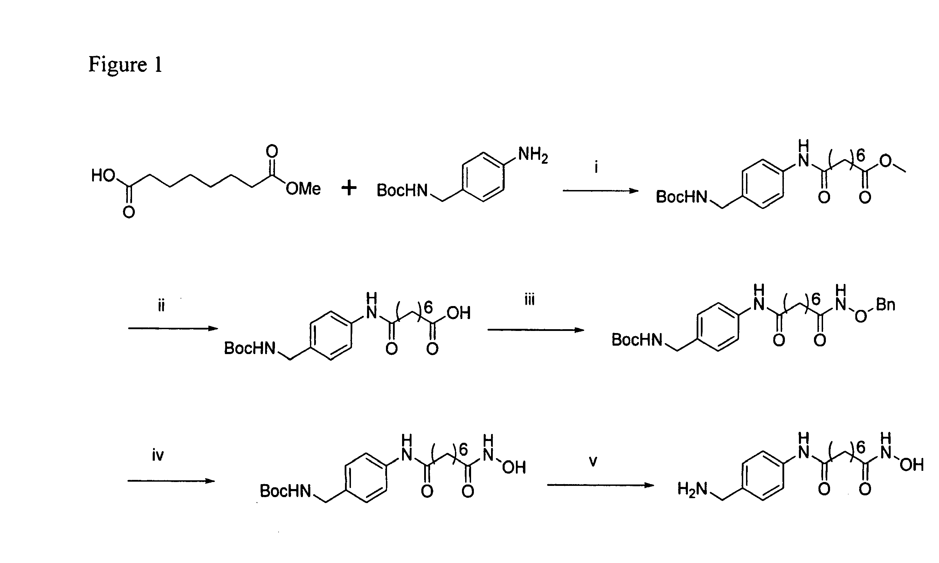

This example describes the synthesis of compounds (Figure 1) and methods for their immobilization on a solid support yielding the affinity matrix used in the following examples for the capturing of HDACs from cell lysates.

### Synthesis of methyl 8-(4-((tert-butoxycarbonylamino)methyl)phenylamino)-8-oxooctanoate

To a stirred solution of suberic acid monomethyl ester (3.75mmol, 1eq, 0.706g), 4[N-Boc aminomethyl]aniline (4.5mmol, 1.2eq, 1g), Hydroxybenzotrizol (4.5mmol, 1.2eq, 0.608g) in DMF (20ml) was added dicyclocarbodiamide (4.5mmol, 1.2eq, 0.928g). The reaction was stirred at room temperature overnight. The reaction precipitate was filtered. The filtrate was treated with water (20ml). The resulting precipitate was filtered. The filtrate was then concentrated and purified by Flash Chromatography (Hexane/Ethyl acetate 1: 1) to yield the desired compound as a white solid (m=1.25 g, 88%). LCMS (method A) Rt=2.93mn, M+H=393, M+Na=415.

### Synthesis of 8-(4-((tert-butoxycarbonylamino)methyl)phenylamino)-8-oxooctanoic acid

To a stirred solution of 8-(4-((tert-butoxycarbonylamino)methyl)phenylamino)-8-oxooctanoate (0.579mmol, 0.227g) in methanol (7ml) was added 2N aqueous sodium hydroxide (1.16mmol, 2eq, 0.580ml). The reaction was stirred at room temperature overnight. The solvent was removed. The residue was dissolved in water. The pH was raised to 6 by addition of 2N aqueous hydrochloric acid. The resulting precipitate was filtered and dried in the vacuum oven (40°C) overnight to yield the desired compound as a white solid (m=0.169g, 77%) LCMS (Method B): Rt=2.48mn M+H=379, M+Na=401

### Synthesis of tert-butyl 4-(8-(benzylaminooxy)-8-oxooctanamido)benzylcarbamate

To a stirred solution of 8-(4-((tert-butoxycarbonylamino)methyl)phenylamino)-8-oxooctanoic acid (0.397mmo, 1eq, 0.150g) and O-benzylhydroxylamine hydrochloride (0.397mmol, 1eq, 0.063g) in dimethylformamide (5ml) and diisopropylethylamine (1.59mmol, 4eq, 0.277ml) was added Bromo-tris-pyrrolidino phosphoniumhexafluorophosphate (0.595mmol, 1.5eq, 0.277g). The reaction was stirrrd at room temperature overnight. The reaction was diluted with water (10ml) and extracted with Ethyl acetate (2x50ml). The organic layers were dried over magnesium sulphate, then concentrated and purified by flash chromatography (Hexane/Ethyl acetate (30-100%) to yield the desired product as an oil (m=0.105g, 55%) LCMS (method B) rt=2.73mn, M+H=484, M+Na=506.

### Synthesis of tert-butyl 4-(8-(aminooxy)-8-oxooctanamido)benzylcarbamate

To a degassed solution of tert-butyl 4-(8-(benzylaminooxy)-8-oxooctanamido)benzylcarbamate (0.787mmol, 1eq, 0.380g) in ethanol (15ml) was added 10% Pd/C (10%, 38mg). The reaction was saturated in hydrogen and stirred under hydrogen atmosphere overnight. The catalyst was filtered and the filtrate concentrated to yield the desired compound as a yellow oil (0.309g, 99%). LCMS (method C) RT=2.18mn, M+H=394, M+Na=416.

### Synthesis of N-(4-(aminomethyl)phenyl)-8-(aminooxy)-8-oxooctanamide

To tert-butyl 4-(8-(aminooxy)-8-oxooctanamido)benzylcarbamate (0.22mmol, 0.150g) was added 2ml of hydrochloric acid 4N in Dioxane. The mixture was stirred at room temperature for 3 hours. The reaction was evaporated and purified by HPLC (high pH) to yield the desired compound as an off white solid (m=51mg, 46%). LCMS (method C): Rt=1.46mn 2M+H=587, M+Na=316. ¹NMR (DMSO-d6, 400MHz): δ= 9.81 (s, 1H), 7.51 (d,2H), 7.23 (d, 2H), 3.64 (s, 2H), 2.27 (d, 2H), 1.93 (d, 2H), 1.56 (m, 2H), 1.48 (m, 2H), 1.26 (m, 4H).

### LCMS conditions

Columns: Phenomenex Gemini-C18, 3.0 x 30 mm
Flow rate: 1.2 ml/mn
Temperature: 40°C
Wavelength: 254nm (reference at 400nm fro method B), 210nm (reference at 360nm for method B)

The mass spectrometry data were gathered in positive or negative mode, scanning for masses between 150 and 700amu, using a fragmentor ramp (method B) from 118V to 400V (for MW= 118.09 to MW= 922.01 respectively) and a fragmentor set up to 100V for methods A and C.

### Solvents:

A= Water with 0.1% formic acid
B= Acetonitrile with 0.1 % formic acid
C= Water with 0.1% ammonia
D= (95% : 5%, acetonitrile : water) with 0.1% ammonia

### Gradient conditions

**Table 1: Method A: Short Column Analytical, Low pH, Positive ion**

| **Time (min)** | **%A** | **%B** |
|---|---|---|
| **0.00** | **95.0** | **5.0** |
| **3.00** | **5.0** | **95.0** |
| **4.50** | **5.0** | **95.0** |
| **4.60** | **95.0** | **5.0** |
| **5.00** | **95.0** | **5.0** |

**Table 2: Method B : Short Column Analytical, Low pH, Positive ion**

| **Time (min)** | **%A** | **%B** |
|---|---|---|
| **0.00** | **95.0** | **5.0** |
| **3.00** | **5.0** | **95.0** |
| **4.50** | **5.0** | **95.0** |
| **4.60** | **95.0** | **5.0** |
| **5.00** | **95.0** | **5.0** |

**Table 3: Method C : Short Column Analytical, High pH, Positive ion**

| **Time (min)** | **%C** | **%D** |
|---|---|---|
| **0.00** | **95.0** | **5.0** |
| **3.00** | **0.0** | **100.0** |
| **4.50** | **0.0** | **100.0** |
| **4.60** | **95.0** | **5.0** |
| **5.00** | **95.0** | **5.0** |

### Conditions for the Prep HPLC High pH

Column: Phenomenex Gemini C18 100 x 21.20mm 5µm
Solvents: A= Water +0.1% Ammonia
B= (95% Acetonitrile: 5% Water) + 0.1% Ammonia
Flow Rate: 20ml/min
Temperature: Room temperature
Gradient conditions: The gradient conditions were variable depending on the retention time of each compound
Wavelength: PDA detection from 200-400nm (or 220nm, depending on the purification system used)
Mass spec conditions: The mass spec data were gathered in positive and negative mode, from 150 to 700 amu.

**Table 4: Abbreviations**

| | |
|---|---|
| Boc | tert-butoxycarbonyl |
| DCM | Dichloromethane |
| DMSO | dimethylsulfoxide |
| MeOH | Methanol |
| EtOH | Ethanol |
| ⁱPr₂NEt | Diisopropylethylamine |
| NH₂OH·HCl | hydroxylaminehydrochloride |
| Pd(dppf)(Cl)₂ | [1,1'bis(diphenylphosphino)ferrocene] dichloro-palladium (II) |
| DMF | N,N-Dimethylformamide |
| THF | tetrahydrofuran |
| s | singlet |
| d | Doublet |
| dd | Doubledoublet |
| br | Broad |
| mL | millilitres |
| L | litre |
| t | Triplet |
| m | Multiplet |
| Rt | Retention time |

### Immobilization of compounds on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in the following examples. Control beads (no compound immobilized) were generated by blocking the NHS-groups by incubation with aminoethanol as described above.

### Example 2: HDAC experiment using an immobilized compound and a K-562 cell lysate

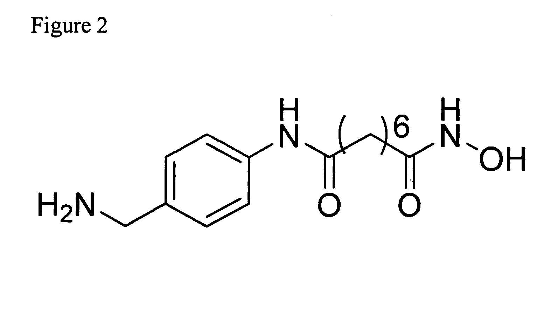

This example illustrates the use of a competition binding assay in cell lysate to identify and characterize HDAC protein complexes and to establish the selectivity profile of the test compound SAHA (vorinostat, suberoylanilide hydroxamic acid). This compound was added at defined concentrations (10 µM, 2.5 µM, 0.625 µM, 0.156 µM and 0.039 µM) to aliquots of K-562 cell lysates thereby allowing the test compound to bind to the target proteins in the lysate. Then the lysate was contacted with the affinity matrix (immobilized compound; Figure 2) to capture remaining free target proteins. The proteins bound to the immobilized compound were eluted with detergent-containing buffer, separated on a SDS-polyacryamide gel and analyzed by mass spectrometry.

The peptide extracts corresponding to samples treated with different concentrations of the test compound (10 µM, 2.5 µM, 0.625 µM, 0.156 µM and 0.039 µM) and the solvent control (0.5% DMSO) were treated with different variants of the isobaric tagging reagent (TMT-reagents, Thermofisher). The TMT reagents are a set of multiplexed, amine-specific, stable isotope reagents that can label peptides in up to six different biological samples enabling simultaneous identification and quantitafication of peptides. The TMT-reagents reagents were used according to instructions provided by the manufacturer.

The combined samples were analyzed with a nano-flow liquid chromatography system coupled online to a tandem mass spectrometer (LC-MS/MS) experiment followed by reporter ion quantification in the MS/MS spectra (Ross et al., 2004. Mol. Cell. Proteomics 3(12):1154-1169; Dayon et al., 2008. Anal. Chem. 80(8):2921-2931; Thompson et al., 2003. Anal. Chem. 75(8):1895-1904). Further experimental protocols can be found in WO2006/134056 and a previous publication (Bantscheff et al., 2007. Nature Biotechnology 25, 1035-1044).

The test compound SAHA was used at five different concentrations in the cell lysate and the IC₅₀ values were normalized to the DMSO control. For selected HDACs and HDAC interactors the IC₅₀ values were plotted against the concentration of SAHA and curve fitting was performed using the Xlfit program (ID Business Solutions Ltd.) as previously described. (Bantscheff et al., 2007. Nature Biotechnology 25, 1035-1044). The IC₅₀ value corresponds to the test compound concentration at which the relative intensity of the MS signal for a protein is 50% compared to the solvent (DMSO) control.

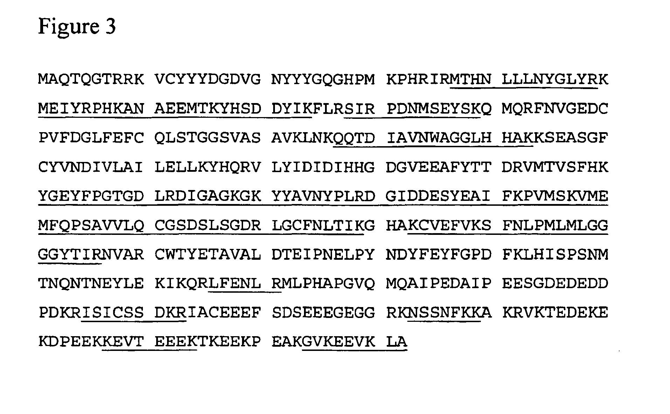

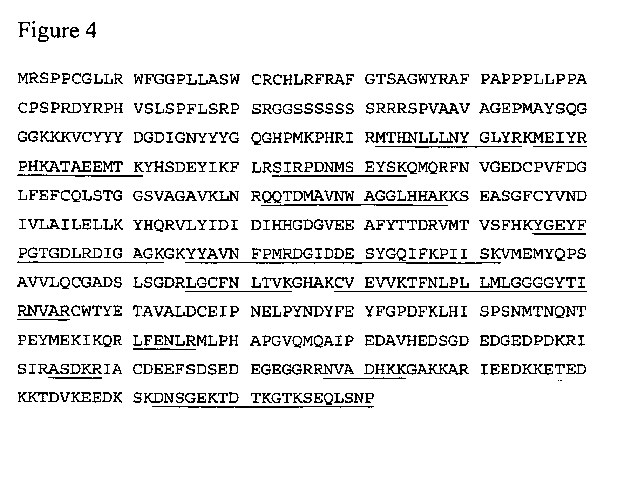

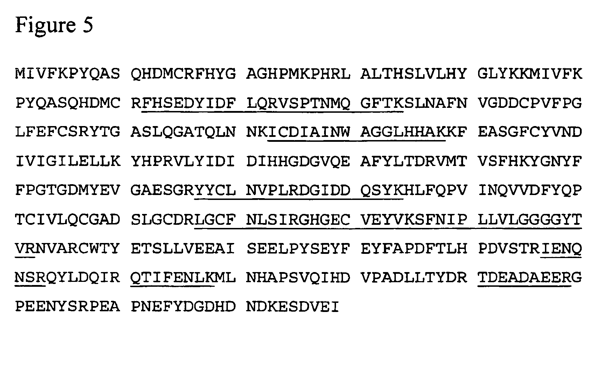

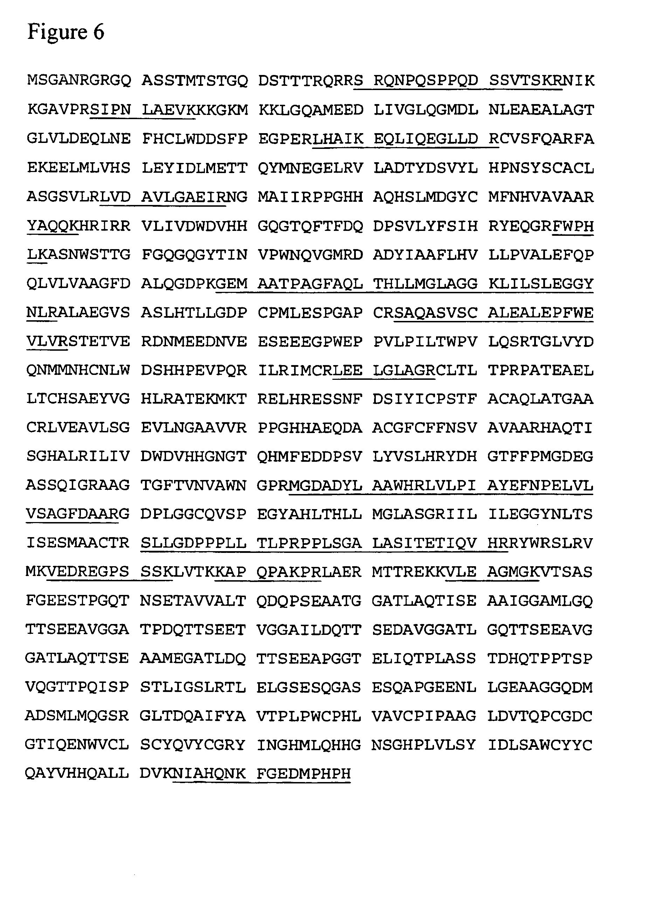

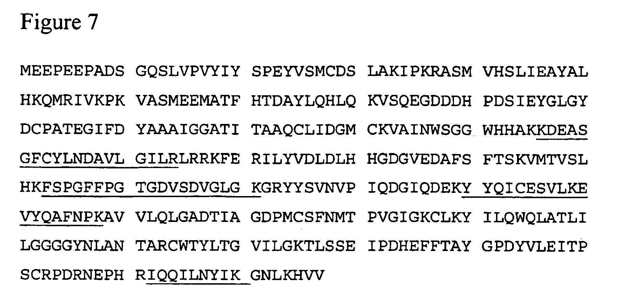

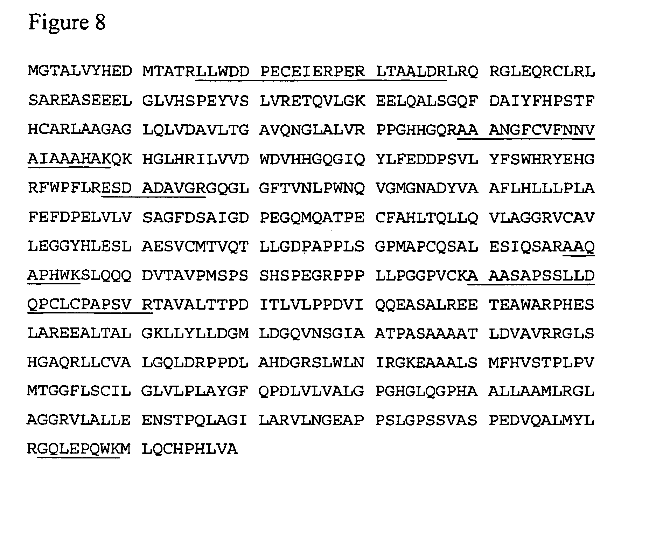

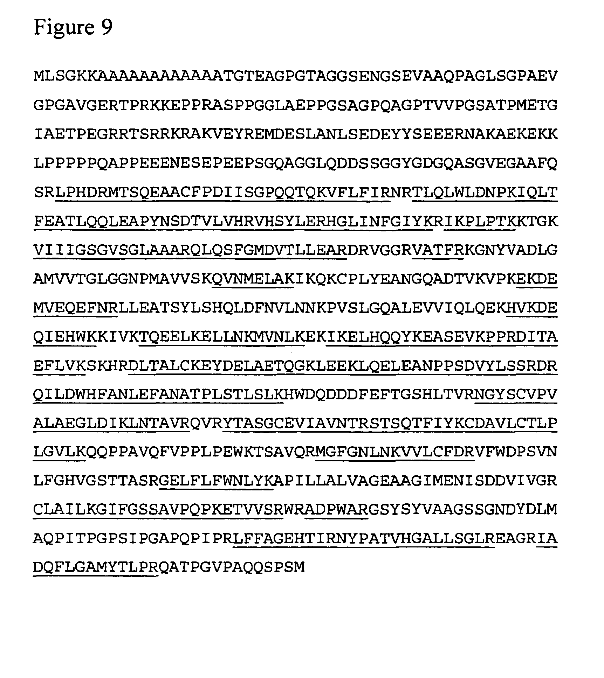

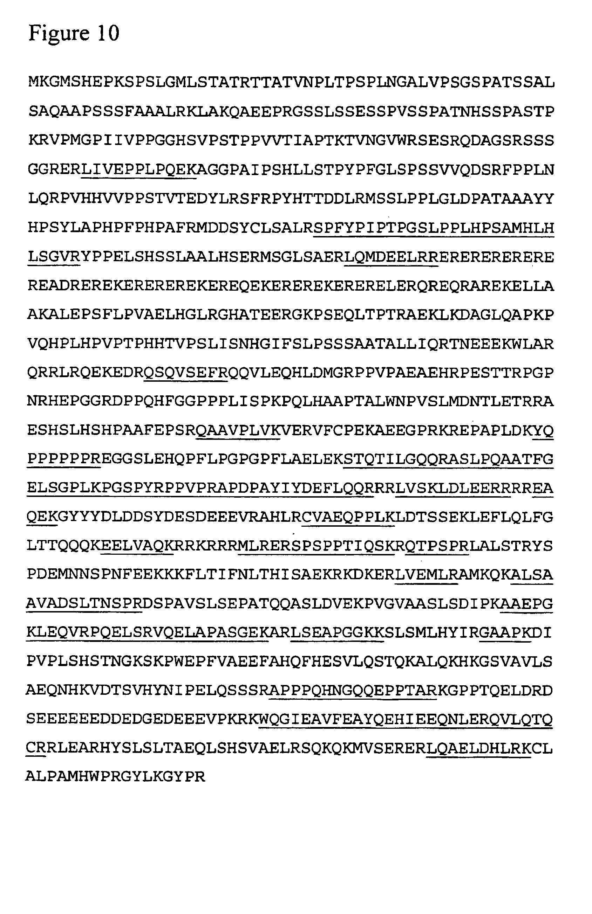

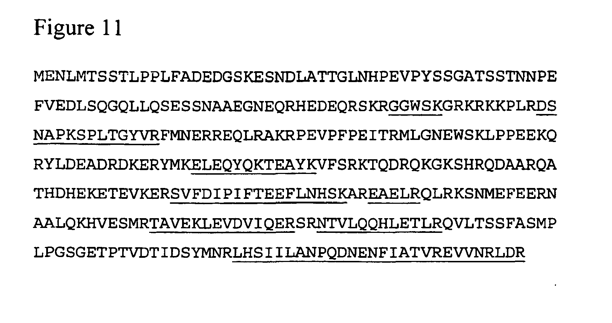

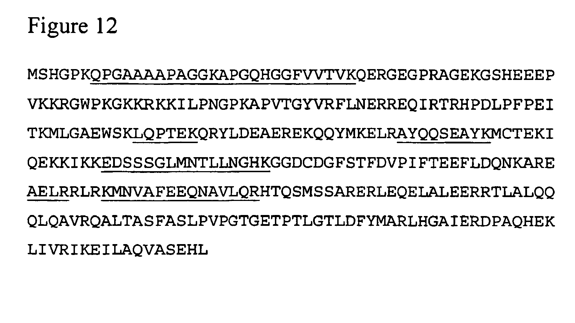

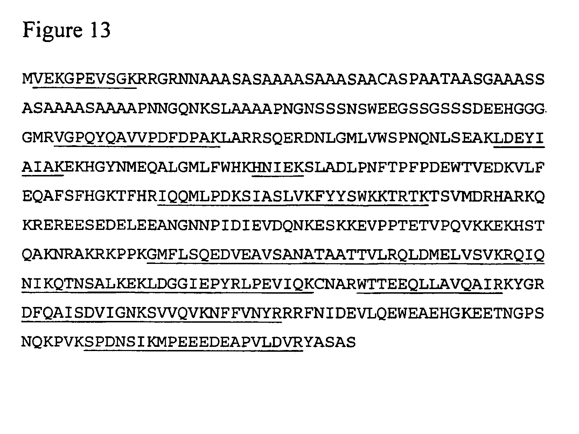

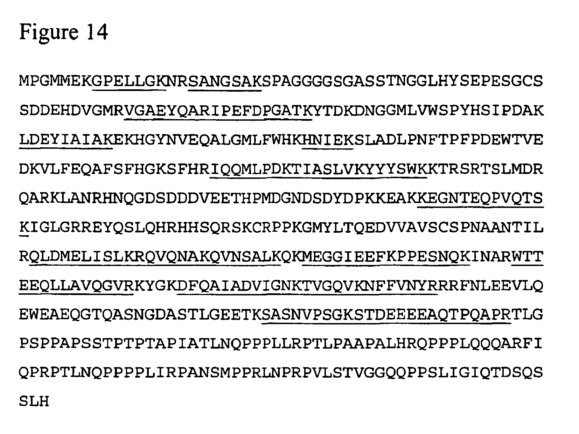

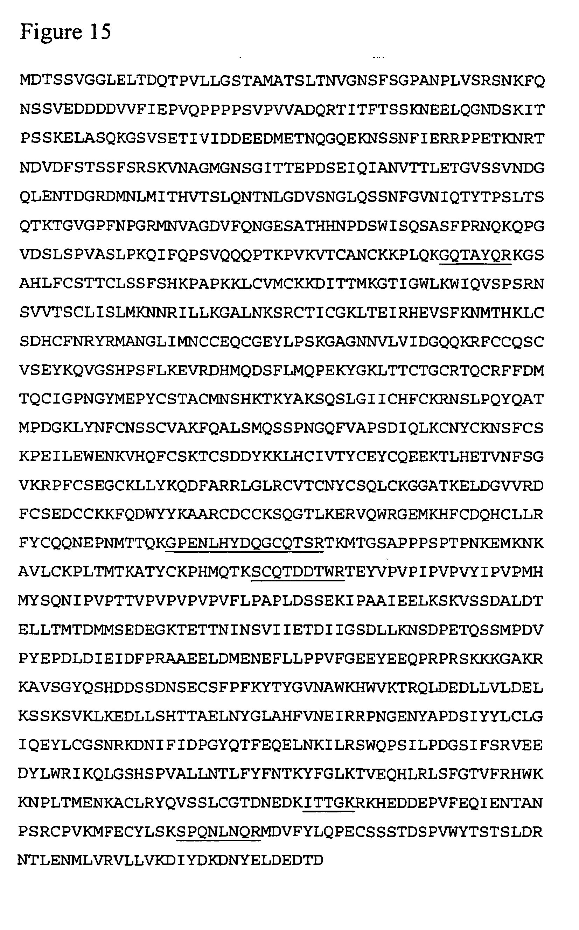

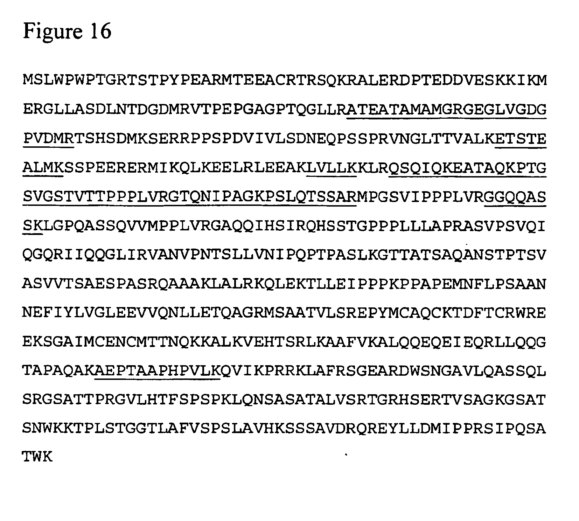

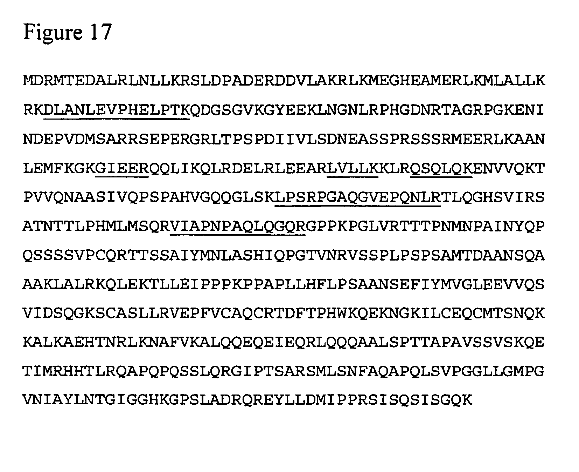

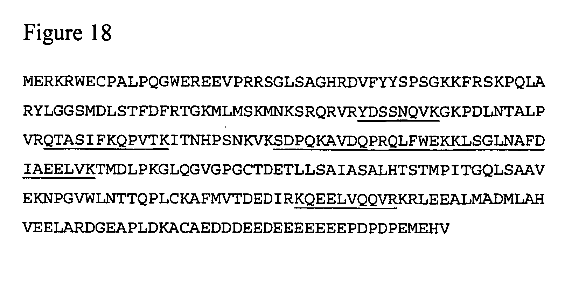

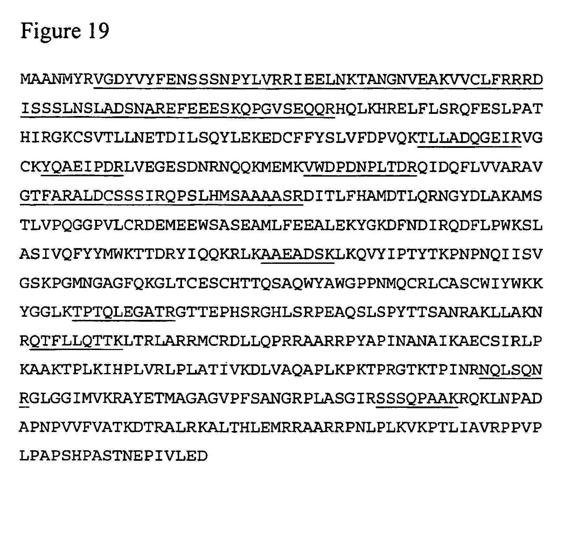

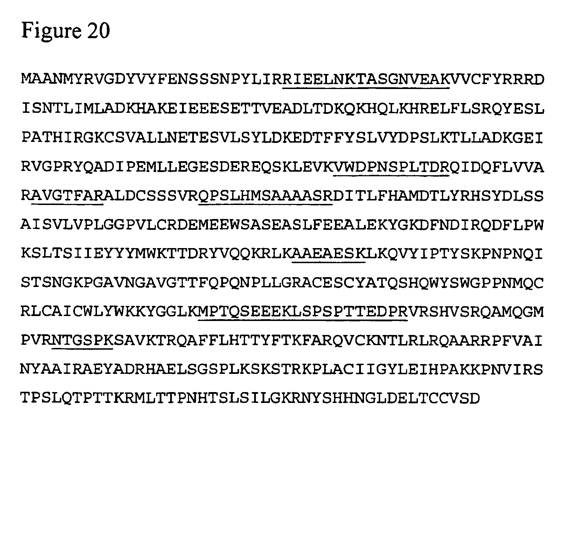

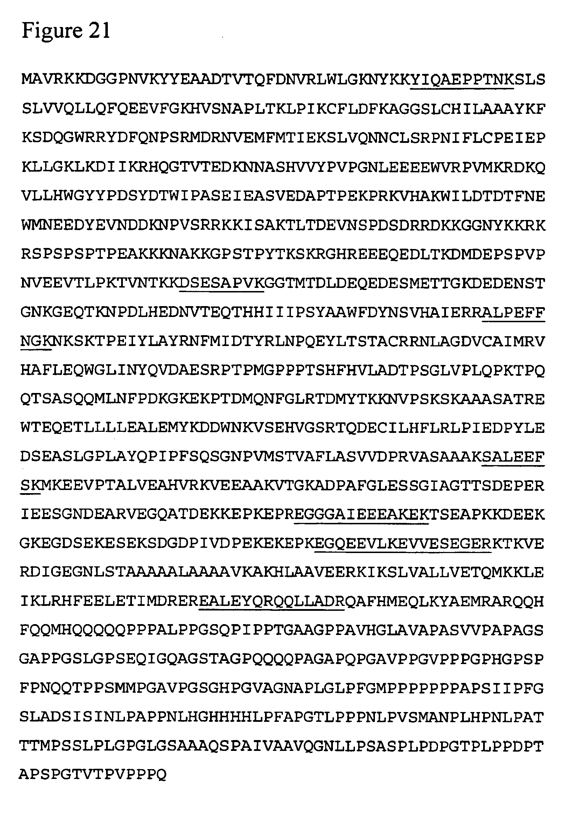

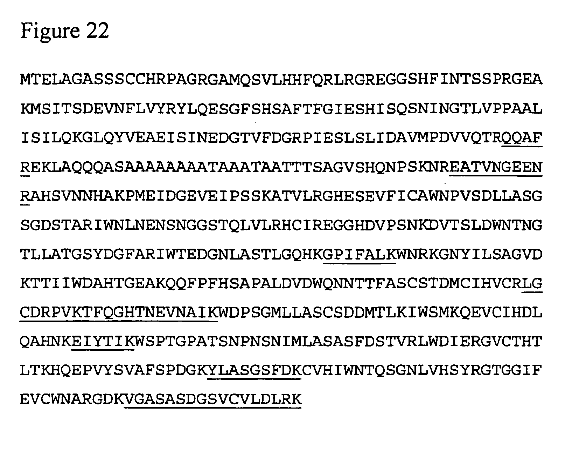

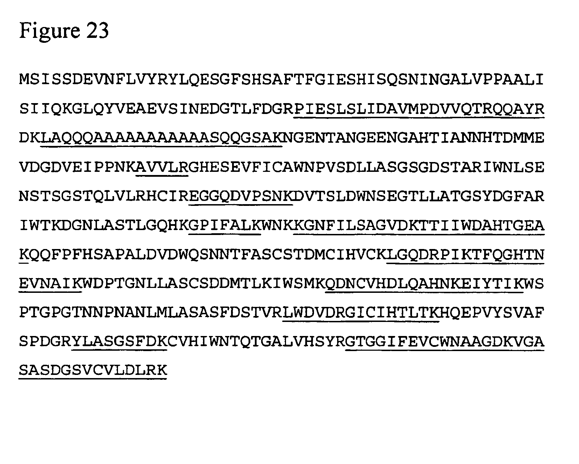

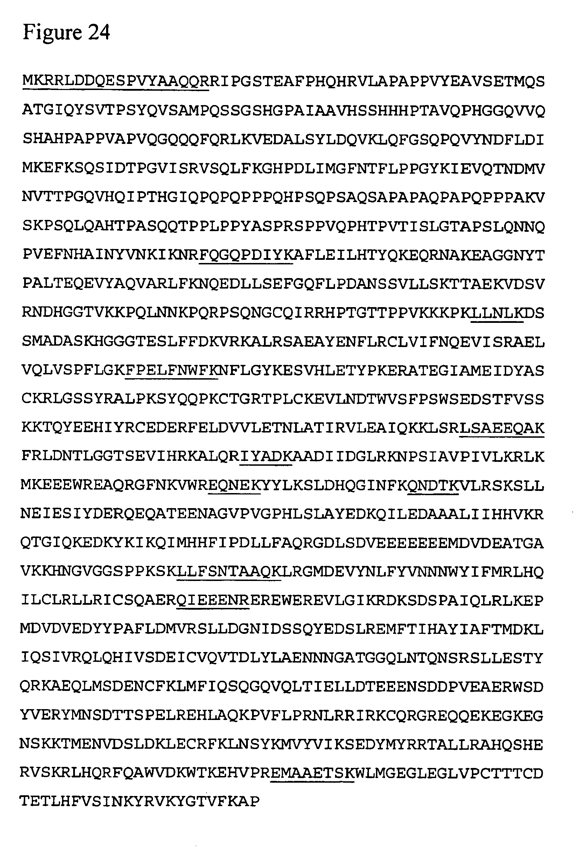

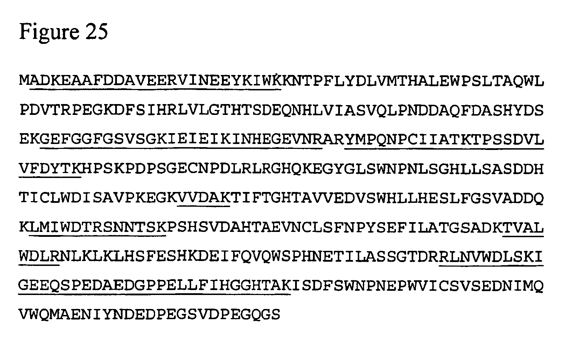

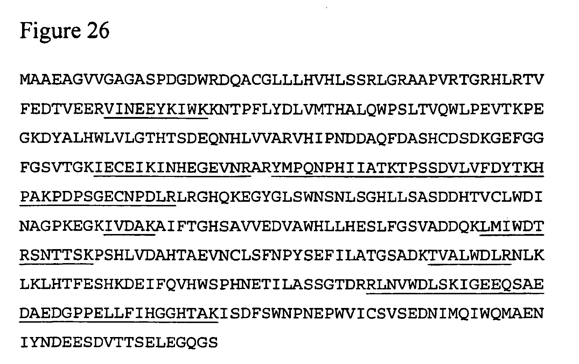

The identified HDACs and interactors are shown in Table 6 including the IC₅₀ values. In total six different HDACs were identified. For illustration, the identified peptides for HDAC1, HDAC2, HDAC3, HDAC6, HDAC8 and HDAC10 are shown in Figures 3 to 8. In addition, sequences of HDAC proteins complex components with idendified peptides underlined, are shown in Figures 9 to 26. Sequence identifiers are defined by the International Protein Index (IPI) (Kersey et al., 2004. Proteomics 4(7): 1985-1988).

### 1. Cell culture

In this example K-562 cell lysate was used (Bantscheff et al., 2007. Nature Biotechnology 25, 1035-1044). K-562 cells (American Type Culture Collection-No. CCL-243) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106). Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

Cells were homogenized in a Potter S homogenizer in lysis buffer: 50 mM Tris-HCl, 0.8% NP40, 5% glycerol, 150 mM NaCl, 1.5 mM MgCl₂, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 20 times using a mechanized POTTER S, transferred to 50 ml falcon tubes, incubated for 30 minutes rotating at 4° C and spun down for 10 minutes at 20,000 x g at 4°C (10,000 rpm in Sorvall SLA600, precooled). The supernatant was transferred to an ultracentrifuge (UZ)-polycarbonate tube (Beckmann, 355654) and spun for 1 hour at 145.000 x g at 4°C (40.000 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Capturing of proteins from cell lysate

Sepharose-beads with the immobilized compound (35 µl beads per pull-down experiment) were equilibrated in lysis buffer and incubated with a cell lysate sample containing 5 mg of protein on an end-over-end shaker (Roto Shake Genie, Scientific Industries Inc.) for 2 hours at 4°C. Beads were collected, transferred to Mobicol-columns (MoBiTech 10055) and washed with 10 ml lysis buffer containing 0.4% NP40 detergent, followed by 5 ml lysis buffer containing 0.2 % detergent. To elute bound proteins, 60 µl 2x SDS sample buffer was added to the column. The column was incubated for 30 minutes at 50°C and the eluate was transferred to a siliconized microfuge tube by centrifugation. Proteins were then alkylated with 108 mM iodoacetamid. Proteins were then separated by SDS-Polyacrylamide electrophoresis (SDS-PAGE).

### 4. Protein Identification by Mass Spectrometry

### 4.1 Protein digestion prior to mass spectrometric analysis

Gel-separated proteins were digested in-gel essentially following a previously described procedure (Shevchenko et al., 1996, Anal. Chem. 68:850-858). Briefly, gel-separated proteins were excised from the gel using a clean scalpel, destained twice using 100 µl 5mM triethylammonium bicarbonate buffer (TEAB; Sigma T7408) and 40% ethanol in water and dehydrated with absolute ethanol. Proteins were subsequently digested in-gel with porcine trypsin (Promega) at a protease concentration of 10 ng/µl in 5mM TEAB. Digestion was allowed to proceed for 4 hours at 37°C and the reaction was subsequently stopped using 5 µl 5% formic acid.

### 4.2 Sample preparation prior to analysis by mass spectrometry

Gel plugs were extracted twice with 20 µl 1% formic acid and three times with increasing concentrations of acetonitrile. Extracts were subsequently pooled with acidified digest supernatants and dried in a vacuum centrifuge.

### 4.3 TMT labeling of peptide extracts

The peptide extracts of samples treated with different concentrations of SAHA (10 µM, 2.5 µM, 0.625 µM, 0.156 µM and 0.039 µM) and the solvent control (0.5% DMSO) were treated with different variants of the isobaric tagging reagent (TMTsixplex Label Reagent Set, part number 90066, Thermo Fisher Scientific Inc., Rockford, IL 61105 USA). The TMT reagents are a set of multiplexed, amine-specific, stable isotope reagents that can label peptides on amino groups in up to six different biological samples enabling simultaneous identification and quantitation of peptides. The TMT reagents were used according to instructions provided by the manufacturer. The samples were resuspended in 10 µl 50 mM TEAB solution, pH 8.5 and 10 µl acetonitril were added. The TMT reagent was dissolved in acetonitril to a final concentration of 24 mM and 10 µl of reagent solution were added to the sample. The labeling reaction was performed at room temperature for one hour on a horizontal shaker and stopped by adding 5 µl of 100 mM TEAB and 100 mM glycine in water. The labeled samples were then combined, dried in a vacuum centrifuge and resuspended in 200mM TEAB60%/40%acetonitril. 2 µl of a 2.5% NH2OH solution in water were added, incubated for 15 min and finally the reaction was stopped by addition of 10 µl of 20% formic acid in water. After freeze-drying samples were resuspended in 50 µl 0.1% formic acid in water.

### 4.4 Mass spectrometric data acquisition

Peptide samples were injected into a nano LC system (CapLC, Waters or nano-LC 1D+, Eksigent) which was directly coupled either to a quadrupole TOF (QTOF Ultima, QTOF Micro, Waters), ion trap (LTQ) or Orbitrap mass spectrometer. Peptides were separated on the LC system using a gradient of aqueous and organic solvents (see below). Solvent A was 0.1% formic acid and solvent B was 70% acetonitrile in 0.1% formic acid.

**Table 5: Peptides elution off the LC system**

| Method file | Flow | Gradient |
|---|---|---|
| | rate(nL/min) | Time(min)-%B |
| PQD_265min | 190 | 00-5.263 |
| | | 07-10 |
| | | 190-40.263 |
| | | 210-52.105 |
| | | 223-60 |
| | | 230-90 |
| | | 236-90 |
| | | 240-5.263 |
| | | 260-5.263 |

### 4.5 Protein identification

The peptide mass and fragmentation data generated in the LC-MS/MS experiments were used to query a protein data base consisting of an in-house curated version of the International Protein Index (IPI) protein sequence database combined with a decoy version of this database (Elias and Gygi, 2007, Target-decoy search strategy for increased confidence in large-scale protein identifications by mass spectrometry. Nature Methods 4, 207-214). Proteins were identified by correlating the measured peptide mass and fragmentation data with data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis. Protein acceptance thresholds were adjusted to achieve a false discovery rate of below 1% as suggested by hit rates on the decoy data base (Elias and Gygi, 2007, Target-decoy search strategy for increased confidence in large-scale protein identifications by mass spectrometry. Nature Methods 4, 207-214).

### 4.6 Protein quantitation

Relative protein quantitation was performed using peak areas of iTMT reporter ion signals essentially as described in an earlier publication (Bantscheff et al., 2007. Nature Biotechnology 25, 1035-1044).

**Table 6: Selectivity profile for SAHA (K562 cells; experiment X003787)**

| **Representative Sequence** | **Protein Name** | **Protein Complex** | **Protein Function** | **Redun dant Peptide s** | **Quantified Spectra** | **IC₅₀ (µM)** |
|---|---|---|---|---|---|---|
| IPI00013774.1 | HDAC1 | CLASS I HDAC | HDAC | 133 | 86 | 0.90 |
| IPI00289601.10 | HDAC2 | CLASS I HDAC | HDAC | 232 | 76 | 0.93 |
| IPI00217965.1 | HDAC3 | CLASS I HDAC | HDAC | 120 | 38 | 2.56 |
| IPI00747259.2 | HDAC8 | CLASS I HDAC | HDAC | 51 | 17 | 10.00 |
| IPI00012439.5 | HDAC10 | CLASS II HDAC | HDAC | 51 | 14 | 10.00 |
| IPI00005711.4 | HDAC6 | CLASS II HDAC | HDAC | 83 | 81 | 0.32 |
| IPI00217540.7 | AOF2 | CoREST | HDAC Interactor | 214 | 191 | 0.54 |
| IPI00215963.5 | GSE1 | CoREST | HDAC Interactor | 306 | 88 | 0.64 |
| IPI00018924.3 | HMG20A | CoREST | HDAC Interactor | 33 | 21 | 0.35 |
| IPI00464951.5 | HMG20B | CoREST | HDAC Interactor | 17 | 13 | 1.20 |
| IPI00008531.1 | RCOR1 | CoREST | HDAC Interactor | 85 | 64 | 0.48 |
| IPI00914887.1 | RCOR3 | CoREST | HDAC Interactor | 164 | 60 | 0.39 |
| IPI00294603.6 | ZMYM2 | CoREST | HDAC Interactor | 15 | 13 | 1.67 |
| IPI00478128.2 | GATAD2A | Mi-2/NuRD | HDAC Interactor | 78 | 20 | 0.79 |
| IPI00103554.1 | GATAD2B | Mi-2/NuRD | HDAC Interactor | 18 | 8 | 2.50 |
| IPI00439194.1 | MBD3 | Mi-2/NuRD | HDAC Interactor | 40 | 19 | 0.61 |
| IPI00171798.1 | MTA2 | Mi-2/NuRD | HDAC Interactor | 42 | 29 | 10.00 |
| IPI00165357.4 | MTA3 | Mi-2/NuRD | HDAC Interactor | 64 | 4 | 1.93 |
| IPI00216047.3 | SMARCC2 | SMRT/NCoR | HDAC Interactor | 19 | 14 | 10.00 |
| IPI00640917.1 | TBL1X | SMRT/NCoR | HDAC Interactor | 56 | 4 | 0.29 |
| IPI00002922.5 | TBL1XR1 | SMRT/NCoR | HDAC Interactor | 55 | 29 | 0.29 |
| IPI00170596.1 | SIN3A | Sin3/CoREST | HDAC Interactor | 26 | 18 | 0.57 |
| IPI00328319.8 | RBBP4 | Sin3/Mi2-NuRD | HDAC Interactor | 130 | 29 | 0.79 |
| IPI00646512.1 | RBBP7 | Sin3/Mi2-NuRD | HDAC Interactor | 55 | 19 | 10.00 |
| IPI00006663.1 | ALDH2 | | | 195 | 151 | 0.63 |
| IPI00022305.4 | BZW2 | | | 108 | 31 | 3.07 |
| IPI00057097.3 | DNTTIP1 | | | 7 | 6 | 0.48 |
| IPI00784154.1 | HSPD1 | | | 152 | 132 | 10.00 |
| IPI00304082.8 | ISOC1 | | | 103 | 93 | 1.10 |
| IPI00003031.3 | ISOC2 | | | 106 | 74 | 0.24 |
| IPI00012833.1 | PPP4C | | | 55 | 32 | 10.00 |
| IPI00100933.1 | PTER | | | 90 | 38 | 10.00 |

### Example 3: HDAC experiment using an immobilized compound and a Molt-4 cell lysate with Trichostatin A

This example illustrates the use of a competition binding assay in cell lysate to identify and characterize HDAC protein complexes and to establish the selectivity profile of the test compound Trichostatin A (Sigma-Aldrich). This compound was added at defined concentrations (10 µM, 2.5 µM, 0.625 µM, 0.156 µM and 0.039 µM) to aliquots of Molt-4 cell lysates thereby allowing the test compound to bind to the target proteins in the lysate. Then the lysate was contacted with the immobilized compound (Figure 2) to capture remaining free target proteins. The proteins bound to the immobilized compound were eluted with detergent-containing buffer, separated on a SDS-polyacryamide gel and analyzed by mass spectrometry as described in example 2.

**Table 7: Selectivity profile for Trichostatin A (Molt-4; experiment X003750)**

| **Representative Sequence** | **Protein Name** | **Protein Complex** | **Protein Function** | **Redundant Peptides** | **Quantified Spectra** | **IC₅₀ (µM)** |
|---|---|---|---|---|---|---|
| IPI00013774.1 | HDAC1 | CLASS I HDAC | HDAC | 308 | 98 | 0.04 |
| IPI00289601.10 | HDAC2 | CLASS I HDAC | HDAC | 259 | 80 | 0.05 |
| IPI00217965.1 | HDAC3 | CLASS I HDAC | HDAC | 153 | 49 | 0.09 |
| IPI00747259.2 | HDAC8 | CLASS I HDAC | HDAC | 33 | 11 | 3.00 |
| IPI00012439.5 | HDAC10 | CLASS II HDAC | HDAC | 42 | 7 | 3.00 |
| IPI00005711.4 | HDAC6 | CLASS II HDAC | HDAC | 110 | 94 | 0.40 |
| IPI00217540.7 | AOF2 | CoREST | HDAC Interactor | 292 | 134 | 0.02 |
| IPI00215963.5 | GSE1 | CoREST | HDAC Interactor | 195 | 55 | 0.02 |
| IPI00018924.3 | HMG20A | CoREST | HDAC Interactor | 14 | 9 | 0.03 |
| IPI00878364.1 | HMG20B | CoREST | HDAC Interactor | 8 | 8 | 0.02 |
| IPI00008531.1 | RCOR1 | CoREST | HDAC Interactor | 78 | 65 | 0.02 |
| IPI00914887.1 | RCOR3 | CoREST | HDAC Interactor | 106 | 37 | 0.02 |
| IPI00294603.6 | ZMYM2 | CoREST | HDAC Interactor | 14 | 12 | 0.04 |
| IPI00478128.2 | GATAD2 A | Mi-2/NuRD | HDAC Interactor | 66 | 22 | 0.04 |
| IPI00103554.1 | GATAD2 B | Mi-2/NuRD | HDAC Interactor | 22 | 13 | 0.06 |
| IPI00439194.1 | MBD3 | Mi-2/NuRD | HDAC Interactor | 60 | 24 | 0.04 |
| IPI00012773.1 | MTA1 | Mi-2/NuRD | HDAC Interactor | 108 | 4 | 0.06 |
| IPI00171798.1 | MTA2 | Mi-2/NuRD | HDAC Interactor | 50 | 25 | 0.06 |
| IPI00165357.4 | MTA3 | Mi-2/NuRD | HDAC Interactor | 168 | 19 | 0.03 |
| IPI00012301.1 | GPS2 | SMRT/NCoR | HDAC Interactor | 16 | 5 | 0.10 |
| IPI00640917.1 | TBL1X | SMRT/NCoR | HDAC Interactor | 80 | 4 | 0.02 |
| IPI00002922.5 | TBL1XR1 | SMRT/NCoR | HDAC Interactor | 86 | 44 | 0.07 |
| IPI00170596.1 | SIN3A | Sin3/CoREST | HDAC Interactor | 26 | 21 | 0.06 |
| IPI00328319.8 | RBBP4 | Sin3/Mi2-NuRD | HDAC Interactor | 122 | 24 | 0.05 |
| IPI00646512.1 | RBBP7 | Sin3/Mi2-NuRD | HDAC Interactor | 55 | 16 | 0.12 |
| IPI00006663.1 | ALDH2 | | | 38 | 36 | 3.00 |
| IPI00872929.2 | APOBEC 3C | | | 10 | 5 | 3.00 |
| IPI00022305.4 | BZW2 | | | 58 | 26 | 2.45 |
| IPI00057097.3 | DNTTIP1 | | | 11 | 10 | 0.04 |
| IPI00784154.1 | HSPD1 | | | 124 | 114 | 3.00 |
| IPI00304082.8 | ISOC1 | | | 135 | 111 | 3.00 |
| IPI00006408.4 | NOSIP | | | 6 | 5 | 3.00 |
| IPI00012833.1 | PPP4C | | | 62 | 40 | 3.00 |
| IPI00100933.1 | PTER | | | 90 | 39 | 3.00 |

### Example 4: HDAC experiment using an immobilized compound and a K-562 cell lysate with Trichostatin A

This example illustrates the use of a competition binding assay in cell lysate to identify and characterize HDAC protein complexes and to establish the selectivity profile of the test compound Trichostatin A (Sigma-Aldrich). This compound was added at defined concentrations (10 µM, 2.5 µM, 0.625 µM, 0.156 µM and 0.039 µM) to aliquots of K-562 cell lysates thereby allowing the test compound to bind to the target proteins in the lysate. Then the lysate was contacted with the immobilized compound (Figure 2) to capture remaining free target proteins. The proteins bound to the immobilized compound were eluted with detergent-containing buffer, separated on a SDS-polyacryamide gel and analyzed by mass spectrometry as described in example 2.

**Table 8: Selectivity profile for Trichostatin A (K-562; experiment X003754)**

| **Representative Sequence** | **Protein Name** | **Protein Complex** | **Protein Function** | **Redundant Peptides** | **Quantified Spectra** | **IC₅₀ (µM)** |
|---|---|---|---|---|---|---|
| IPI00013774.1 | HDAC1 | CLASS I HDAC | HDAC | 126 | 72 | 0.03 |
| IPI00289601.10 | HDAC2 | CLASS I HDAC | HDAC | 256 | 81 | 0.05 |
| IPI00217965.1 | HDAC3 | CLASS I HDAC | HDAC | 114 | 34 | 0.13 |
| IPI00747259.2 | HDAC8 | CLASS I HDAC | HDAC | 28 | 7 | 3.00 |
| IPI00012439.5 | HDAC10 | CLASS II | HDAC | 45 | 14 | 3.00 |
| | | HDAC | | | | |
| IPI00005711.4 | HDAC6 | CLASS II HDAC | HDAC | 108 | 99 | 0.64 |
| IPI00217540.7 | AOF2 | CoREST | HDAC Interactor | 175 | 157 | 0.03 |
| IPI00215963.5 | GSE1 | CoREST | HDAC Interactor | 99 | 88 | 0.03 |
| IPI00018924.3 | HMG20A | CoREST | HDAC Interactor | 42 | 28 | 0.02 |
| IPI00464951.5 | HMG20B | CoREST | HDAC Interactor | 24 | 19 | 0.03 |
| IPI00008531.1 | RCOR1 | CoREST | HDAC Interactor | 79 | 60 | 0.02 |
| IPI00914887.1 | RCOR3 | CoREST | HDAC Interactor | 146 | 50 | 0.03 |
| IPI00294603.6 | ZMYM2 | CoREST | HDAC Interactor | 24 | 10 | 0.06 |
| IPI00478128.2 | GATAD2A | Mi-2/NuRD | HDAC Interactor | 57 | 17 | 0.06 |
| IPI00103554.1 | GATAD2B | Mi-2/NuRD | HDAC Interactor | 12 | 10 | 0.06 |
| IPI00439194.1 | MBD3 | Mi-2/NuRD | HDAC Interactor | 38 | 16 | 0.05 |
| IPI00171798.1 | MTA2 | Mi-2/NuRD | HDAC Interactor | 11 | 7 | 0.07 |
| IPI00165357.4 | MTA3 | Mi-2/NuRD | HDAC Interactor | 68 | 14 | 0.06 |
| IPI00216047.3 | SMARCC2 | SMRT/NCoR | HDAC Interactor | 28 | 12 | 3.00 |
| IPI00640917.1 | TBL1X | SMRT/NCoR | HDAC Interactor | 72 | 8 | 0.05 |
| IPI00002922.5 | TBL1XR1 | SMRT/NCoR | HDAC | 57 | 21 | 0.07 |
| | | | Interactor | | | |
| IPI00170596.1 | SIN3A | Sin3/CoREST | HDAC Interactor | 16 | 14 | 0.10 |
| IPI00328319.8 | RBBP4 | Sin3/Mi2-NuRD | HDAC Interactor | 128 | 30 | 0.05 |
| IPI00646512.1 | RBBP7 | Sin3/Mi2-NuRD | HDAC Interactor | 144 | 12 | 0.18 |
| IPI00006663.1 | ALDH2 | | | 293 | 248 | 3.00 |
| IPI00872929.2 | APOBEC3C | | | 30 | 15 | 3.00 |
| IPI00022305.4 | BZW2 | | | 84 | 35 | 2.79 |
| IPI00057097.3 | DNTTIP1 | | | 12 | 11 | 0.05 |
| IPI00784154.1 | HSPD1 | | | 160 | 143 | 3.00 |
| IPI00304082.8 | ISOC1 | | | 220 | 174 | 3.00 |
| IPI00003031.3 | ISOC2 | | | 237 | 171 | 3.00 |
| IPI00006408.4 | NOSIP | | | 7 | 4 | 3.00 |
| IPI00012833.1 | PPP4C | | | 27 | 16 | 3.00 |
| IPI00100933.1 | PTER | | | 100 | 43 | 3.00 |
| IPI00005492.2 | WDR5 | | | 6 | 6 | 0.42 |

### Example 5: HDAC experiment using an immobilized compound and a mix of Jurkat and Ramos cell lysates with SAHA

This example illustrates the use of a competition binding assay in cell lysate to identify and characterize HDAC protein complexes and to establish the selectivity profile of the test compound SAHA (vorinostat, suberoylanilide hydroxamic acid). This compound was added at defined concentrations (10 µM, 2.5 µM, 0.625 µM, 0.156 µM and 0.039 µM) to aliquots of a mix of Jurkat and Ramos cell lysates thereby allowing the test compound to bind to the target proteins in the lysate. Then the lysate was contacted with the immobilized compound (Figure 2) to capture remaining free target proteins. The proteins bound to the immobilized compound were eluted with detergent-containing buffer, separated on a SDS-polyacryamide gel and analyzed by mass spectrometry as described in example 2.

For the preparation of lysates, Jurkat cells (ATCC number TIB-152) and Ramos cells (ATCC number CRL-1596) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106) at a density between 0.2 x 10⁶ and 1.0 x 10⁶ cells/ml. Cells were harvested by centrifugation, washed once with 1 x PBS buffer (Invitrogen, #14190-094) and cell pellets were frozen in liquid nitrogen and subsequently stored at -80°C.

Examples of dose response curves for individual proteins are shown in Figures 27 to 29.

**Table 9: Selectivity profile for SAHA (Jurkat, Ramos cells; experiment X003475)**

| **Representative Sequence** | **Protein Name** | **Protein Complex** | **Protein Function** | **Redundant Peptides** | **Quantified Spectra** | **IC₅₀ (µM)** |
|---|---|---|---|---|---|---|
| IPI00013774.1 | HDAC1 | CLASS I HDAC | HDAC | 52 | 25 | 0.78 |
| IPI00289601.10 | HDAC2 | CLASS I HDAC | HDAC | 42 | 15 | 2.81 |
| IPI00005711.4 | HDAC6 | CLASS II HDAC | HDAC | 24 | 22 | 0.20 |
| IPI00217540.7 | AOF2 | CoREST | HDAC Interactor | 82 | 34 | 0.14 |
| IPI00008531.1 | RCOR1 | CoREST | HDAC Interactor | 12 | 8 | 0.16 |
| IPI00914887.1 | RCOR3 | CoREST | HDAC Interactor | 16 | 4 | 0.15 |
| IPI00171798.1 | MTA2 | Mi-2/NuRD | HDAC Interactor | 20 | 13 | 0.53 |
| IPI00165357.4 | MTA3 | Mi-2/NuRD | HDAC Interactor | 10 | 4 | 1.82 |
| IPI00216047.3 | SMARCC 2 | SMRT/NCoR | HDAC Interactor | 26 | 6 | 10.0 0 |
| IPI00002922.5 | TBL1XR1 | SMRT/NCoR | HDAC Interactor | 12 | 10 | 0.22 |
| IPI00328319.8 | RBBP4 | Sin3/Mi2-NuRD | HDAC Interactor | 34 | 6 | 0.24 |
| IPI00006663.1 | ALDH2 | | | 52 | 23 | 4.81 |
| IPI00022305.4 | BZW2 | | | 8 | 7 | 10.0 0 |
| IPI00784154.1 | HSPD1 | | | 130 | 58 | 10.0 0 |
| IPI00304082.8 | ISOC1 | | | 76 | 29 | 2.19 |
| IPI00003031.3 | ISOC2 | | | 25 | 19 | 0.22 |
| IPI00012833.1 | PPP4C | | | 12 | 7 | 10.0 0 |
| IPI00100933.1 | PTER | | | 9 | 8 | 10.0 0 |

### Example 6: Comparison of IC₅₀ inhibition values for Trichostatin A with literature data

This examples shows a comparison of IC₅₀ inhibition values for the HDAC inhibitor Trichostatin A, a hydroxamic acid, as determined by the present invention using endogenous HDACs (example 3: Molt4 cell lysate; example 4: K-562 cell lysate) with inhibition values reported in the literature which were generated in conventional *in vitro* HDAC enzyme assays (Blackwell et al., 2008. Life Sciences 82(21-22):1050-1058; Khan et al., 2008. Biochemical Journal 409(2):581-589).

Blackwell and colleagues used HDAC isoforms expressed as 6x His-tagged fusion proteins in a baculovirus system in Sf9 insect cells (HDACs 1, 2, 3, 6 and 8 were expressed as full length fusion proteins; the HDAC 10 fusion protein was expressed as a carboxy-terminal deletion of 38 amino acids, residues 632-669) and amino terminal carboxyfluorescein (FAM) labelled acetylated peptides as enzyme substrates (Blackwell et al., 2008. Life Sciences 82(21-22):1050-1058).

Khan and colleagues used GST-tagged or FLAG-epitope tagged recombinant human HDACs in insect Sf9 cells with a baculoviral expression system and a Fluor de Lys™ (Biomol International) HDAC assay (Khan et al., 2008. Biochemical Journal 409(2):581-589).

**Table 10: Comparison of IC₅₀ values for Trichostatin A.**

| IC₅₀ values that could not be derived (as maximal inhibition could not be achieved) are represented as greater than the highest concentration used in the assay. Conversely, if maximal inhibition was achieved at the lowest concentration the value is designated less than the lowest concentration measured. HDACs in which IC₅₀ values were not obtained are designate "ND" for not determined. | | | | | |
|---|---|---|---|---|---|
| **HDAC** | **HDAC class** | **IC₅₀ (nM) Molt4 Example 3** | **IC₅₀ (nM) K-562 Example 4** | **IC₅₀ (nM) Blackwell et al., 2008** | **IC₅₀ (nM) Khan et al., 2008** |
| **HDAC1** | **I** | **40** | **34** | **5** | **2** |
| **HDAC2** | **I** | **50** | **45** | **21** | **3** |
| **HDAC3** | **I** | **90** | **125** | **<5** | **4** |
| **HDAC6** | **IIb** | **400** | **635** | **<5** | **3** |
| **HDAC8** | **I** | **>3000** | **>3000** | **1100** | **456** |
| **HDAC10** | **IIb** | **>3000** | **>3000** | **5** | **ND** |

The first observation is that IC₅₀ values as determined in the present invention are generally higher (less potent) than the values obtained with recombinant HDAC enzymes. Nonetheless, values are in good agreement for the two independent experiments performed in Molt 4 and K-562 cell lysates.

The second observation is that different selectivity profiles for Trichostatin A are obtained by the two approaches. For example, Blackwell et al. and Khan et al. report for HDAC6 (class IIb HDAC) very potent inhibition similar to HDAC1 (class I HDAC) whereas in the present method HDAC6 is tenfold (Molt4) or eighteenfold (K-562) less sensitive to inhibition with Trichostatin A than HDAC1. In addition, Blackwell reports an IC₅₀ value of 5 nM for HDAC10 (class IIb HDAC) whereas no significant inhibition was measured in Example 3 or 4 for HDAC10.

### Example 7: HDAC experiment using an immobilized compound and a K-562 cell lysate with HDAC inhibitor MS-275

This example illustrates the use of a competition binding assay in cell lysate to identify and characterize HDAC protein complexes and to establish the selectivity profile of the HDAC inhibitor MS-275 (Suzuki et al., 1999. J. Med. Chem. 1999; US6,174,905). This compound was added at defined concentrations (300 µM, 75 µM, 18.75 µM, 4.69 µM and 1.18 µM) to aliquots of K-562 cell lysates thereby allowing the MS-275 compound to bind to the target proteins in the lysate. Then the lysate was contacted with the immobilized compound (Figure 2) to capture remaining free target proteins. The proteins bound to the immobilized compound were eluted with detergent-containing buffer, separated on a SDS-polyacryamide gel and analyzed by mass spectrometry as described in example 2.

**Table 11: Selectivity profile for MS-275 (K-562; experiment X010500)**

| **Representative Sequence** | **Protein Name** | **Protein Complex** | **Protein Function** | **Redundant Peptides** | **Quantified Spectra** | **IC₅₀ (µM)** |
|---|---|---|---|---|---|---|
| IPI00013774.1 | HDAC1 | CLASS I HDAC | HDAC | 286 | 77 | 300.00 |
| IPI00289601.10 | HDAC2 | CLASS I HDAC | HDAC | 334 | 105 | 300.00 |
| IPI00217965.1 | HDAC3 | CLASS I HDAC | HDAC | 117 | 36 | 72.79 |
| IPI00747259.2 | HDAC8 | CLASS I HDAC, | HDAC | 27 | 9 | 300.00 |
| IPI00012439.5 | HDAC10 | CLASS II HDAC | HDAC | 126 | 20 | 300.00 |
| IPI00005711.4 | HDAC6 | CLASS II HDAC | HDAC | 140 | 129 | 300.00 |
| IPI00217540.7 | AOF2 | CoREST | HDAC Interactor | 568 | 256 | 300.00 |
| IPI00293963.4 | CDYL | CoREST | HDAC Interactor | 20 | 4 | 300.00 |
| IPI00215963.5 | GSE1 | CoREST | HDAC Interactor | 144 | 125 | 300.00 |
| IPI00018924.3 | HMG20A | CoREST | HDAC Interactor | 65 | 46 | 300.00 |
| IPI00464951.5 | HMG20B | CoREST | HDAC Interactor | 32 | 25 | 300.00 |
| IPI00008531.1 | RCOR1 | CoREST | HDAC Interactor | 117 | 97 | 300.00 |
| IPI00914887.1 | RCOR3 | CoREST | HDAC Interactor | 186 | 73 | 300.00 |
| IPI00478128.2 | GATAD2A | Mi-2/NuRD | HDAC Interactor | 54 | 16 | 300.00 |
| IPI00103554.1 | GATAD2B | Mi-2/NuRD | HDAC Interactor | 26 | 16 | 300.00 |
| IPI00439194.1 | MBD3 | Mi-2/NuRD | HDAC Interactor | 78 | 35 | 300.00 |
| IPI00171798.1 | MTA2 | Mi-2/NuRD | HDAC Interactor | 97 | 85 | 300.00 |
| IPI00165357.4 | MTA3 | Mi-2/NuRD | HDAC Interactor | 70 | 18 | 300.00 |
| IPI00012301.1 | GPS2 | SMRT/NCoR | HDAC Interactor | 14 | 7 | 219.49 |
| IPI00216047.3 | SMARCC2 | SMRT/NCoR | HDAC lnteractor | 20 | 6 | 300.00 |
| IPI00640917.1 | TBL1X | SMRT/NCoR | HDAC Interactor | 74 | 10 | 78.00 |
| IPI00002922.5 | TBL1XR1 | SMRT/NCoR | HDAC Interactor | 60 | 30 | 60.14 |
| IPI00022019.1 | SAP30 | Sin3 | HDAC Interactor | 11 | 11 | 300.00 |
| IPI00170596.1 | SIN3A | Sin3/CoREST | HDAC Interactor | 29 | 24 | 300.00 |
| IPI00328319.8 | RBBP4 | Sin3/Mi2-NuRD | HDAC Interactor | 114 | 26 | 300.00 |
| IPI00646512.1 | RBBP7 | Sin3/Mi2-NuRD | HDAC Interactor | 141 | 14 | 300.00 |
| IPI00006663.1 | ALDH2 | | | 296 | 264 | 300.00 |
| IPI00872929.2 | APOBEC3C | | | 46 | 21 | 300.00 |
| IPI00022305.4 | BZW2 | | | 120 | 50 | 300.00 |
| IPI00057097.3 | DNTTIP1 | | | 21 | 18 | 300.00 |
| IPI00784154.1 | HSPD1 | | | 190 | 175 | 300.00 |
| IPI00304082.8 | ISOC1 | | | 325 | 280 | 300.00 |
| IPI00003031.3 | ISOC2 | | | 424 | 359 | 300.00 |
| IPI00006408.4 | NOSIP | | | 7 | 7 | 300.00 |
| IPI00012833.1 | PPP4C | | | 36 | 26 | 300.00 |
| IPI00100933.1 | PTER | | | 126 | 61 | 300.00 |
| IPI00005492.2 | WDR5 | | | 30 | 25 | 300.00 |

### Example 8: Comparison of IC₅₀ inhibition values for MS-275 with literature data

This example shows a comparison of IC₅₀ inhibition values for the HDAC inhibitor MS-275, a benzamide compound (Suzuki et al., 1999. J. Med. Chem. 1999; US6,174,905), as determined by the present invention using endogenous HDACs (example 7: K-562 cell lysate) with inhibition values reported in the literature which were generated in conventional *in vitro* HDAC enzyme assays (Blackwell et al., 2008. Life Sciences 82(21-22):1050-1058; Khan et al., 2008. Biochemical Journal 409(2):581-589).

Blackwell and colleagues used HDAC isoforms expressed as 6x His-tagged fusion proteins in a baculovirus system in Sf9 insect cells (HDACs 1, 2, 3, 6 and 8 were expressed as full length fusion proteins; the HDAC 10 fusion protein was expressed as a carboxy-terminal deletion of 38 amino acids, residues 632-669) and amino terminal carboxyfluorescein (FAM) labelled acetylated peptides as enzyme substrates (Blackwell et al., 2008. Life Sciences 82(21-22):1050-1058).

Khan and colleagues used GST-tagged or FLAG-epitope tagged recombinant human HDACs in insect Sf9 cells with a baculoviral expression system and a Fluor de Lys™ (Biomol International) HDAC assay (Khan et al., 2008. Biochemical Journal 409(2):581-589).

**Table 12: Comparison of IC₅₀ values for MS-275.**

| IC₅₀ values that could not be derived (as maximal inhibition could not be achieved) are represented as greater than the highest concentration used in the assay. Conversely, if maximal inhibition was achieved at the lowest concentration the value is designated less than the lowest concentration measured. HDACs in which IC₅₀ values were not obtained are designate "ND" for not determined. | | | | |
|---|---|---|---|---|
| **HDAC** | **HDAC class** | **IC₅₀ (µM) K-562 Example 7** | **IC₅₀ (µM) Blackwell et al., 2008** | **IC₅₀ (µM) Khan et** al., **2008** |
| **HDAC1** | **I** | **>300** | **13** | **0.181** |
| **HDAC2** | **I** | **>300** | **0.51** | **1.155** |
| **HDAC3** | **I** | **73** | **0.07** | **2.311** |
| **HDAC6** | **IIb** | **>300** | **21** | **>10** |
| **HDAC8** | **I** | **>300** | **>30** | **>10** |
| **HDAC1 0** | **IIb** | **>300** | **11.5** | **ND** |

In the present method MS-275 inhibits of the six detected HDACs only HDAC3 with an IC₅₀ value of 73 µM. Blackwell et al. observe a less selective profile with HDAC3 as most sensitive to MS-275 inhibition followed by HDAC2, 10, 1 and 8. Khan et al. find that MS-275 is most potent for HDAC 1 followed by HDAC2 and HDAC3.

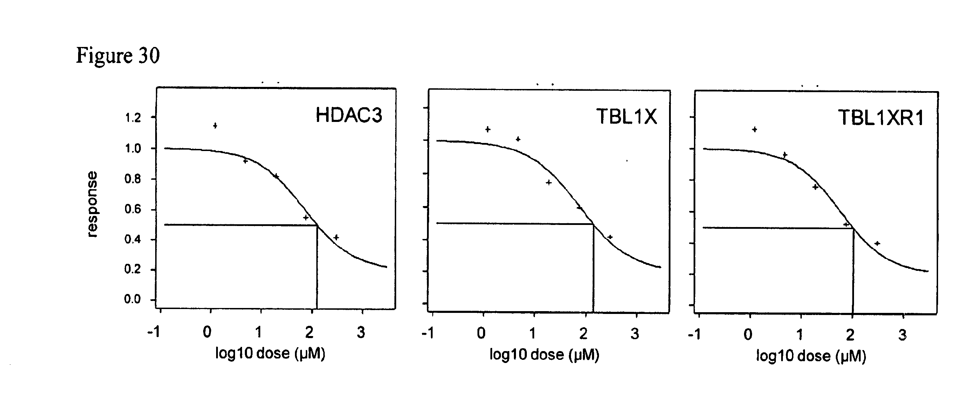

The finding of the present study that MS-275 selectively inhibits HDAC3 is further supported by the inhibition values for the NCoR complex components TBL1X (IC₅₀= 78 µM), TBL1XR1 (IC₅₀= 60 µM), and GPS2 (IC₅₀= 219 µM) (Table 11 and Figure 30). No other HDAC complex components detected in this experiment are affected by MS-275 (Table 11). It is known that HDAC3 forms the core of the NCoR/SMRT complex (Yang and Seto, 2008. Nat. Rev. Mol. Cell Biol. 9(3):206-218, see Table 1).

### Example 9: Chemoproteomics profiling of HDAC inhibitors reveals targeting of multiple HDAC complexes with compound class-dependent selectivity

Histone deacetylase inhibitors (HDACi) display marked anti-cancer and anti-inflammatory properties but the development of isoform-selective drugs has proven challenging.

This example shows the chemoproteomics profiling of 16 HDACi using a hydroxamate affinity matrix and quantitative mass spectrometry reveals that these drugs target multiple HDAC complexes built around ELM-SANT domain scaffolds, including a novel mitotic deacetylase complex (MiDAC). The targeted complexes were further characterized by quantitative immunoaffinity purifications. Inhibitors cluster in distinct groups by their target profiles with aminobenzamides showing preferential binding to the HDAC3 / NCoR complex, and several non-HDAC targets for the hydroxamate class were identified. Distinct inhibitor target profiles correlate with differential effects on downstream targets. Application of the approach to compound screening identified the anti-inflammatory drug bufexamac as a Class IIb (HDAC6; HDAC10) inhibitor. Our approach enables the discovery of both novel targets and inhibitors and suggests that compound selectivity should be considered in the context of protein complexes.

Protein lysine acetylation is a key mechanism in the epigenetic control of gene expression and the regulation of cell metabolism¹⁻³ , and the partaking enzymes represent targets for the therapy of cancer, autoimmunity, and neurodegenerative disease⁴. The first mammalian histone deacetylase was discovered in 1996 by means of a chemical biology approach⁵. Based on sequence phylogeny and function, there are four distinct classes: Class I (HDAC1, 2, 3, and 8), class IIa (HDAC4, 5, 7, and 9), class IIb (HDAC6 and 10) and class IV (HDAC11) represent Zn²⁺-dependent amidohydrolases, whereas class III comprises the mechanistically diverse NAD⁺-dependent sirtuins⁶. HDACs form the catalytic core of megadalton complexes involved in chromatin modification and gene repression. Four such molecular machines have been characterized to date: The CoREST, NuRD, and Sin3 complexes contain a HDAC1/HDAC2 dimer as core, whereas the NCoR complex is formed around HDAC3⁷. The roles of these complexes are diverse and often cell-type specific, and whereas more data are emerging regarding their role in the determination of cell fate, their functions in tissue homeostasis are less well understood⁸. The CoREST complex couples histone deacetylation to demethylation to repress neuronal genes^{9,10}, the NuRD complex links deacetylation to a chromatin-remodeling ATPase and promotes gene silencing ^{11,12}, and the Sin3 complex represses genes downstream of various developmental pathways^{13, 14}. The NCoR complex is the major corepressor for nuclear receptors^{15,16}. Class IIa HDACs exhibit low enzymatic activity and were proposed to have mainly "modification reader" or other scaffold functions^{17, 18}. Class IIb HDACs are thought to have mostly non-epigenetic functions in regulating protein folding and turnover¹⁹

Small molecule HDAC inhibitors (HDACi) were discovered by their ability to induce redifferentiation of transformed cells²⁰. SAHA (vorinostat) and romidepsin are approved for the treatment of cutaneous T cell lymphoma, valproate is in clinical use as an anticonvulsant, and several HDACi are in clinical development for a number of indications²¹ . The development of HDACi has been hampered by a lack of target selectivity which increases the risk of toxic liabilities and also limits their use as tool compounds²¹. The perceived lack of selectivity may originate from the optimization of these compounds in industry standard assays using recombinant enzymes or protein fragments, which do not properly reflect the native conformation and activity of the target and its physiological context, due to incorrect protein folding, post-translational modifications, and absence of regulatory subunits. Notably, purified class I HDACs exhibit increased activity in the presence of interacting proteins^{22, 23}. Most HDACi adhere to a distinctive pharmacophore comprising a "cap", which binds to the rim of the substrate channel, a spacer spanning the channel, and a Zn²⁺-chelating function. A photoaffinity analog of SAHA was shown to label not only HDACs but also RCOR1, MBD3 and MTA1/2, indicating that these proteins are in proximity of the active site, and purporting that the "cap" conveys inhibitor selectivity²⁴.

Recent advances in quantitative chemoproteomics have enabled binding studies of small molecule enzyme inhibitors to endogenous proteins in cells and tissues²⁵⁻²⁷ Here we extend this approach towards the monitoring of clinical and tool HDACi binding to native megadalton protein complexes, and towards the discovery of novel targets and inhibitors. We find that HDACi target known and novel protein complexes which are precisely defined by matching IC₅₀ values for a given inhibitor for all complex subunits, and confirm complex composition by quantitative immunoaffinity purifications. Notably, inhibitor selectivity data for native drug target complexes deviate from literature values obtained with recombinant enzymes in isolation, indicating in some cases an unexpected degree of selectivity.

### Results

### Synthesis of a target class specific HDAC probe matrix

Target-class directed chemical probes provide tools for the identification of drug targets directly in cells and tissues. Suitable probes consist of a moiety which binds to a ligand pocket conserved within the target class under investigation, and a functional group for immobilization, thus enabling efficient enrichment of bound proteins for analysis^{28, 29}. HDACs share a conserved substrate pocket, and most hydroxamate inhibitors are nonselective³⁰. We synthesized a target-class specific probe matrix by derivatizing sepharose with analogues of the hydroxamates SAHA and ITF2357 (givinostat). The probe matrix was exposed to cell extracts under close to physiological conditions and aliquots of the sample were treated with excess inhibitor which competes with the immobilized probes for target protein binding. The difference in captured proteins between vehicle-treated and inhibitor-treated samples was quantified by isobaric tagging (iTRAQ or TMT) of tryptic peptides and tandem mass spectrometry analysis (MS/MS) of the combined peptide pools³¹. For each identified protein the decrease of reporter signal responses relative to the vehicle control reflects the competition by the 'free' inhibitor drug for its target. The results comprise binding data for both direct enzyme targets as well as proteins residing in a complex with the target, since these are predicted to exhibit matching IC₅₀ profiles across a set of drugs. Associations of proteins in complexes were confirmed by immunoaffinity purifications (Fig. 31).

### Quantitative mapping of probe matrix interactomes

Initially we tested the binding of the probe matrix to recombinant HDACs 1-11 purified from Sf9 cells. The proteins were found to bind to the matrix, but the amount captured was only affected at a high excess of free SAHA, indicating that the bulk of the purified enzyme exhibit low activity. The activity of the enzymes in peptide deacetylation assays may be due to a small fraction of properly folded protein, or due to contamination with co-purifying insect cell activities.

More substantial results were obtained when the SAHA- or ITF2357-derivatized matrix was used to probe endogenous HDACs in K562 whole cell extracts. From the approx. 2900 proteins captured and quantified by MS/MS, about 300 exhibited competition by a large excess of inhibitor and thus were considered specific, whereas the remainder was considered background. All class I/class IIb HDACs and many known HDAC complex subunits were captured in specific fashion, as binding was competed by excess inhibitor. In addition many other proteins were specifically captured, implicating them either as novel inhibitor targets or as co-purifying proteins in target complexes. Compared to the SAHA matrix, the ITF2357 matrix exhibited higher background but specifically retained a few additional proteins. Since both types of matrix captured the same set of HDACs, other proteins found primarily with one type of matrix represent drug-specific off-targets or interactors thereof. Examples are 5,10-methenyltetrahydrofolate synthetase (MTHFS) and hydroxysteroid (17-beta) dehydrogenase 4 (HSD17B4), which emerge as prominent givinostat but not SAHA off-targets. We defined the specific set of captured proteins as the probe matrix interactome. Taking the results from several experiments together, the matrix interactome comprises approx. 500 proteins including the six class I and class IIb HDACs, and 29 proteins known to associate with class I HDACs in the CoREST, NuRD, Sin3, and NCoR complexes. The remaining proteins without a previous connotation to deacetylases are likely to represent either off-targets or proteins interacting with HDACs or with off-targets. Next, the probe matrix interactome was differentially mapped in nuclear versus cytosolic fractions of Jurkat cells, and in whole cell extracts from six human cell lines and six mouse tissues. Most proteins in the interactome appeared to be ubiquitously expressed, and as expected the majority of proteins are enriched in the nuclear fraction as compared to whole cell extract. Because of the function of class I HDACs in cell division³², we also conducted differential mapping of the interactome in HeLa cells arrested in mitosis (by nocodazole treatment) compared to G1/S phase (by aphidicolin treatment) and untreated cells. Notably three proteins were captured in increased amounts from mitotic cells and may constitute a novel HDAC complex; DNTTIP 1 (deoxynucleotidyltransferase interacting protein), C14ORF43, a protein of unknown function with homology to the REST corepressor which we dubbed MIDEAS (for mitotic deacetylase-associated ELM and SANT domain), and the histone acetyltransferase CDYL. Remarkably, the expression level of DNTTIP1 is not increased in mitotic cells, and hence the data indicate the formation of a dedicated mitotic complex.

### Proteomic target profiling of clinical and tool compounds

The probe matrix offers an efficient tool to probe a subproteome of putative targets with drugs under close to physiological conditions. We selected a set of 16 structurally diverse HDACi including approved drugs, clinical development compounds, and tool compounds. Aliquots of K562 cell extract were spiked with vehicle or different inhibitors in 5 concentrations typically ranging from 40 nM to 10 µM. For low or very high potency compounds, concentrations were adjusted. Subsequently, samples were incubated with the probe matrix, captured proteins were quantitatively mapped by MS/MS, and a set of IC₅₀ values was determined for each inhibitor (Fig. 32a). To assess the reproducibility of our methodology, two to seven replicate experiments were performed per inhibitor profile using targeted data acquisition³³ for a defined subset of proteins. The data were sufficiently reproducible to distinguish fairly small (two-fold) IC₅₀ differences and we found small albeit statistically significant potency differences between distinct HDAC1/2 complexes, e.g. NuRD and CoREST (Fig. 32b). To determine the impact of target protein depletion by the probe matrix on the IC₅₀ values, an aliquot of the cell extract was subjected to two sequential incubations with the matrix. This procedure allows the calculation of apparent dissociation constants (K_{d}^{app})²⁷.However the resulting deviation between K_{d}^{app} and IC₅₀ values was less than twofold for 99% of the proteins.

The dataset comprises concentration-inhibition profiles for 16 compounds versus a total of 344 proteins, including the class I and IIb HDACs, components of HDAC complexes, and putative novel complex components or targets (Table 13). Proteins associated in a complex exhibit matching K_{d}^{app} values for a given inhibitor, implying that these proteins remain associated with the molecular target during the assay procedure. Thus, the inhibition profiles across the compound set can be used to trace protein complexes. It should be noted that proteins known to reside in two or more complexes (e.g. HDAC1, HDAC2, RBBP4, RBBP7 and LSD1^{7, 34}) are likely to exhibit K_{d}^{app} values representing aggregates between complexes. While the HDAC1/2-containing CoREST and NuRD complexes showed a similar inhibition profile across the compound set, there were marked differences in the Sin3A inhibition profile, with all benzamides showing a pronounced drop in potency for this complex compared to CoREST and NuRD. The profiles accurately delineate a novel mitotic deacetylase complex (termed MiDAC) formed by HDAC1/2, DNTTIP1, CDYL and MIDEAS (Fig. 32c). Bidirectional hierarchical clustering of the complete dataset clearly outlined the major complexes including MiDAC (Fig. 32d). In the chemical dimension the clustering is driven by the major chemotypes with several hydroxamate sub-clusters which differed in their effect on Class II HDACs. In agreement with published data we found that the peptidic and hydroxamate compounds are substantially more potent than the aminobenzamides (Table 13). A notable observation is the unexpected degree of selectivity of benzamide class inhibitors which show a preference for the HDAC3-NCoR complex. It is interesting to compare the target profiles of SAHA and its analog BML-210, which are identical except an amidobenzamide group in BML-210 replacing the hydroxamate function, causing a general drop of potency for class I HDACs (and complete loss of potency for class IIb) concomitant with an increase in selectivity for HDAC3 over HDAC1/HDAC2 (Fig. 32a).

### Deconvolution of protein complexes by quantitative IP-MS/MS

In order to differentiate between novel direct targets and novel components of HDAC complexes, a set of quantitative co-immunoaffinity purifications (co-IP) with quantitative MS/MS analysis was performed. We evaluated a set 21 antibodies and ultimately selected nine, directed against three class I HDACs, four known complex subunits (LSD1 from CoREST, MTA3 from NuRD, SIN3A from Sin3, TBL1XR1 from NCoR), and two novel complex components (TRERF1, DNTTIP1) (Fig. 33a). After isobaric tagging, IP samples from two different antibodies each were combined with matched control IgG samples for MS/MS analysis such that specifically co-immunopurified proteins were clearly discriminated from background (Fig. 33b). Most known complex components and several of the new targets or components were identified in the IP samples. However, in some cases the IP samples contained hundreds of enriched proteins interfering with the identification of true interacting proteins. Data complexity is drastically reduced when orthogonal chemoproteomics and co-IP data are combined, thus enabling efficient and unbiased deconvolution of HDACi target complexes (Fig. 33c). The MiDAC complex was confirmed in the DNTTIP1 IP sample which comprised MIDEAS, HDAC1, and HDAC2 but no known HDAC complex subunits. TRERF1, an ELSA (ELM-SANT) domain protein related to MIDEAS, was co-purified with HDAC2 and with DNTTIP1. Immunoaffinity purification of TRERF1 itself indeed confirmed its association with DNTTIP1 and HDAC1/2, but not with MIDEAS, suggesting that TRERF1 represents an alternative scaffold for a similar complex. Other ELSA proteins were also found to co-purify with HDAC1 or HDAC2 including MIER1, 2, and 3, which were identified with HDAC2, and RERE, which was identified with HDAC1 and HDAC2. Since the inhibition profiles of the MIER and RERE proteins did not align with any of the other complexes (Fig. 32c), these proteins likely represent components of distinct HDAC complexes formed around ELSA scaffolds³⁵.

### Cell-based profiling of clinical and tool compounds

To investigate the correlation of proteomic target profiles with substrate selectivity, a subset of the reference inhibitors was subjected to cell-based tubulin and histone modification assays. K562 and HeLa cells were treated with vehicle or compounds including nonselective inhibitors (SAHA, PCI-24781), class I selective inhibitors (tacedinaline, romidepsin, valproate) and the HDAC8 inhibitor PCI-34051. Cell viability was monitored and drug effects were detected by antibodies for acetylated tubulin as the major substrate of HDAC6¹⁹ and histones H3 and H4 as the major class I HDAC substrates by immunofluorescence and western blotting (Fig 34a). The nonselective HDACi increased steady state acetylation of tubulin and histones manifest by the staining of acetylated microtubules as well as punctuate nuclear staining of acetylated histones. The class I selective HDACi elicited staining of acetylated histones but did not affect tubulin as expected. Aliquots of vehicle-treated and drug-treated cells were also compared by differential mapping of histone acetylation and methylation marks using isobaric tagging and MS/MS (Fig. 34b). The results confirmed the range of activities observed in chemoproteomics profiling and indicate a pronounced abundance of hyperacetylated histone peptides after treatment with nonselective HDACi, in particular TSA and romidepsin. In contrast, valproate exhibited a more selective effect. For instance, the acetylation of histone H3K14 in the presence of methylated K9 was increased to a similar degree as with other nonselective HDACi, but little effect was observed on the acetylation of K9 in peptides containing acetylated K 14.

### Bufexamac as a novel Class IIb HDAC inhibitor

To enable the discovery of novel selective HDACi we developed a high throughput variation of the chemoproteomics protocol in which MS/MS detection is replaced with multiplexed fluorescent antibody detection on "dot blot" type arrays. The method was applied to the screening of a focused compound library in whole cell extract of Jurkat and Ramos cells for inhibitors of HDAC1, 2, 3 and 6. Several hits were obtained with a few compounds displaying a notable degree of selectivity (Fig. 35a). Two benzamides (o-aminoanilides) were identified as hit compounds exhibiting selectivity for HDAC3. Bufexamac, a non-steroidal anti-inflammatory drug with an unknown mechanism of action³⁶ was found to preferentially affect HDAC6. Bufexamac was subjected to quantitative proteomic profiling as described above for the reference HDACi set, which confirmed its selectivity for HDAC6 and HDAC10, the other class IIb isoform, in addition to several non-HDAC targets (Fig. 35b). The results are consistent with tubulin immunofluorescence and western blot data which showed a strong increase in acetylated tubulin, the major HDAC6 substrate, but not in acetylated histones as substrates of Class I HDACs (Fig. 35c, see also data in Fig. 34b). The cellular potency for tubulin deacetylation correlated with the potency for one of the drugs' anti-inflammatory effects, the secretion of IFNα in peripheral blood mononuclear cells (Fig. 35d).

### Discussion

Gene transcription and its epigenetic regulation are controlled by megadalton protein complexes^{37, 38}. Hence the action of drugs which modulate epigenetic mechanisms should be considered in the context of the multiprotein complexes which constitute their targets. We developed an affinity capture method combined with highly sensitive multiplexed protein quantification by mass spectrometry to probe the interaction of drug molecules with drug targets in cells or tissue under conditions which preserve the integrity of protein complexes. The strategy, applied here to HDAC inhibitors, has the potential to be extended to other pharmacological target classes, and enables the discovery of drug leads, their molecular targets, and functional target protein complexes. The biological activity of compounds is assessed in a functional molecular context, without use of recombinant purified proteins or protein overexpression. The major prerequisite is a probe matrix which binds to a sub-proteome or interactome which is characterized by a shared chemical ligand space, typically based on a substrate- or cofactor-binding site. We developed a probe matrix which captures HDACs and related proteins by binding to the substrate pocket. The matrix interactome comprises the class I and class IIb HDACs, and the majority of previously reported subunits of HDAC complexes. Class IIa HDACs were not identified, presumably because they exhibit low catalytic activity and low affinity for the hydroxamate probes¹⁷. Moreover, the interactome contains many other enzymes which potentially represent targets sharing a similar chemical ligand space, including other metalloenzymes, as well as other proteins which may be associated with enzymes in protein complexes.

One basic application of a probe matrix is the differential expression profiling of a subproteome - its interactome - across a range of biological samples and conditions. In line with a pleiotropic function most proteins in the hydroxamate matrix interactome displayed minor differences across a panel of cell lines and tissues. However two proteins lacking an obvious small molecule binding site were captured predominantly from mitotically arrested cells, in line with the specific formation of a mitotic HDAC complex comprising novel subunits, a finding subsequently confirmed and extended by inhibitor profiling and co-IP data.

A powerful application of the probe matrix is the profiling of the interactions of drugs and lead molecules with the interactome in cell extract. We demonstrated that robust quantitative data are obtained by high sensitivity LC/LC-MS/MS to measure protein binding to the matrix in whole cell extract as a function of the concentration of competing "free" compounds. Bidirectional hierarchical clustering of the proteomic target profiles of 16 inhibitors (Fig. 32d) guides (i) the classification of drugs in selectivity clusters, which are predicted to exhibit similar pharmacological effects, (ii) the grouping of protein targets in chemical space, and (iii) the assignment of targets to protein complexes. In order to distinguish individual targets which share a structurally similar ligand binding space from proteins associated in a complex, the chemoproteomics clustering may be deconvoluted using literature data, or bioinformatics predictions of ligand binding sites³⁹. However the highest quality orthogonal data are acquired from co-IP experiments which delineate protein complexes in the same biological context, while avoiding overexpression artifacts and the impact of different cell types⁴⁰.

Our chemoproteomics dataset clusters 16 HDACi versus 344 proteins specifically interacting with the probe matrix. The clustering of inhibitors is driven by the major chemotypes represented by hydroxamates and aminobenzamides, with several hydroxamate sub-clusters, and reveals an unexpected degree of selectivity for inhibitors previously perceived as nonselective⁴¹⁻⁴³. However much of the published data is inconsistent raising issues with the enzyme assays employed. Notably, a recent carefully controlled extensive enzyme kinetic study of drugs and tool compounds also reported a higher degree of inhibitor selectivity¹⁷. We found that all compounds in the benzamide cluster showed a preference for the HDAC3-NCoR complex and that within the class I HDAC complexes they did not affect Sin3. No inhibitor outside this class displayed this profile, and this feature of the benzamide class may contribute to a more favorable toxicology profile. Remarkably, the clinically relevant drug valproate did also affect the Sin3 complex to a substantially lesser degree than NuRD and CoREST complexes.

A number of non-HDAC targets are potently affected by several hydroxamate HDACi but do not appear to be components of HDAC complexes, given that their inhibition profiles do not match any HDAC (Fig. 32c), and because they were not enriched in the co-IP set (Fig. 33a). These proteins may represent off-targets sharing a similar chemical ligand space. Examples are the basic leucine zipper/W2 domain protein BZW2 and the isochorismatase domain protein ISOC2, and several other Zn²⁺-dependent metalloenzymes including aldehyde dehydrogenases.

In the protein dimension, the clustering data delineate target protein complexes, since proteins exhibiting matching inhibition profiles across the inhibitor panel are likely to be physically associated. This is evident by the almost perfect clustering of the four major HDAC-containing complexes (Fig. 32). To our knowledge this is the first time that small molecule binding data are used to characterize target protein complexes, and hence we extended our data by an extensive co-IP analysis of endogenous HDAC complexes from the same cell extract. A few previously reported class I HDAC complex components which had been absent from the probe matrix interactome were identified in the co-IP samples and may represent interactions that are sensitive to inhibitor binding, e.g. the ING2 subunit of the Sin3 complex⁴⁴. The co-IP results confirmed the existence of the additional HDAC1/HDAC2 complexes delineated in the analysis of the chemoproteomic data. These complexes are built around ELSA domain proteins which are phylogenetically related to corepressor components of NuRD and CoREST complexes³⁵. Our data indicate that each complex contains only one type of ELSA scaffold, and that several of such complexes exist, formed around MIER1/2/3, RERE, TRERF1, and MIDEAS, a previously unannotated gene product. One function of HDAC complexes is likely the coordination of deacetylation with other epigenetic modifications. The CoREST complex couples HDACs to the demethylase LSD1¹⁰, and the ELSA proteins MIER1 and RERE were shown to scaffold HDACs with the EHMT methyltransferases³⁵, and our inhibition profiles confirmed these complexes as HDACi targets. The composition of these HDAC complexes was deconvoluted further by the co-IP data, in particular the MiDAC complex formed in mitotic cells by HDAC1/2, MIDEAS and DNTTIP1. DNTTIP1 is a DNA binding protein that has been described to modulate the activity of terminal deoxynucleotidyl transferase (TDT), a specialized DNA polymerase that incorporates nontemplated nucleotides to the 3' end of DNA templates to mediate the junctional diversity of immunoglobulin genes⁴⁵. However, we did not consistently identify an association of TDT with the MiDAC complex suggesting a TDT independent function of the complex in cell division. The inhibition profiles also implicated the REST corepressor CDYL⁴⁶ as a component of MiDAC, in line with the increase in CDYL captured from mitotic cells by the SAHA matrix. However, CDYL co-immunopurified with HDAC2 but not with the MiDAC subunit DNTTIP1, and hence further analysis is required to clarify whether it is a component of MiDAC or of an alternative complex.

The fourth class I enzyme, HDAC8, is more difficult to assign to a complex, because it is only targeted by a few inhibitors, and we did not identify a suitable antibody to characterize it by co-IP. Notably, the HDAC8 inhibitor PCI-34051⁴⁷ is the only compound in our panel which was specific for a single HDAC, and its target profile comprised other proteins which might represent components of a complex. The class IIb enzymes HDAC6 and HDAC10 were only inhibited by hydroxamate type compounds and both do not appear to form robust complexes as no strong associations with other proteins in the inhibition profiles were detected. HDAC6 has recently been implicated in chromatin regulation⁴⁸ but the HDAC6/HDAC 10 inhibitor bufexamac did not affect the acetylation of histones, which does not support a direct role of class IIb deacetylases in histone modification.

Our chemoproteomics technology can be adapted to high throughput screening by using an antibody-based readout instead of MS/MS to reduce sample requirements and process time. We conducted a screen of a focused compound library for selective inhibitors. The HDAC3 selective hit compounds were benzamides, confirming the selectivity preferences of this pharmacophore class. The screen also identified the hydroxamate bufexamac, an NSAID with an unknown mechanism of action³⁶ as a class IIb selective inhibitor. The drug induced tubulin hyperacetylation in drug concentrations matching its anti-inflammatory effect. Therefore, HDAC6 inhibition may contribute to the drug's clinical efficacy.

In conclusion, we have shown for the first time that a chemoproteomics strategy based on small molecule inhibitors can be applied to discover and classify molecular complexes around drug target proteins. The approach confirms and extends orthogonal protein-protein interaction mapping, e.g. based on co-IP methodology. We have demonstrated the utility of this strategy in drug discovery by defining distinctive target profiles for clinical HDAC inhibitors in cell extracts, and employed it in screening for novel small molecule inhibitors. The data support the value of drug discovery strategies based on target proteins in their biological context.

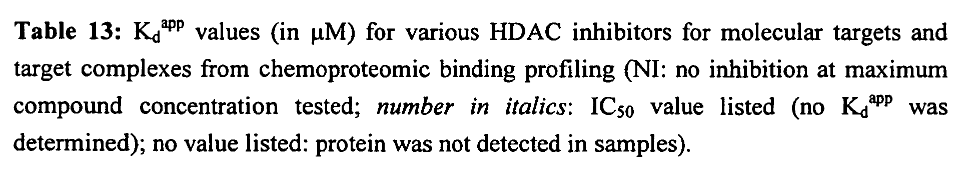

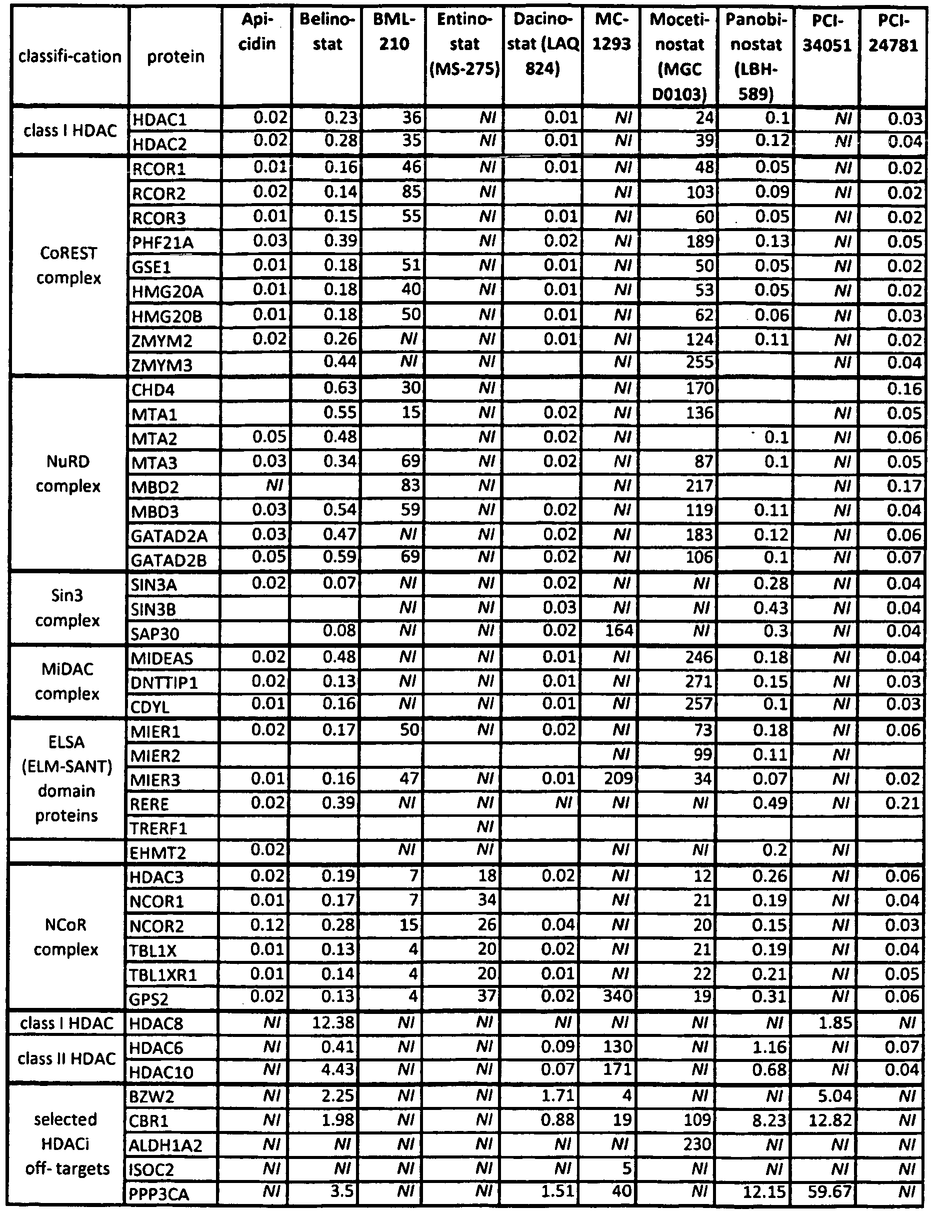

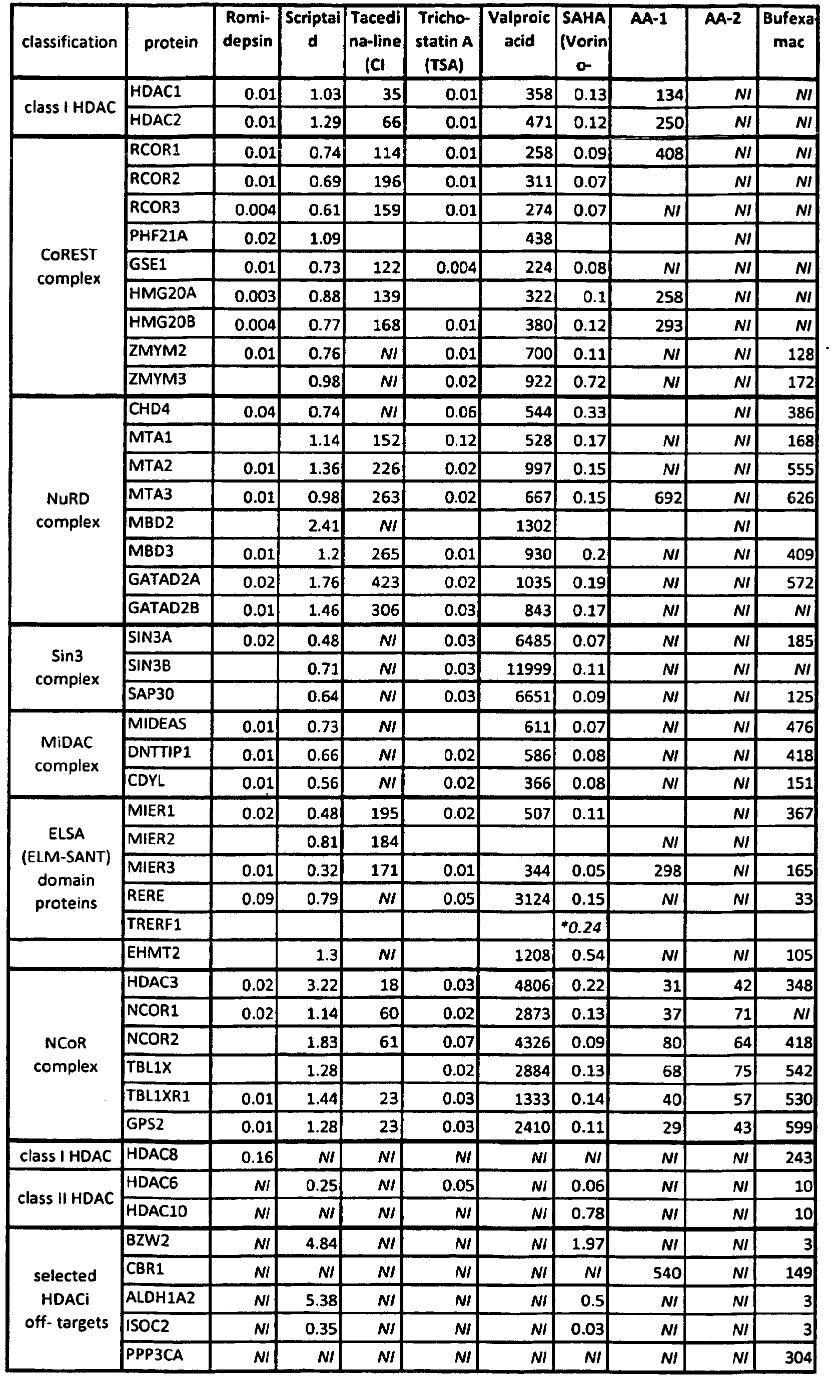

### References of Example 9

1. Kouzarides,T. Chromatin modifications and their function. Cell 128, 693-705 (2007).
2. Choudhary,C. et al. Lysine acetylation targets protein complexes and co-regulates major cellular functions. Science 325, 834-840 (2009).
3. Zhao,S. et al. Regulation of cellular metabolism by protein lysine acetylation. Science 327, 1000-1004 (2010).
4. Karberg,S. Switching on epigenetic therapy. Cell 139, 1029-1031 (2009).
5. Taunton,J., Hassig,C.A., & Schreiber,S.L. A mammalian histone deacetylase related to the yeast transcriptional regulator Rpd3p. Science 272, 408-411 (1996).
6. Gregoretti,I.V., Lee,Y.M., & Goodson,H.V. Molecular evolution of the histone deacetylase family: functional implications of phylogenetic analysis. J. Mol. Biol. 338, 17-31 (2004).
7. Yang,X.J. & Seto,E. The Rpd3/Hda1 family of lysine deacetylases: from bacteria and yeast to mice and men. Nat. Rev. Mol. Cell Biol. 9, 206-218 (2008).
8. Cunliffe,V.T. Eloquent silence: developmental functions of Class I histone deacetylases. Curr. Opin. Genet. Dev. 18, 404-410 (2008).
9. Lakowski,B., Roelens,I., & Jacob,S. CoREST-like complexes regulate chromatin modification and neuronal gene expression. J Mol. Neurosci. 29, 227-239 (2006).
10. You,A., Tong,J.K., Grozinger,C.M., & Schreiber,S.L. CoREST is an integral component of the CoREST- human histone deacetylase complex. Proc. Natl. Acad. Sci. U S. A 98, 1454-1458 (2001).
11. Tong,J.K., Hassig,C.A., Schnitzler,G.R., Kingston,R.E., & Schreiber,S.L. Chromatin deacetylation by an ATP-dependent nucleosome remodelling complex. Nature 395, 917-921 (1998).
12. Zhang,Y., LeRoy,G., Seelig,H.P., Lane,W.S., & Reinberg,D. The dermatomyositis-specific autoantigen Mi2 is a component of a complex containing histone deacetylase and nucleosome remodeling activities. Cell 95, 279-289 (1998).
13. Grzenda,A., Lomberk,G., Zhang,J.S., & Urrutia,R. Sin3: master scaffold and transcriptional corepressor. Biochim. Biophys. Acta 1789, 443-450 (2009).
14. Zhang,Y., Iratni,R., Erdjument-Bromage,H., Tempst,P., & Reinberg,D. Histone deacetylases and SAP18, a novel polypeptide, are components of a human Sin3 complex. Cell 89, 357-364 (1997).
15. Guenther,M.G. et al. A core SMRT corepressor complex containing HDAC3 and TBL1, a WD40-repeat protein linked to deafness. Genes Dev. 14, 1048-1057 (2000).
16. Karagianni,P. & Wong,J. HDAC3: taking the SMRT-N-CoRrect road to repression. Oncogene 26, 5439-5449 (2007).
17. Bradner,J.E. et al. Chemical phylogenetics of histone deacetylases. Nat. Chem. Biol. 6, 238-243 (2010).
18. Lahm,A. et al. Unraveling the hidden catalytic activity of vertebrate class IIa histone deacetylases. Proc. Natl. Acad. Sci. U. S. A 104, 17335-17340 (2007).
19. Boyault,C., Sadoul,K., Pabion,M., & Khochbin,S. HDAC6, at the crossroads between cytoskeleton and cell signaling by acetylation and ubiquitination. Oncogene 26, 5468-5476 (2007).
20. Marks,P.A. & Breslow,R. Dimethyl sulfoxide to vorinostat: development of this histone deacetylase inhibitor as an anticancer drug. Nat. Biotechnol. 25, 84-90 (2007).
21. Bolden,J.E., Peart,M.J., & Johnstone,R.W. Anticancer activities of histone deacetylase inhibitors. Nat. Rev. Drug Discov. 5, 769-784 (2006).
22. Zhang,Y. et al. Analysis of the NuRD subunits reveals a histone deacetylase core complex and a connection with DNA methylation. Genes Dev. 13, 1924-1935 (1999).
23. Guenther,M.G., Barak,O., & Lazar,M.A. The SMRT and N-CoR corepressors are activating cofactors for histone deacetylase 3. Mol. Cell Biol. 21, 6091-6101 (2001).
24. Salisbury,C.M. & Cravatt,B.F. Activity-based probes for proteomic profiling of histone deacetylase complexes. Proc. Natl. Acad. Sci. U. S. A 104, 1171-1176 (2007).
25. Bantscheff,M. et al. Quantitative chemical proteomics reveals mechanisms of action of clinical ABL kinase inhibitors. Nat. Biotechnol. 25, 1035-1044 (2007).
26. Ong,S.E. et al. Identifying the proteins to which small-molecule probes and drugs bind in cells. Proc. Natl. Acad. Sci. U. S. A 106, 4617-4622 (2009).
27. Sharma,K. et al. Proteomics strategy for quantitative protein interaction profiling in cell extracts. Nat. Methods 6, 741-744 (2009).
28. Cravatt,B.F., Wright,A.T., & Kozarich,J.W. Activity-based protein profiling: from enzyme chemistry to proteomic chemistry. Annu. Rev. Biochem. 77, 383-414 (2008).
29. Bantscheff,M., Scholten,A., & Heck,A.J. Revealing promiscuous drug-target interactions by chemical proteomics. Drug Discov. Today 14, 1021-1029 (2009).
30. Marks,P.A. & Dokmanovic,M. Histone deacetylase inhibitors: discovery and development as anticancer agents. Expert. Opin. Investig. Drugs 14, 1497-1511 (2005).
31. Bantscheff,M. et al. Robust and sensitive iTRAQ quantification on an LTQ Orbitrap mass spectrometer. Mol. Cell Proteomics. 7, 1702-1713 (2008).
32. Kruhlak,M.J. et al. Regulation of global acetylation in mitosis through loss of histone acetyltransferases and deacetylases from chromatin. J Biol. Chem. 276, 38307-38319(2001).
33. Savitski,M.M. et al. Targeted Data Acquisition for Improved Reproducibility and Robustness of Proteomic Mass Spectrometry Assays. J. Am. Soc. Mass Spectrom.(2010).
34. Wang,Y. et al. LSD1 is a subunit of the NuRD complex and targets the metastasis programs in breast cancer. Cell 138, 660-672 (2009).
35. Wang,L., Charroux,B., Kerridge,S., & Tsai,C.C. Atrophin recruits HDAC1/2 and G9a to modify histone H3K9 and to determine cell fates. EMBO Rep. 9, 555-562 (2008).
36. Trommer,H. et al. Examinations of the antioxidative properties of the topically administered drug bufexamac reveal new insights into its mechanism of action. J. Pharm. Pharmacol. 55, 1379-1388 (2003).
37. Alberts,B. The cell as a collection of protein machines: preparing the next generation of molecular biologists. Cell 92, 291-294 (1998).
38. Gavin,A.C. et al. Proteome survey reveals modularity of the yeast cell machinery. Nature 440, 631-636 (2006).
39. Scheiber,J. et al. Gaining insight into off-target mediated effects of drug candidates with a comprehensive systems chemical biology analysis. J. Chem. Inf. Model. 49, 308-317 (2009).
40. Malovannaya,A. et al. Streamlined analysis schema for high-throughput identification of endogenous protein complexes. Proc. Natl. Acad. Sci. U. S. A 107, 2431-2436 (2010).
41. Beckers,T. et al. Distinct pharmacological properties of second generation HDAC inhibitors with the benzamide or hydroxamate head group. Int. J. Cancer 121, 1138-1148 (2007).
42. Blackwell,L., Norris,J., Suto,C.M., & Janzen,W.P. The use of diversity profiling to characterize chemical modulators of the histone deacetylases. Life Sci. 82, 1050-1058 (2008).
43. Khan,N. et al. Determination of the class and isoform selectivity of small-molecule histone deacetylase inhibitors. Biochem. J. 409, 581-589 (2008).
44. Smith,K.T., Martin-Brown,S.A., Florens,L., Washburn,M.P., & Workman,J.L. Deacetylase inhibitors dissociate the histone-targeting ING2 subunit from the Sin3 complex. Chem. Biol. 17, 65-74 (2010).
45. Kubota,T., Maezawa,S., Koiwai,K., Hayano,T., & Koiwai,O. Identification of functional domains in TdIF1 and its inhibitory mechanism for TdT activity. Genes Cells 12, 941-959 (2007).
46. Mulligan,P. et al. CDYL bridges REST and histone methyltransferases for gene repression and suppression of cellular transformation. Mol. Cell 32, 718-726 (2008).
47. Balasubramanian,S. et al. A novel histone deacetylase 8 (HDAC8)-specific inhibitor PCI-34051 induces apoptosis in T-cell lymphomas. Leukemia 22, 1026-1034 (2008).
48. Wang,Z. et al. Genome-wide mapping of HATs and HDACs reveals distinct functions in active and inactive genes. Cell 138, 1019-1031 (2009).

### Methods

### 1. Reagents.

All reagents were purchased from Sigma unless otherwise noted below. Antibodies were purchased from the following suppliers: sc-7872 (HDAC1), sc-81599 (HDAC2), sc-17795 (HDAC3), sc-11405 (HDAC8), sc-81325 (MTA3), sc-100908 (TBL1XR1), sc-81082 and sc-166296 (DNTTIP1) and sc47778 (□-actin) from Santa Cruz; 05-814 (HDAC2), 05-813 (HDAC3), 07-505 (HDAC8) from Millipore; ab46985 (HDAC1), ab3479 (Sin3A), ab70039 (DNTTIP1); NB100-81655 (TRERF1) and NB100-81654 (TRERF1) from Novus Biologicals; and ab61236 (H4-AcK5) from Abcam; H-3034 (HDAC-3) and T-6793 (Actubulin) from Sigma; H00010013-M01 (HDAC6) from Abnova; #2184 (LSD1) from Cell Signaling Technologies; and 382158 (H3-AcK9/K14) from Calbiochem. Secondary antibodies labelled with IRDye^{®}680 and IRDye^{®}800 were from LICOR, and antibodies labelled with Alexa^{®}480 and Alexa^{®}594 were from Invitrogen. Reference compounds were purchased from the following suppliers: vorinostat (SAHA), belinostat (PXD-101), dacinostat (LAQ-824), panobinostat (LBH-589), PCI-24781, and entinostat (MS-275) from Selleck; trichostatin A, PCI-34051, bufexamac and apicidin from Sigma; scriptaid and tacedinaline (CI-994) from Tocris; MC-1293 and BML-210 from Enzo Life Sciences; MGCD-0103 from Chemietek; romidepsin (FK-228) from ACC Corp; valproic acid from Calbiochem, and SRT1720 from Cayman.

### 2. Cell culture and cell-based assays.

Jurkat E6.1, HL60, Ramos and HeLa cells were purchased from ATCC; K562 cells were purchased from DSMZ (Braunschweig, Germany). Jurkat E6.1 cells were cultured in RPMI1640 supplemented with 4.5 g/L glucose, 10mM Hepes, 1mM sodium pyruvate and 10% Fetal Calf Serum (FCS). Ramos cells were cultured in RPMI1640 containing 10% FCS. K562 cells were cultured in RPMI medium containing 10% FCS. Cells were expanded to maximal 1x10⁶ cells/ml. HeLa cells were cultured in Minimum Essential Media (MEM) supplemented with 1 mM pyruvate, 0.1 mM nonessential amino acids and 10% FCS. For indirect immunofluorescence experiments, the FCS content was reduced to 2%. For cell cycle arrest of HeLa cells in G1/S-phase or mitosis cells were treated for 16 hours with 15 µg/ml aphidicolin (Sigma) or with 0.3 µM nocodazole (Sigma). Control HeLa cells were treated with DMSO for 16 hours. For cell-based protein acetylation assays, HeLa or K562 cells (96-well format, 5x10⁴ cells per well) were treated with compounds for 6 hours. Cells were washed with cold PBS and lysed directly in SDS-sample buffer, followed by denaturation at 95°C. Lysates (10 µl) were resolved on SDS-gels, transferred to PVDF membranes and analyzed for tubulin and histone acetylation by immune-detection using IRDye^{®}-labelled secondary antibodies and a LiCOR Odyssey scanner. Data analysis of the quantified bands was performed using Excel and GraphPad Prism. For the IFNα secretion assay, human peripheral blood mononuclear cells were isolated with Histopaque 1077 (Sigma) from fresh human donor blood and were plated at a concentration of 0.5×10⁶ cells/ml. Cells were incubated with test compounds for 45 minutes prior to stimulation with plasmacytoid dendritic cell-specific TLR9 agonist ODN2216 (Invivogen). Interferon (IFN)-α released into cell supernatants was measured in triplicates after 16 hours by Flex-Set IFNalpha (BD Biosciences) by flow cytometry (FACSCalibur, BD Biosciences). For all cell-based assays viability was assessed in parallel (MTT kit, Roche).

### 3. Indirect immunofluorescence analysis.

HeLa cells were plated to sub-confluency on polylysine-coated glass chamber slides and treated after recovery with the indicated compounds for 4 hours. Samples were fixed in cold methanol, permeabilized with Triton-X100, blocked in 1% BSA and treated for immuno-detection of acetylated tubulin and acetylated histone H3. Cells were counterstained for nucleic acids using 4',6-diamidino-2-phenylindole (*DAPI*). Multichannel fluorescence microscopy was performed on an Olympus IX-70 microscope. Images were acquired using a monochrome CCD camera (CoolSNAP HQ Digital) and analyzed with MetaMorph (Universal Imaging Corporation). The instrument was adjusted to ensure proper comparison of levels of acetylated tubulin and acetylated histone H3 before and after inhibitor treatment.

### 4. Preparation of cell lysates.

Frozen cell pellets were homogenized in lysis buffer (50 mM Tris-HCl, 0.8% Igepal-CA630, 5% glycerol, 150 mM NaCl, 1.5 mM MgCl2, 25 mM NaF, 1 mM sodium vanadate, 1 mM DTT, pH 7.5). One complete EDTA-free protease inhibitor tablet (Roche) per 25 ml was added. The sample was dispersed using a Dounce homogenizer, kept rotating for 30 minutes at 4° C and spun for 10 minutes at 20,000 x g at 4°C. The supernatant was spun again for 1 hour at 145,000 x g. The protein concentration was determined by Bradford assay (BioRad), and aliquots were snap frozen in liquid nitrogen and stored at -80°C.

### 5. Compound profiling.

Affinity profiling assays were performed as described previously^{1, 2} with minor modifications. Derivatized sepharose beads (35 µl beads per sample) were equilibrated in lysis buffer and incubated with 1 ml (5 mg protein) cell lysate, which had been preincubated with test compound or vehicle, on an end-over-end shaker for 1 hour at 4°C. Beads were transferred to disposable columns (MoBiTec), washed with lysis buffer containing 0.2 % detergent and eluted with 50 µl 2x SDS sample buffer. Proteins were alkylated with 200 mg/ml iodoacetamide for 30 minutes, separated on 4-12% NuPAGE (Invitrogen), and stained with colloidal Coomassie.

### 6. Compound screening.

The compounds for screening was selected either for their potential to be Zinc chelators or based on a similarity search of the compound collections from Asinex Corp. (Winston-Salem, NC, USA) and Enamine (Mammouth_Jct., NJ, USA), using a training set of 140 known HDAC inhibitors. Competition binding assays using SAHA-beads were performed essentially as described above but adapted to a 96-well format. 1mg of cell lysate and 5 µl of beads were used per well. Compounds from the screening library including reference compounds as standards were added at 20 µM and 100 µM final concentration from 50x DMSO stocks. Each plate contained 8 positive (TSA 50 µM) and eight negative controls (2% DMSO). Beads were eluted in SDS sample buffer (100 mM Tris pH 7.4, 4% SDS, 20% glycerol, 0.01% bromophenol blue, 50 mM DTT) and spotted in duplicate on nitrocellulose membranes (600 nl/spot) using an automated liquid dispenser (FluidX). After drying, the membranes were rehydrated in 20% ethanol, and processed for detection with specific antibodies as indicated. Spot intensities were quantified using a LiCOR Odyssey scanner and percentage inhibition was calculated using positive and negative controls as 100% and 0% inhibition respectively.

### 7. Quantitative co-immunopurification.

Antibodies were tested for suitability in co-IP assays by standard immunoprecipitation (IP)-western procedures³. Western blotting was performed using a LI-COR Odyssey System. Suitable antibodies (40-100 µg) were coupled to 100 µl AminoLink resin (Thermo Fisher Scientific). Cell lysate samples (10 mg) were incubated with pre-washed immuno resin on a shaker for 2 hours at 4 ° C. Beads were washed in lysis buffer containing 0.4% Igepal-CA630 and lysis buffer without detergent. Bound proteins were eluted in 100 µl 2x SDS sample buffer. Protein samples were reduced, alkylated and separated by SD-PAGE. To provide a specificity control for quantitative LC-MS analysis, IgG from the same species was used in an analogous "mock IP" carried out in parallel from an aliquot of the same lysate sample. Typically, four IP reactions, which were subsequently combined in a single iTRAQ sample for MS/MS analysis, were performed in parallel, two with different antibodies directed against the same (or different) antigen(s) and two "mock IP" samples.

### 8. Mass Spectrometry sample preparation.

Gels were cut into slices across the entire separation range and subjected to in-gel digestion'. Peptide extracts were labeled with iTRAQ™ reagents (Applied Biosystems) or TMT™ (Thermo-Fisher Scientific) in 40 mM triethylammoniumbicarbonate (TEAB), pH 8.53. After quenching of the reaction with glycin labeled extracts were combined. For compound profiling experiments extracts from vehicle treated samples were labeled with TMT reagent 131, and combined with extracts from compound-treated samples labeled with TMT reagents 126-131, fractionated using reversed phase chromatography at pH 12, dried and acidified prior to LC-MS/MS analysis.

### 9. LC-MS/MS analysis.

Samples were dried *in vacuo* and re-suspended in 0.1 % formic acid in water and aliquots of the sample were injected into a nano-LC system (Eksigent 1D+) coupled to LTQ-Orbitrap mass spectrometers (Thermo-Finnigan). Peptides were separated on custom 50 cm x .75uM (ID) reversed phase columns (Reprosil) at 40°C. Gradient elution was performed from 2% acetonitrile to 40% acetonitrile in 0.1 % formic acid over 2-3 hrs. LTQ-Orbitrap XL and Orbitrap Velos instruments were operated with XCalibur 2.0/2.1 software. Intact peptides were detected in the Orbitrap at 30.000 resolution. Internal calibration was performed using the ion signal from (Si(CH₃)₂O)₆H⁺ at m/z 445.120025⁴. Data dependent tandem mass spectra were generated for up to six peptide precursors using a combined CID/HCD approach⁵ or using HCD at a resolution of 7500 for histone modification data. For CID up to 5000 ions (Orbitrap XL) or up to 3000 ions (Orbitrap Velos) were accumulated in the ion trap within a maximum ion accumulation time of 200 msec. For HCD target ion settings were 50000 (Orbitrap XL) and 25000 (Orbitrap Velos), respectively.

### 10. Peptide and protein identification.

Mascot™ 2.0 (Matrix Science) was used for protein identification using 10 ppm mass tolerance for peptide precursors and 0.8 Da (CID) or 20 mDa (HCD) tolerance for fragment ions. Carbamidomethylation of cysteine residues and iTRAQ/TMT modification of lysine residues were set as fixed modifications and S,T,Y phosphorylation, methionine oxidation, N-terminal acetylation of proteins and iTRAQ/TMT modification of peptide N-termini were set as variable modifications. The search data base consisted of a customized version of the IPI protein sequence database combined with a decoy version of this database created using a script supplied by Matrix Science⁶. Unless stated otherwise, we accepted protein identifications as follows: i) For single spectrum to sequence assignments, we required this assignment to be the best match *and* a minimum Mascot score of 31 *and* a 10x difference of this assignment over the next best assignment. Based on these criteria, the decoy search results indicated <1% false positive identification rate; ii) For multiple spectrum to sequence assignments and using the same parameters, the decoy search results indicate <0.1% false positive identification rate. For protein quantification a minimum of 2 sequence assignments matching to unique peptides was required. False positive identification rate for quantified proteins was <<0.1%. Localization of post-translational modifications on histone peptides was validated by remapping the de-isotoped and deconvoluted tandem MS spectra to b and y ions expected from the peptide hit within 20 ppm mass accuracy. Only SSMs where fragment ions support localization on only one amino acid were considered for further analysis.

### 11. Peptide and protein quantification.

Centroided iTRAQ/TMT reporter ion signals were computed by the XCalibur software operating and extracted from MS data files using customized scripts. Only peptides unique for identified proteins were used for relative protein quantification. Reporter ion intensities were multiplied with the ion accumulation time yielding an area value proportional to the number of reporter ions present in the mass analyzer. For compound competition binding experiments fold changes are reported based on reporter ion areas in comparison to vehicle control and were calculated using a linear model. Fold changes were corrected for isotope purity as described and adjusted for interference caused by co-eluting nearly isobaric peaks as estimated by the s2i measure⁷. Dose-response curves were fitted using R software⁸ and the drc software⁹ as described previously¹. IC₅₀ values were confirmed in replicate experiments using targeted data acquisition⁷ for a subset of proteins. In order to compare selectivities of compounds displaying different absolute potencies relative potencies were calculated as (pIC₅₀-min(pIC₅₀))/(max(pIC₅₀)-min(pIC₅₀)) for each experiment. For the robustness estimation (Fig. 32b), displayed pIC₅₀ values were median normalized. Apparent dissociation constants (K_{D}^{app}) were derived from IC₅₀ values as described¹⁰. For immunoprecipitations, the Enrichment E was calculated as (A(IP) - A(mock IP))/(A(IP)+A(mock IP)) and scales between 0 and 1. 'A' represents the summed-up reporter ion response for the protein of interest. Relative enrichment (Fig. 33a) was calculated as RE(IP1) = ((A(IP1)-A(mock IP)) /(A(IP1)+ A(IP2)+A(mock IP)). A RE of 0.5 means that the protein was precipitated in both IPs equally well.

### 12. Heat map generation.

Heat maps and t-tests were performed using the R-software package. For unbiased hierarchical clustering of compound profiling data, all quantified proteins identified in at least 10 independent experiments were considered. Protein and compound clustering was based on relative potency using the Euclidean distance measure and the complete linkage method provided in R.

### 13. Synthetic procedures

### 13.1 Synthesis of a methylamino SAHA analogue N1-(4-(aminomethyl)phenyl)-N8-hydroxyoctanediamide for immobilization to beads

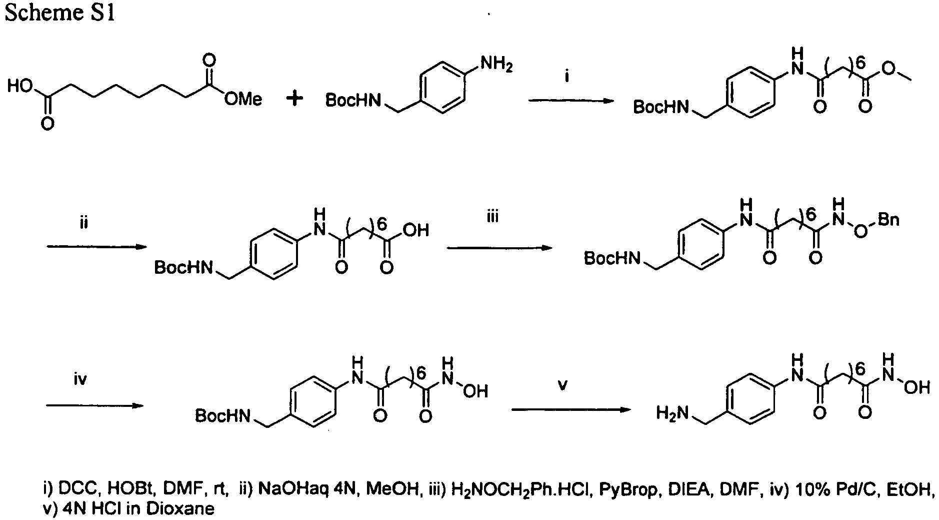

### 13.2 Synthesis of methyl 8-(4-((tert-butoxycarbonylamino)methyl)phenylamino)-8-oxooctanoate.

To a stirred solution of suberic acid monomethyl ester (3.75mmol, 1eq, 0.706g), 4[N-Boc aminomethyl]aniline (4.5mmol, 1.2eq, 1g), Hydroxybenzotrizol (4.5mmol, 1.2eq, 0.608g) in DMF (20ml), dicyclocarbodiamide (4.5mmol, 1.2eq, 0.928g) was added. The reaction was stirred at room temperature overnight. The reaction precipitate was filtered. The filtrate was treated with water (20ml). The resulting precipitate was filtered. The filtrate was then concentrated and purified by Flash Chromatography (hexane/ethyl acetate 1:1) to yield the desired compound as a white solid (m=1.25 g, 88%). LC/MS analysis yielded Rt=2.93mn, M+H=393, M+Na=415.

### 13.3 Synthesis of 8-(4-((tert-butoxycarbonylamino)methyl)phenylamino)-8-oxooctanoic acid.

To a stirred solution of 8-(4-((tert-butoxycarbonylamino)methyl)phenylamino)-8-oxooctanoate (0.579mmol, 0.227g) in methanol (7ml) was added 2N aqueous sodium hydroxide (1.16mmol, 2eq, 0.580ml). The reaction was stirred at room temperature overnight and the solvent was removed. The residue was dissolved in water. The pH was raised to 6 by addition of 2N aqueous hydrochloric acid. The resulting precipitate was filtered and dried in a vacuum oven (40°C) overnight to yield the desired compound as a white solid (m=0.169g, 77%) LC/MS analysis yielded Rt=2.48mn M+H=379, M+Na=401.

### 13.4 Synthesis of tert-butyl 4-(8-(benzylaminooxy)-8-oxooctanamido)benzylcarbamate.

To a stirred solution of 8-(4-((tert-butoxycarbonylamino)methyl)phenylamino)-8-oxooctanoic acid (0.397mmo, 1eq, 0.150g) and O-benzylhydroxylamine hydrochloride (0.397mmol, 1eq, 0.063g) in dimethylformamide (5ml) and diisopropylethylamine (1.59mmol, 4eq, 0.277ml) was added bromo-tris-pyrrolidino phosphoniumhexafluorophosphate (0.595mmol, 1.5eq, 0.277g). The reaction was stirred at room temperature overnight, diluted with water (10ml) and extracted with ethyl acetate (2x50ml). The organic layers were dried over magnesium sulphate, then concentrated and purified by flash chromatography (hexane/ethyl acetate (30-100%) to yield the desired product as an oil (m=0.105g, 55%) LC/MS analysis yielded rt=2.73mn, M+H=484, M+Na=506.

### 13.5 Synthesis of tert-butyl 4-(8-(aminooxy)-8-oxooctanamido)benzylcarbamate.

To a degassed solution of tert-butyl 4-(8-(benzylaminooxy)-8-oxooctanamido)benzylcarbamate (0.787mmol, 1eq, 0.380g) in ethanol (15ml) was added 10% Pd/C (10%, 38mg). The reaction was saturated in hydrogen and stirred under hydrogen atmosphere overnight. The catalyst was filtered and the filtrate concentrated to yield the desired compound as a yellow oil (0.309g, 99%). LC/MS analysis yielded RT=2.18mn, M+H=394, M+Na=416.

### 13.6 Synthesis of N-(4-(aminomethyl)phenyl)-8-(aminooxy)-8-oxooctanamide.

To tert-butyl 4-(8-(aminooxy)-8-oxooctanamido)benzylcarbamate (0.22mmol, 0.150g) was added 2ml of hydrochloric acid 4N in dioxane. The mixture was stirred at room temperature 3 hours. The reaction was evaporated and purified by HPLC (high pH) to yield the desired compound as an off white solid (m=51mg, 46%). LC/MS analysis yielded Rt=1.46mn 2M+H=587, M+Na=316. ¹NMR (DMSO-d6, 400MHz): δ= 9.81 (s, 1H), 7.51 (d,2H), 7.23 (d, 2H), 3.64 (s, 2H), 2.27 (d, 2H), 1.93 (d, 2H), 1.56 (m, 2H), 1.48 (m, 2H), 1.26 (m, 4H).

### 13.7 Preparation of the HDAC affinity matrix.

Beads were prepared by immobilizing N1-(4-(aminomethyl)phenyl)-N8-hydroxyoctanediamide (analogue of SAHA) and (6-(aminomethyl)naphthalen-2-yl)methyl (4-(hydroxycarbamoyl)phenyl)carbamate (analogue of ITF2357), on NHS-activated Sepharose 4 beads (GE Healthcare), as described previously ^{1, 2}. Ligand concentrations in the coupling reaction were 2 and 3 µmol/mL beads for the SAHA and ITF2357 analogs, respectively, and the completion of the coupling reaction was monitored by HPLC. Beads were washed with 10 ml of DMSO and isopropanol and were stored in isopropanol at - 20°C.

### 13.8 Synthesis of a methylamino ITF2357 analogue (6-(aminomethyl)naphthalen-2-yl)methyl (4-(hydroxycarbamoyl)phenyl)carbamate for immobilization to beads.

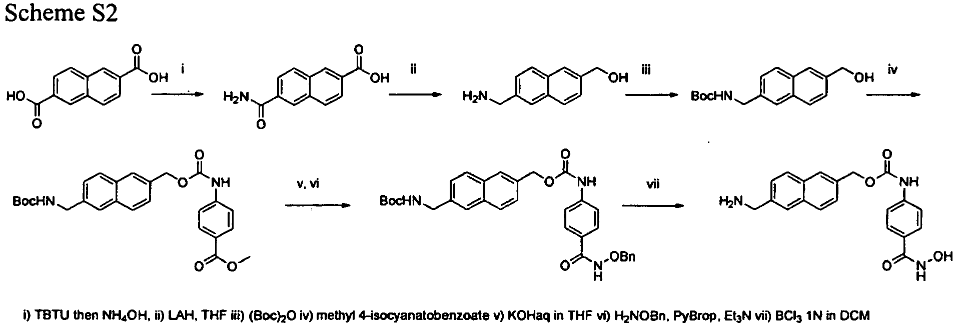

### 13.9 Synthesis of 6-carbamoyl-2-naphthoic acid.

To a solution of naphthalene-2,6-dicarboxylic acid (55.6mmol, 12g) in DMF (11) under nitrogen N-methyl morpholine (83.3mmol, 93.1ml) was added followed by TBTU (55.6mmol, 17.8g). The mixture was stirred at room temperature for 2 hours. Ammonium hydroxide (85% weight, 166.7mmol, 9ml) was then added and the reaction stirred at room temperaturet overnight. The reaction was filtered, the filtrate partitioned between saturated NaHCO₃ and Ethyl acetate (11). The aqueous layer was separated and acidified with 5N HCl. The resulting solid was separated, washed with water then with Ethyl acetate. The solid was dried to yield the desired compound (10.9g) as a mixture of desired product and starting material (2: 1) which was carried to the next step without further purification. LCMS Rt=2.73mn M+H=216.

### 13.10 Synthesis of (6-(aminomethyl)naphthalen-2-yl)methanol.

To a stirred suspension of LiAlH₄ (203mmol, 7.7g) in THF (300ml) was added slowly (over 1 hour) 6-carbamoyl-2-naphthoic acid (mixture 2:1 of desired product/di acid from the previous step). The reaction was stirred for 6 days. LiAlH₄ (50.7mmol, 2g) was added and the reaction refluxed overnight. The reaction was poured on ice/water. The solid was filtered and the organic layer separated. The organic layer was treated with 1N HCl, the solid removed, the aqueous layer basified, the solid removed and the filtrate extracted with DCM to yield the desired compound as a white solid (1.2g). ¹NMR (DMSO-d6, 400MHz): δ= 7.9-7.7 (m, 4H), 7.6-7.5 (q,2H), 4.6 (s, 2H), 3.85 (s, 2H).

### 13.11 Synthesis of tert-butyl ((6-(hydroxymethyl)naphthalen-2-yl)methyl)carbamate.

(Boc)₂O (7.1mmol, 1.54g) in DCM (10ml) was added slowly to a solution of (6-(aminomethyl)naphthalen-2-yl)methanol (6.4mmol, 1.2g) in DCM (200ml) at 0°C. The reaction was then stirred at room temperature overnight, then was washed with water (50ml), and the organic layer dried over Na₂SO₄. Purification by flash chromatography (10 to 50% EtOAc in Pet Ether) yielded the desired compound as a white solid (0.53mg, 29% yield). LCMS Rt=3.77mn M+H=288.

### 13.12 Synthesis of methyl 4-((((6-(((tert-butoxycarbonyl)amino)methyl)naphthalen-2-yl)methoxy)carbonyl) amino)benzoate.

To a solution of tert-butyl ((6-(hydroxymethyl)naphthalen-2-yl)methyl)carbamate (1.25 mmol, 0.360g) in toluene (50ml) under nitrogen was added slowly methyl 4-isocyanatobenzoate (3.14 mmol, 0.55mg). The reaction was stirred at 50°C overnight, then cooled at room temperature. The resulting precipitate was filtered, dissolved in DCM and extracted 5 times with water. The organic layer was dried over MgSO4, and evaporated to yield the desired compound as a white solid (0.420g, 72% yield). LCMS: Rt=4.47mn M-H=463.

### 13.13 Synthesis of tert-butyl ((6-((((4-((benzyloxy)carbamoyl)phenyl)carbamoyl)oxy)-methyl)naphthalen-2-yl)methyl)-carbamate.

To a solution of methyl 4-((((6-(((tert-butoxycarbonyl)amino)methyl)naphthalen-2-yl)methoxy)carbonyl)amino)benzoate (0.58mmol, 0.27g) in THF (20ml) a solution of 0.1M KOH in water (1.16mmol, 11.6ml) was added. The reaction was stirred at 50°C overnight, then evaporated to give a white solid. The solid was dissolved in DMF (70ml) followed by triethylamine (5.4mmol, 0.75ml), O-benzylhydroxylamine (1.16mmol, 0.185g), and PyBrop (1.16mmol, 0.600g). The reaction was stirred at room temperature overnight. The solvent was removed and the residue triturated in DCM. The solid was filtered to yield the desired compound as a white solid (0.180g, 54% yield). LCMS: Rt=4.29mn M-H=554.

### 13.14 Synthesis of (6-(aminomethyl)naphthalen-2-yl)methyl (4-(hydroxycarbamoyl)phenyl)-carbamate.

BCl₃ 1N in DCM (1.8mmol, 1.8ml) was added to a solution of tert-butyl ((6-((((4-((benzyloxy)carbamoyl)phenyl)carbamoyl)oxy)methyl)naphthalen-2-yl)methyl)carbamate (0.18mmol, 0.100g) in THF (25ml) at 0°C. The reaction was then refluxed for 1h. After cooling on ice, 1N HCl (20ml) was added. The reaction was then evaporated and loaded on a SCX column in methanol. The column was then eluted with 5%, then 10%, then 20% ammonia in methanol. The combined eluates were evaporated to yield the desired compound as a white solid (0.35mg, 53% yield). LCMS (method C): Rt=2.44mn M-H=364. ¹NMR (DMSO-d6, 400MHz): δ= 10 (s, 1H), 7.9 (m ,4H), 7.7 (m, 2H), 7.55 (0, 4H), 5.3 (s. 2H), 3.9 (s, 2H).

### References of the Method Section of Example 9

Bantscheff,M. et al. Quantitative chemical proteomics reveals mechanisms of action of clinical ABL kinase inhibitors. Nat. Biotechnol. 25, 1035-1044 (2007)
Drewes,G. et al. Process for the identification of novel enzyme interacting compounds. Patent WO 2006/134056 A1.
Harlow,E. & Lane,D. Antibodies: a Laboratory Manual. 1988. New York, Cold Spring Harbor Laboratory Publications.
Olsen,J.V. et al. Parts per million mass accuracy on an Orbitrap mass spectrometer via lock mass injection into a C-trap. Mol. Cell Proteomics. 4, 2010-2021 (2005).
Köcher,T. et al. High precision quantitative proteomics using iTRAQ on an LTQ Orbitrap: a new mass spectrometric method combining the benefits of all. J. Proteome. Res. 8, 4743-4752 (2009).
Elias,J.E., Haas,W., Faherty,B.K., & Gygi,S.P. Comparative evaluation of mass spectrometry platforms used in large-scale proteomics investigations. Nat. Methods 2, 667-675 (2005).
Savitski,M.M. et al. Targeted Data Acquisition for Improved Reproducibility and Robustness of Proteomic Mass Spectrometry Assays. J. Am. Soc. Mass Spectrom.(2010). R Development Core Team R: A language and environment for statistical computing. Vienna, Austria, 2007).
Ritz,C. & Streibig,J.C. Bioassay Analysis using R. J. Statist. Software 12, (2007). Sharma,K. et al. Proteomics strategy for quantitative protein interaction profiling in cell extracts. Nat. Methods 6, 741-744 (2009).

## Claims

1. A method for the identification of a histone deacetylase (HDAC) interacting compound, comprising the steps of
a) providing an HDAC protein complex containing protein preparation derived from a cell endogenously expressing an HDAC,
b) contacting the protein preparation with a non-proteinaceous HDAC ligand immobilized on a solid support and with a given compound thereby allowing the reversible binding of said ligand to said HDAC protein complex, and
c) determining to what extent in step b) a binding between the ligand and the protein complex has occurred.

2. A method for the identification of a histone deacetylase (HDAC) interacting compound, comprising the steps of
a) providing two aliquots of an HDAC protein complex containing protein preparation derived from a cell endogenously expressing an HDAC,
b) contacting one aliquot with a non-proteinaceous HDAC ligand immobilized on a solid support thereby allowing the reversible binding of said ligand to said HDAC protein complex,
c) contacting the other aliquot with a non-proteinaceous HDAC ligand immobilized on a solid support and with a given compound thereby allowing the reversible binding of said ligand to said HDAC protein complex, and
d) determining to what extent in steps b) and c) a binding between the ligand and the protein complex has occurred.

3. A method for the identification of a histone deacetylase (HDAC) interacting compound, comprising the steps of:
a) providing two aliquots of a cell preparation each comprising at least one cell endogenously expressing an HDAC,
b) incubating one aliquot with a given compound,
c) harvesting the cells of each aliquot,
d) lysing the cells of each aliquot in order to obtain protein preparations containing an HDAC protein complex,
e) contacting the protein preparations with a non proteinaceous HDAC ligand immobilized on a solid support thereby allowing the reversible binding of said ligand to said HDAC protein complex, and
f) determining to what extent in step e) a binding between the ligand and the protein complex has occurred.

4. The method of any of claims 1 to 3, wherein the HDAC is selected from the group consisting of HDAC1, HDAC2, HDAC3, HDAC6, HDAC8, and HDAC10.

5. The method of any of claims 2 to 4, wherein a reduced binding observed for the aliquot incubated with the compound in comparison to the aliquot not incubated with the compound indicates that HDAC is a target of the compound.

6. The method of any of claims 1 to 5, wherein the extent of the binding is determined by separating the HDAC protein complex from the ligand and subsequent detection of a component of the separated HDAC protein complex or subsequent determination of the amount of a component of the separated HDAC protein complex.

7. The method of claim 6, wherein said detection or said determination is performed by mass spectrometry or immunodetection methods, preferably with an antibody directed against the component.

8. The method of any of claims 1 to 7, wherein said given compound is selected from the group consisting of synthetic compounds, or organic synthetic drugs, more preferably small molecule organic drugs, and natural small molecule compounds.

9. The method of any of claims 1 to 8, wherein the given compound is an HDAC inhibitor.

10. The method of any of claims 1 to 9, wherein the provision of a protein preparation includes the steps of harvesting at least one cell endogenously expressing an HDAC and lysing the cell.

11. The method of any of claims 1 to 10, wherein the binding of the ligand to the HDAC protein complex steps is performed under essentially physiological conditions.

12. The method of any of claims 1 to 11, performed as an high-throughput-method.

13. A method for the identification of a histone deacetylase (HDAC) interacting compound, comprising the steps of
a) providing at least two aliquots of an HDAC containing protein preparation derived from a cell endogenously expressing an HDAC,
b) contacting one aliquot with a non-proteinaceous HDAC ligand immobilized on a solid support thereby allowing the reversible binding of said ligand to HDAC,
c) contacting the remaining aliquots with an HDAC ligand immobilized on a solid support and with various concentrations of a compound thereby allowing the reversible binding of said ligand to HDAC,
d) separating the HDAC from the the ligand, and
e) determining by mass spectrometry or immunodetection the amount of HDAC eluted from the ligand, thereby determining to what extent in steps b) and c) a binding between the ligand and the protein has occurred.

14. The method of claim 13, with the features as defined in any of claims 4, 5, and 8 to 11.

15. The method of any of claims 1 to 14, wherein the ligand has a molecular weight of less than 1000 Da.

## Patentansprüche

1. Verfahren zur Identifizierung einer Histondeacetylase-(HDAC)-interagierenden Verbindung, umfassend die Schritte
a) Bereitstellen eines HDAC-Proteinkomplexes, enthaltend eine Proteinzubereitung, die von einer Zelle hergeleitet ist, die eine HDAC endogen exprimiert,
b) Inkontaktbringen der Proteinzubereitung mit einem nicht-proteinösen HDAC-Liganden, immobilisiert auf einem festen Träger und mit einer gegebenen Verbindung, wodurch das reversible Binden des Liganden an den HDAC-Proteinkomplex ermöglicht wird, und
c) Bestimmen, in welchem Maße eine Bindung zwischen dem Liganden und dem Proteinkomplex in Schritt b) erfolgt ist.

2. Verfahren zur Identifizierung einer Histondeacetylase-(HDAC)-interagierenden Verbindung, umfassend die Schritte
a) Bereitstellen zweier Aliquote eines HDAC-Proteinkomplexes, enthaltend eine Proteinzubereitung, die von einer Zelle hergeleitet ist, die eine HDAC endogen exprimiert,
b) Inkontaktbringen eines Aliquots mit einem nicht-proteinösen HDAC-Liganden, immobilisiert auf einem festen Träger, wodurch das reversible Binden des Liganden an den HDAC-Proteinkomplex ermöglicht wird,
c) Inkontaktbringen des anderen Aliquots mit einem nicht-proteinösen HDAC-Liganden, immobilisiert auf einem festen Träger und mit einer gegebenen Verbindung, wodurch das reversible Binden des Liganden an den HDAC-Proteinkomplex ermöglicht wird, und
d) Bestimmen, in welchem Maße eine Bindung zwischen dem Liganden und dem Proteinkomplex in den Schritten b) und c) erfolgt ist.

3. Verfahren zur Identifizierung einer Histondeacetylase-(HDAC)-interagierenden Verbindung, umfassend die Schritte
a) Bereitstellen zweier Aliquote einer Zellzubereitung, jeweils umfassend mindestens eine Zelle, die eine HDAC endogen exprimiert,
b) Inkubieren eines Aliquots mit einer gegebenen Verbindung,
c) Ernten der Zellen eines jeden Aliquots,
d) Lysieren der Zellen eines jeden Aliquots, um Proteinzubereitungen zu erhalten, die einen HDAC-Proteinkomplex enthalten,
e) Inkontaktbringen der Proteinzubereitungen mit einem nicht-proteinösen HDAC-Liganden, immobilisiert auf einem festen Träger, wodurch das reversible Binden des Liganden an den HDAC-Proteinkomplex ermöglicht wird, und
f) Bestimmen, in welchem Maße eine Bindung zwischen dem Liganden und dem Proteinkomplex in Schritt e) erfolgt ist.

4. Verfahren nach einem beliebigen der Ansprüche 1 bis 3, wobei die HDAC ausgewählt ist aus der Gruppe bestehend aus HDAC1, HDAC2, HDAC3, HDAC6, HDAC8 und HDAC10.

5. Verfahren nach einem beliebigen der Ansprüche 2 bis 4, wobei eine beobachtete verringerte Bindung, welche für das Aliquot, welches mit der Verbindung inkubiert wird, im Vergleich zu dem Aliquot, welches nicht mit der Verbindung inkubiert wird, anzeigt, dass HDAC ein Ziel dieser Verbindung ist.

6. Verfahren nach einem beliebigen der Ansprüche 1 bis 5, wobei das Maß der Bindung durch Abtrennen des HDAC-Proteinkomplexes von dem Liganden und anschließender Detektion einer Komponente des abgetrennten HDAC-Proteinkomplexes oder anschließender Bestimmung der Menge einer Komponente des abgetrennten HDAC-Proteinkomplexes bestimmt wird.

7. Verfahren nach Anspruch 6, wobei die Detektion oder die Bestimmung durch Massenspektrometrie- oder Immundetektionsverfahren durchgeführt wird, vorzugsweise mit einem Antikörper, der gegen die Komponente gerichtet ist.

8. Verfahren nach einem beliebigen der Ansprüche 1 bis 7, wobei die gegebene Verbindung ausgewählt ist aus der Gruppe bestehend aus synthetischen Verbindungen oder organischen synthetischen Wirkstoffen, vorzugsweise niedermolekularen organischen Wirkstoffen und natürlich vorkommenden niedermolekularen Verbindungen.

9. Verfahren nach einem beliebigen der Ansprüche 1 bis 8, wobei die gegebene Verbindung ein HDAC-Inhibitor ist.

10. Verfahren nach einem beliebigen der Ansprüche 1 bis 9, wobei die Bereitstellung einer Proteinzubereitung die Schritte des Emtens mindestens einer Zelle, die eine HDAC endogen exprimiert, und Lysieren der Zelle einschießt.

11. Verfahren nach einem beliebigen der Ansprüche 1 bis 10, wobei der Schritt des Bindens des Liganden an den HDAC-Proteinkomplex unter im Wesentlichen physiologischen Bedingungen durchgeführt wird.

12. Verfahren nach einem beliebigen der Ansprüche 1 bis 11, durchgeführt als ein Hochdurchsatzverfahren.

13. Verfahren zur Identifizierung einer Histondeacetylase-(HDAC)-interagierenden Verbindungen, umfassend die Schritte
a) Bereitstellen mindestens zweier Aliquote einer HDAC enthaltenden Proteinzubereitung, die von einer Zelle hergeleitet ist, die eine HDAC endogen exprimiert,
b) Inkontaktbringen eines Aliquots mit einem nicht-proteinösen HDAC-Liganden immobilisiert auf einem festen Träger, wodurch das reversible Binden des Liganden an HDAC ermöglicht wird,
c) Inkontaktbringen der verbleibenden Aliquote mit einem HDAC-Liganden, immobilisiert auf einem festen Träger und mit verschiedenen Konzentrationen einer Verbindung, wodurch das reversible Binden des Liganden an HDAC ermöglich wird,
d) Abtrennen des HDAC von dem Liganden, und
e) Bestimmen der vom Liganden eluierten Menge an HDAC mittels Massenspektrometrie oder Immundetektion, wodurch bestimmt wird, in welchem Maße eine Bindung zwischen dem Liganden und dem Protein in den Schritten b) und c) erfolgt ist.

14. Verfahren nach Anspruch 13, des Weiteren mit den Merkmalen, wie definiert in einem beliebigen der Ansprüche 4, 5 und 8 bis 11.

15. Verfahren nach einem beliebigen der Ansprüche 1 bis 14, wobei der Ligand ein Molekulargewicht von weniger als 1000 Da aufweist.

## Revendications

1. Procédé d'identification d'un composé interagissant avec une histone désacétylase (HDAC), comprenant les étapes de
a) l'apport d'une préparation de protéines contenant un complexe de protéines HDAC, dérivée d'une cellule exprimant une HDAC de manière endogène,
b) la mise en contact de la préparation de protéines avec un ligand non protéique de HDAC immobilisé sur un support solide et avec un composé donné, permettant ainsi la liaison réversible dudit ligand audit complexe de protéines HDAC, et
c) la détermination de la mesure dans laquelle dans l'étape b) une liaison entre le ligand et le complexe de protéine s'est produite.

2. Procédé d'identification d'un composé interagissant avec une histone désacétylase (HDAC), comprenant les étapes de
a) l'apport de deux aliquotes d'une préparation de protéines contenant un complexe de protéines HDAC, dérivée d'une cellule exprimant une HDAC de manière endogène,
b) la mise en contact d'une aliquote avec un ligand non protéique de HDAC immobilisé sur un support solide, permettant ainsi la liaison réversible dudit ligand audit complexe de protéines HDAC,
c) la mise en contact de l'autre aliquote avec un ligand non protéique de HDAC immobilisé sur un support solide et avec un composé donné, permettant ainsi la liaison réversible dudit ligand audit complexe de protéines HDAC, et
d) la détermination de la mesure dans laquelle dans les étapes b) et c) une liaison entre le ligand et le complexe de protéine s'est produite.

3. Procédé d'identification d'un composé interagissant avec une histone désacétylase (HDAC), comprenant les étapes de :
a) l'apport de deux aliquotes d'une préparation cellulaire comprenant chacune au moins une cellule exprimant une HDAC de manière endogène,
b) l'incubation d'une aliquote avec un composé donné,
c) la collecte des cellules de chaque aliquote,
d) la lyse des cellules de chaque aliquote afin d'obtenir des préparations de protéines contenant un complexe de protéines HDAC,
e) la mise en contact des préparations de protéines avec un ligand non protéique de HDAC immobilisé sur un support solide, permettant ainsi la liaison réversible dudit ligand audit complexe de protéines HDAC, et
f) la détermination de la mesure dans laquelle dans l'étape e) une liaison entre le ligand et le complexe de protéine s'est produite.

4. Procédé selon l'une quelconque des revendications 1 à 3, dans lequel la HDAC est sélectionnée dans le groupe consistant en HDAC1, HDAC2, HDAC3, HDAC6, HDAC8 et HDAC 10.

5. Procédé selon l'une quelconque des revendications 2 à 4, dans lequel une liaison réduite observée pour l'aliquote incubée avec le composé par rapport à l'aliquote non incubée avec le composé indique que la HDAC est une cible du composé.

6. Procédé selon l'une quelconque des revendications 1 à 5, dans lequel l'importance de la liaison est déterminée en séparant le complexe de protéines HDAC du ligand, et la détection subséquente du composant du complexe de protéines HDAC séparé ou la détermination subséquente de la quantité d'un composant du complexe de protéines HDAC séparé.

7. Procédé selon la revendication 6, dans lequel ladite détection ou ladite détermination est réalisée par des procédés de spectrométrie de masse ou d'immunodétection, de préférence avec un anticorps dirigé contre le composant.

8. Procédé selon l'une quelconque des revendications 1 à 7, dans lequel ledit composé donné est sélectionné dans le groupe consistant en des composés synthétiques, ou des médicaments organiques synthétiques, plus préférentiellement des médicaments organiques à petite molécule, et des composés naturels à petite molécule.

9. Procédé selon l'une quelconque des revendications 1 à 8, dans lequel le composé donné est un inhibiteur de HDAC.

10. Procédé selon l'une quelconque des revendications 1 à 9, dans lequel l'apport d'une préparation de protéines inclut les étapes de collecte d'au moins une cellule exprimant une HDAC de manière endogène et de lyse de la cellule.

11. Procédé selon l'une quelconque des revendications 1 à 10, dans lequel l'étape de liaison du ligand au complexe de protéines HDAC est réalisée dans des conditions essentiellement physiologiques.

12. Procédé selon l'une quelconque des revendications 1 à 11, réalisé sous la forme d'un procédé à haut débit.

13. Procédé d'identification d'un composé interagissant avec une histone désacétylase (HDAC), comprenant les étapes de
a) l'apport d'au moins deux aliquotes d'une préparation de protéines contenant une HDAC, dérivée d'une cellule exprimant une HDAC de manière endogène,
b) la mise en contact d'une aliquote avec un ligand non protéique de HDAC immobilisé sur un support solide, permettant ainsi la liaison réversible dudit ligand à la HDAC,
c) la mise en contact des aliquotes restantes avec un ligand de HDAC immobilisé sur un support solide et avec diverses concentrations d'un composé, permettant ainsi la liaison réversible dudit ligand à la HDAC,
d) la séparation de la HDAC et du ligand, et
e) la détermination par spectrométrie de masse ou immunodétection de la quantité de HDAC éluée du ligand, pour ainsi déterminer dans quelle mesure dans les étapes b) et c) une liaison entre le ligand et la protéine s'est produite.

14. Procédé selon la revendication 13, avec les caractéristiques telles que définies dans l'une quelconque des revendications 4, 5 et 8 à 11.

15. Procédé selon l'une quelconque des revendications 1 à 14, dans lequel le ligand a un poids moléculaire inférieur à 1000 Da.
